(19) Europäisches Patentamt
European Patent Office
Office européen des brevets

(11) Numéro de publication : **0 340 064 B1**

(12) # FASCICULE DE BREVET EUROPEEN

(45) Date de publication du fascicule du brevet : **17.03.93 Bulletin 93/11**

(21) Numéro de dépôt : **89400969.5**

(22) Date de dépôt : **07.04.89**

(51) Int. Cl.$^5$ : **C07D 487/06,** C07D 471/06, A61K 31/55, // (C07D487/06, 243:00, 209:00), (C07D471/06, 243:00, 221:00)

(54) **Benzodiazépines, leur procédé et intermédiaires de préparation et leurs applications en thérapeutique.**

(30) Priorité : **25.04.88 FR 8805443**

(43) Date de publication de la demande : **02.11.89 Bulletin 89/44**

(45) Mention de la délivrance du brevet : **17.03.93 Bulletin 93/11**

(84) Etats contractants désignés : **AT BE CH DE ES FR GB GR IT LI LU NL SE**

(56) Documents cités : **EP-A- 0 167 919**

(73) Titulaire : **JOUVEINAL S.A.
Tour Maine Montparnasse 33 Avenue du Maine
F-75755 Paris Cedex 15 (FR)**

(72) Inventeur : **Calvet, Alain Pierre
56, Avenue du Colonel de Rochebrune
F-92380 Garches (FR)**
Inventeur : **Junien, Jean-Louis
36, Avenue Eifel
F-92310 Sevres (FR)**
Inventeur : **Pascal, Yves Robert Alain
16, Rue Georges Tournier
F-92500 Rueil-Malmaison (FR)**
Inventeur : **Pascaud, Xavier Bernard Louis
41, Rue de Charenton
F-75012 Paris (FR)**
Inventeur : **Roman, François Joseph
10, Rue de Dieppe
F-92400 Courbevoie (FR)**

(74) Mandataire : **Bourgognon, Jean-Marie et al
Cabinet Flechner 22, Avenue de Friedland
F-75008 Paris (FR)**

EP 0 340 064 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative aux benzodiazépines, à leur procédé et intermédiaires de préparation et à leur application en thérapeutique.

La cholécystokinine, abrégée ci-dessous en CCK, est un peptide comportant trente-trois aminoacides dans la forme isolée à l'origine. Mais il circule dans l'organisme des formes actives comportant trente-neuf, douze et huit aminoacides. La forme comportant les huit aminoacides de l'extrémité acide carboxylique du peptide est le plus court enchaînement d'aminoacides présentant l'activité. Elle est désignée ci-dessous par les abréviations CCK-8, CCK-8 SO4 ou CCK-8 sulfatée ; ces deux dernières abréviations signifiant que le groupe phénol de la tyrosine en position 27 de la cholecystokinine est estérifié par un groupe -SO3H, ce qui est le cas dans la forme naturelle.

On a déjà décrit, au brevet européen 0 167 919, des benzodiazépines qui sont des antagonistes de la cholecystokinine qui se lient spécifiquement aux récepteurs de celles-ci, ce qui permet d'envisager leur utilisation dans le traitement de troubles du système nerveux central, de l'estomac, de l'intestin, du pancréas ou de la vésicule biliaire et d'autres troubles dépendant de la CCK. Parmi les innombrables benzodiazépines englobées au brevet européen 167919, c'est le (+/-)-N-(dihydro-2,3-méthyl-1-oxo-2-phényl-5-1H-benzodiazépin-1,4-yl-3-1H-indolecarboxamide-2), dénommé ci-après composé IIa, qui a été choisi par le breveté en vue d'être développé à l'échelle industrielle (Pharma Project, 12 janvier 1987).

Or on a maintenant trouvé des benzodiazépines qui, tout en présentant des activités sensiblement égales à celles des composés du brevet européen précité les plus actifs, et notamment à celles du composé (IIa), sont nettement moins toxiques, en sorte que leur indice thérapeutique est meilleur. D'autre part, le rapport de la concentration inhibitrice 50 % ($CI_{50}$) pour les récepteurs de la CCK du cerveau de cobaye à la $CI_{50}$ pour les récepteurs du pancréas de rat est en faveur d'une activité périphérique, ce qui permet d'espérer une meilleure sélectivité dans le traitement des affections dépendant d'un contrôle périphérique et donc des médicaments ne présentant que peu ou pas d'effet secondaire.

L'invention a donc pour objet des benzodiazépines de formule (I) :

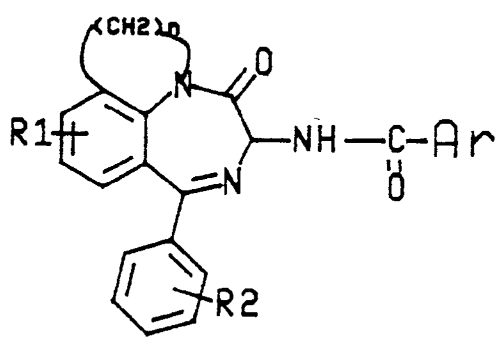

(I)

dans laquelle $R_1$ est H ou halogène, $R_2$ est H ou halogène, Ar est indolyle, phényle, naphtyle, indolyle monosubstitué par un halogène ou par un méthoxy ou phényle mono di ou trisubstitué par un halogène, par un méthoxy ou par un groupe trifluorométhyle et n est 2 ou 3 ; et leurs isomères optiques.

Parmi ces benzodiazépines nouvelles, on préfère tout particulièrement celles dans lesquelles $R_1$ est en position para par rapport à l'atome d'azote commun au cycle diazépine et à l'autre hétérocycle azoté condensé au cycle diazépine. De préférence également, $R_1$ est le chlore. On préfère aussi les composés dans lesquels $R_2$ est en position ortho par rapport à l'atome de carbone reliant le cycle phényle au cycle diazépine, avec une préférence pour la signification fluor pour $R_2$. Parmi les composés préférés figurent ceux pour lesquels Ar est 2-indolyle. On préfère de manière générale les benzodiazépines de formule (I) dans lesquelles l'atome de carbone asymétrique en position alpha par rapport au carbonyle du cycle diazépine possède la configuration absolue S, configuration définie suivant la nomenclature de Cahn, Ingold et Prelog.

L'invention vise également un procédé de préparation de benzodiazépines de formule (I) qui consiste à mettre à réagir

a) une amine racémique de formule (V) :

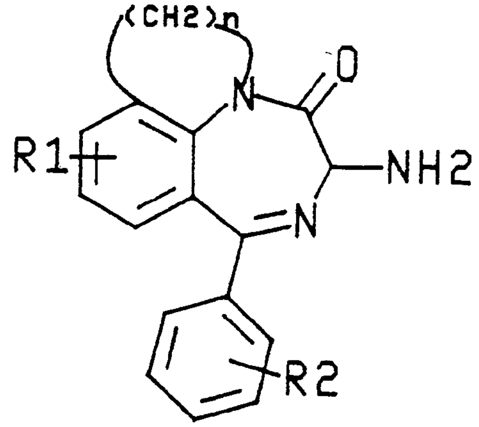

(V)

sur un dérivé d'acide carboxylique de formule Ar-CO-X dans laquelle Ar a la signification indiquée ci-dessus et X est un halogène, un groupe azido (-N3), un groupe imidazol-1-yle, un groupe $-O-CO-R_3$, $R_3$ pouvant être, outre de préférence Ar, un radical alkyle encombré comportant de trois à six atomes de carbone, ou aryle plus encombré que le radical Ar, de préférence substitué par un ou plusieurs halogènes, ou un groupe $-OR_4$, $R_4$ étant un groupe aromatique comportant un ou deux cycles substitué par un ou plusieurs radicaux nitro ou halogènes pour obtenir un composé de formule (I) racémique.

b) une amine de formule V optiquement active de la même façon qu'au paragraphe a) pour obtenir un composé de formule (I) optiquement actif.

On peut notamment opérer de la manière suivante :

un composé de formule (V) est dissous dans 5 à 50 volumes d'un solvant organique anhydre ou hydraté comme par exemple un hydrocarbure chloré comme du dichlorométhane ou du chloroforme, un éther-oxyde linéaire ou cyclique comme du diméthoxy-1,2-éthane, du tétrahydrofuranne ou du dioxanne, un solvant polaire aprotique tel que de la pyridine, du diméthylsulfoxyde ou du diméthylformamide ou tout autre solvant convenable pour y effectuer une réaction de condensation ou encore un mélange approprié de deux ou plusieurs de ces solvants et on y ajoute un à deux équivalents d'un agent d'acylation de formule Ar-CO-X dans laquelle Ar a la signification définie plus haut et X est :

- un halogène de préférence un chlore,
- un groupe azido (-N3)
- un groupe imidazol-1-yle
- un groupe de formule -O-CO-Ar dans laquelle Ar a la signification définie plus haut,
- un groupe de formule $-O-CO-R_3$ dans laquelle $R_3$ a la signification indiquée plus haut, les groupes $R_3$ préférés étant le tertio-butyle ou le trichloro-2,4,6-benzoyle,
- un groupe de formule $-O-R_4$ dans laquelle $R_4$ a la signification indiquée plus haut, les groupes $R_4$ préférés étant le para-nitrophényle, le dinitro-2,4-phényle, le pentachlorophényle, le pentafluorophényle et le benzotriazol-1-yle, dans ce dernier cas le réactif peut être préparé par réaction de l'acide carboxylique Ar-COOH (Ar ayant la signification définie plus haut) et l'hydroxy-1-benzotriazole en présence d'une carbodiimide comme par exemple la dicyclohexylcarbodiimide ou la diisopropylcarbodiimide.

Puis on ajoute la même quantité d'une base minérale ou organique comme une amine aliphatique de préférence de la triéthylamine et on agite à une température comprise entre -20°C et la température d'ébullition du mélange durant une période comprise entre dix minutes et plusieurs heures, une période de trente minutes à une heure étant en général suffisante pour assurer la complétion de la réaction. Le milieu réactionnel éventuellement dilué par un des solvants cités ci-dessus est alors successivement traité par une solution diluée d'un acide minéral comme par exemple un acide halohydrique ou de l'acide sulfurique de préférence de l'acide chlorhydrique environ décinormal, puis par une solution saturée de bicarbonate de sodium puis par de l'eau. Après évaporation du solvant le produit est généralement purifié par chromatographie sur colonne de silice (chromatographie flash) ou par chromatographie liquide sous haute pression (HPLC) puis éventuellement par recristallisation.

Une autre possibilité consiste à utiliser comme réactif d'acylation un mélange de l'acide carboxylique Ar-COOH et d'une carbodiimide, de préférence la dicyclohexylcarbodiimide ou la diisopropylcarbodiimide.

Tout autre procédé connu pour la formation in situ d'un réactif ArCO-X mis en oeuvre pour former une fonction amide est également convenable et en particulier les procédés utilisant l'éthoxy-2-éthyloxycarbonyl-1-dihydro-1,2-quinoléine (EEDQ, The peptides Gross et Meienhofer, vol. 1, Academic Press, 1979, p. 358), le réactif dit de Woodward (Woodward reagent K, The Peptides, Gross et Meienhofer, vol. 1, Academic Press,

1979, p. 122) ou le réactif dit de Castro (hexafluorophosphate de benzotriazol-1-yl-oxy-tris-(diméthylamino)-phosphonium) (J. Chem. Soc. Perkin Trans. 1, 1987, 1915-1919).

On peut préparer l'amine de formule V en aminant en la position alpha par rapport au carbonyle une benzodiazépine-[1,4]-one de formule (III) :

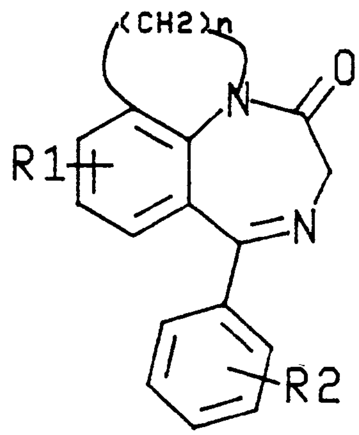

( I I I )

par un dérivé d'hydroxylamine ou par la chloramine. On peut également aminer la benzodiazépine-[1,4]-one de formule (III) en deux stades, le premier stade consistant à la faire réagir sur un réactif d'oximation de formule $R_5$-(N = O)m dans lequel $R_5$ est alcoxy inférieur ou chlore quand m est égal à 1 et est une liaison supplémentaire entre les atomes d'azote quand m est égal à 2, pour obtenir l'oxime de formule (IV) :

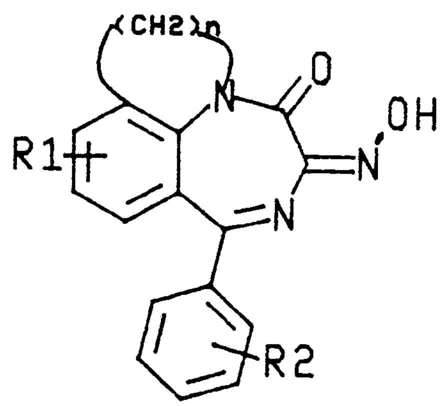

( I V )

que l'on isole, et le second stade consistant à réduire l'oxime catalytiquement par de l'hydrogène en présence d'un catalyseur de réduction ou par réaction avec du zinc en présence d'acide acétique ou avec du chlorure stanneux en présence d'acide chlorhydrique pour obtenir le dérivé aminé de formule V.

On peut encore préparer l'amine de formule (V) en mettant une benzodiazépine-1,4 one de formule (III) à réagir en milieu basique sur un réactif susceptible d'introduire une fonction azoture sur un carbanion pour obtenir un azoture de formule (VI) :

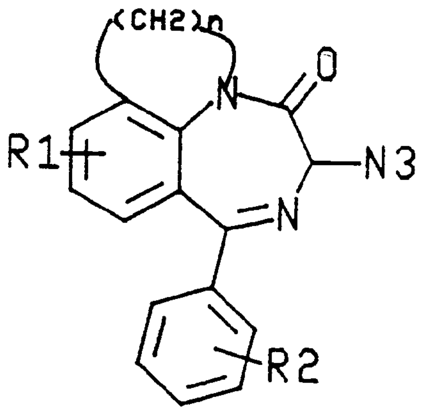

(VI)

que l'on isole, puis en réduisant l'azoture de formule (VI) par un agent réducteur.

On prépare un composé de formule (III) par le procédé décrit par Hester J.B. et autres (J. Med. Chem. ; 13 ; 827 ; 1970).

Pour préparer un composé de formule (V) en une étape, à partir d'un composé de formule (III), on opère de la manière suivante :

Un composé de formule (III) est dissous dans 10 à 100 volumes d'un solvant organique anhydre, comme par exemple un hydrocarbure aromatique ou un éther linéaire ou cyclique, comme par exemple le tétrahydrofuranne ou dans un solvant polaire aprotique tel que le dimethylformamide, l'hexaméthylphosphorotriamide, le diméthylsulfoxyde, la N-méthylpyrrolidinone ou le sulfolane (tétraméthylènesulfone) ou encore dans un mélange de ces solvants. La solution est maintenue à une température comprise entre -50 et 0°C et on ajoute 2 à 4 équivalents d'un agent basique susceptible de déplacer le proton en $\alpha$ du groupe carbonyle du cycle diazépine comme par exemple un alcoolate inférieur de métal alcalin de préférence du tertio-butylate ou du tertio-amylate de potassium. Le mélange est agité durant une période comprise entre 10 et 60 minutes et on y ajoute 3 à 20 équivalents d'un réactif d'amination tel que par exemple un dérivé d'hydroxylamine, l'O-(2,4-dinitrophényl)-hydroxylamine ou l'O-(diphénylphosphinyl)-hydroxylamine ou l'O-(2,4,6-triméthylphénylsulfonyl)-hydroxylamine ou la chloramine, puis on agite durant une période comprise entre 10 et 60 minutes. Le milieu réactionnel est alors concentré, les sels présents dans le milieu sont filtrés, le milieu est éventuellement dilué avec de l'eau et le produit extrait par un solvant organique non miscible à l'eau, et après évaporation du solvant le produit est généralement purifié par chromatographie sur colonne de silice (chromatographie flash) ou par chromatographie liquide sous haute pression (HPLC) puis éventuellement par recristallisation.

Pour préparer un composé de formule (V) en deux étapes à partir d'un composé de formule (III) en isolant un composé intermédiaire de formule (IV) comportant une fonction oxime on opère de la manière suivante (mode opératoire préféré) :

Un composé de formule (III) est dissous dans 10 à 100 volumes d'un solvant organique anhydre comme par exemple un hydrocarbure aromatique ou un éther linéaire ou cyclique comme par exemple le tétrahydrofuranne ou dans un solvant polaire aprotique tel que le dimethylformamide, l'hexaméthylphosphorotriamide, le diméthylsulfoxyde, la N-méthylpyrrolidone ou le sulfolane ou encore dans un mélange de ces solvants. La solution est maintenue à une température comprise entre -50 et 0°C et on ajoute 2 à 4 équivalents d'un agent basique susceptible de déplacer le proton en alpha du C = O, comme par exemple un alcoolate inférieur de métal alcalin de préférence du tertiobutylate ou du tertio-amylate de potassium. Le mélange est agité durant une période comprise entre 10 et 60 minutes et on y ajoute 3 à 20 équivalents d'un nitrite d'alkyle inférieur de préférence du nitrite d'isoamyle ou du chlorure de nitrosyle (NOCl) ou encore du tétroxyde d'azote ($N_2O_4$) et on laisse le mélange revenir à la température ambiante puis on agite durant une période comprise entre 10 et 60 minutes. Le milieu réactionnel est alors neutralisé par addition d'une solution au dixième d'acide acétique et le produit extrait en une ou plusieurs fois par un solvant organique non miscible à l'eau comme par exemple un hydrocarbure aliphatique ou aromatique, un hydrocarbure halogéné, un éther ou un ester d'alcool inférieur d'un acide carboxylique inférieur. Après évaporation du solvant le produit est généralement purifié par chromatographie sur colonne de silice (chromatographie flash) ou par chromatographie sous haute pression (HPLC) puis éventuellement par recristallisation.

Le composé intermédiaire de formule (IV) est alors transformé en composé aminé de formule (V) de la manière suivante :

Un composé de formule (IV) est mis en suspension dans 5 à 100 volumes d'un solvant organique comme par exemple un alcool aliphatique inférieur ou un ester d'alcool aliphatique inférieur d'un acide carboxylique

inférieur en présence d'un catalyseur d'hydrogénation comme par exemple du nickel de Raney, du rhodium sur charbon ou du ruthénium sur charbon qui est le réactif généralement préféré. La suspension est agitée sous une atmosphère d'hydrogène à une pression comprise entre la pression atmosphérique et trente atmosphères durant une période comprise entre une et cinquante heures à une température comprise entre 0°C et 80°C ; une pression légèrement supérieure à la pression atmosphérique, une température d'environ 70°C et une période d'agitation de deux heures étant en général suffisantes pour assurer une réaction complète. Le milieu réactionnel est alors filtré et le catalyseur lavé plusieurs fois avec un solvant du même type que celui cité ci-dessus. Après évaporation du solvant le produit est généralement purifié par chromatographie sur colonne de silice (chromatographie flash) ou par chromatographie liquide sous haute pression (HPLC) puis éventuellement par recristallisation. Il est également possible d'effectuer cette réduction non pas catalytiquement mais chimiquement (de manière stoechiométrique) en opposant le composé de formule (IV) à un agent réducteur comme par exemple du zinc dans de l'acide acétique ou du chlorure stanneux dans de l'acide chlorhydrique ou encore du borure de nickel (réactif préparé par action de borohydrure de sodium sur du chlorure de nickel bivalent).

Pour préparer un composé de formule (V) en deux étapes à partir d'un composé de formule (III) en isolant un composé intermédiaire de formule (VI) comportant une fonction azide on opère de la manière suivante :

Un composé de formule (III) est dissous dans 5 à 100 volumes d'un solvant organique anhydre, comme par exemple un hydrocarbure aromatique ou un éther linéaire ou cyclique comme par exemple le tétrahydrofuranne ou dans un poly-ether (solvant de la série dite des glymes) ou dans un solvant polaire aprotique tel que le dimethylformamide, l'hexaméthylphosphotriamide, le diméthylsulfoxyde, la N-méthylpyrrolidinone ou le sulfolane ou encore dans un mélange de ces solvants. La solution est maintenue à une température comprise entre -50 °C et la température ambiante et on ajoute 1 à 4 équivalents d'un agent basique susceptible de déplacer le proton en alpha du CO d'un composé de formule (III) comme par exemple un alcoolate inférieur de métal alcalin de préférence du tertio-butylate ou du tertio-amylate de potassium, du butyllithium, de l'hydrure de sodium ou de l'amidure de sodium. Le mélange est agité durant une période comprise entre 10 minutes et 6 heures et on y ajoute 1 à 5 équivalents d'un réactif susceptible d'introduire une fonction azide sur un carbanion, le réactif préféré étant l'azidure de tosyle et on laisse éventuellement le mélange revenir à la température ambiante puis on agite durant une période comprise entre 10 minutes et 5 heures à une température comprise entre la température ambiante et la température de reflux du mélange. Les sels qui sont présents dans le milieu réactionnel sont alors éventuellement filtrés, puis le milieu réactionnel neutralisé par addition d'une solution au dixième d'acide acétique et le produit extrait en une ou plusieurs fois par un solvant organique non miscible à l'eau comme par exemple un hydrocarbure aliphatique ou aromatique, un hydrocarbure halogéné, un éther ou un ester d'alcool inférieur d'un acide carboxylique inférieur, la phase organique est alors éventuellement lavée avec une solution diluée d'un acide ou d'une base minérale puis par de l'eau. Après évaporation du solvant le produit est généralement purifié par chromatographie sur colonne de silice (chromatographie flash) ou par chromatographie liquide sous haute pression (HPLC) puis éventuellement par recristallisation.

Le composé intermédiaire de formule (VI) est alors transformé en composé aminé de formule (V) de la manière suivante :

Un composé de formule (VI) est mis en suspension dans 5 à 100 volumes d'un solvant organique comme par exemple un alcool aliphatique inférieur, un ester d'alcool aliphatique inférieur d'un acide carboxylique inférieur, un solvant aromatique comme par exemple du benzène du toluène ou de la pyridine, de l'eau ou encore un mélange de ces solvants et on y ajoute 1 à 10 équivalents d'un agent réducteur comme par exemple du chlorure de vanadium II (VCl2) en solution aqueuse, du borohydrure de sodium (en présence de méthanol), de l'hydrogène sulfuré ou du nickel de Raney. Le milieu réactionnel est agité à une température comprise entre la température ambiante et la température de reflux du mélange durant une période suffisamment longue pour assurer la complètion de la réaction (selon la nature de l'agent réducteur utilisé cette période qui est déterminée par chromatographie sur couche mince peut varier de 10 minutes à plusieurs heures) puis éventuellement refroidi, filtré pour éliminer le précipité qui peut être présent, neutralisé, dilué, le solvant est éliminé par distillation sous pression réduite puis le produit est généralement purifié par chromatographie sur colonne de silice (chromatographie flash) ou par chromatographie liquide sous haute pression (HPLC), puis éventuellement par recristallisation.

Il est également possible d'effectuer cette réduction non pas stoechiométriquement mais d'une manière catalytique en opposant le composé de formule (VI) à un agent réducteur en présence d'un catalyseur de réduction comme par exemple du formiate d'ammonium en présence de palladium sur charbon ou de l'hydrogène (sous une pression comprise entre 1 et 5 atmosphères) en présence de palladium déposé sur du carbonate de calcium (catalyseur dit de Lindlar). Après filtration du catalyseur le produit aminé de formule (V) est isolé d'une manière analogue à celle décrite plus haut.

On donne, ci-après, un schéma illustrant le procédé suivant l'invention.

SCHEMA DE SYNTHESE

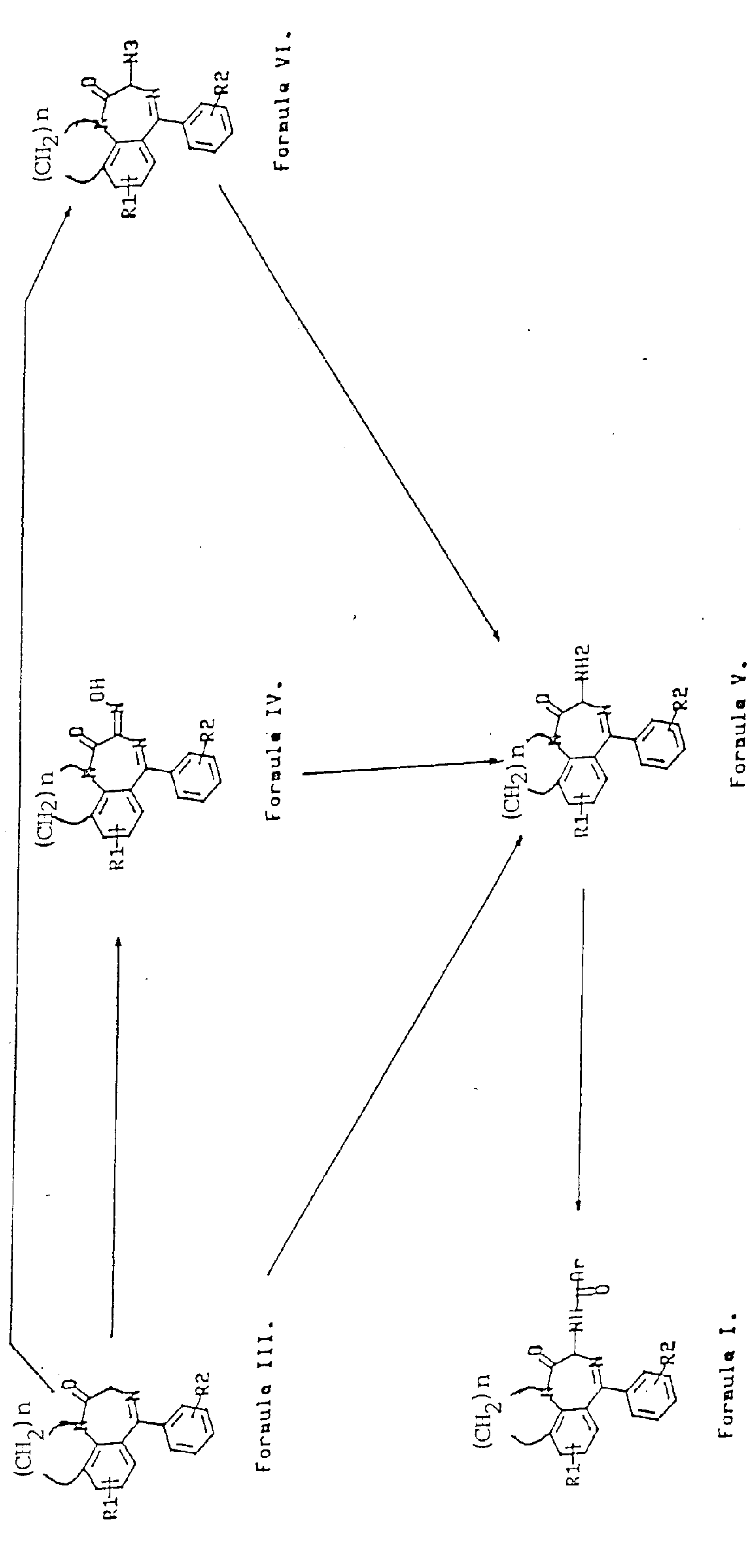

Formula VI.

Formula IV.

Formula V.

Formula III.

Formula I.

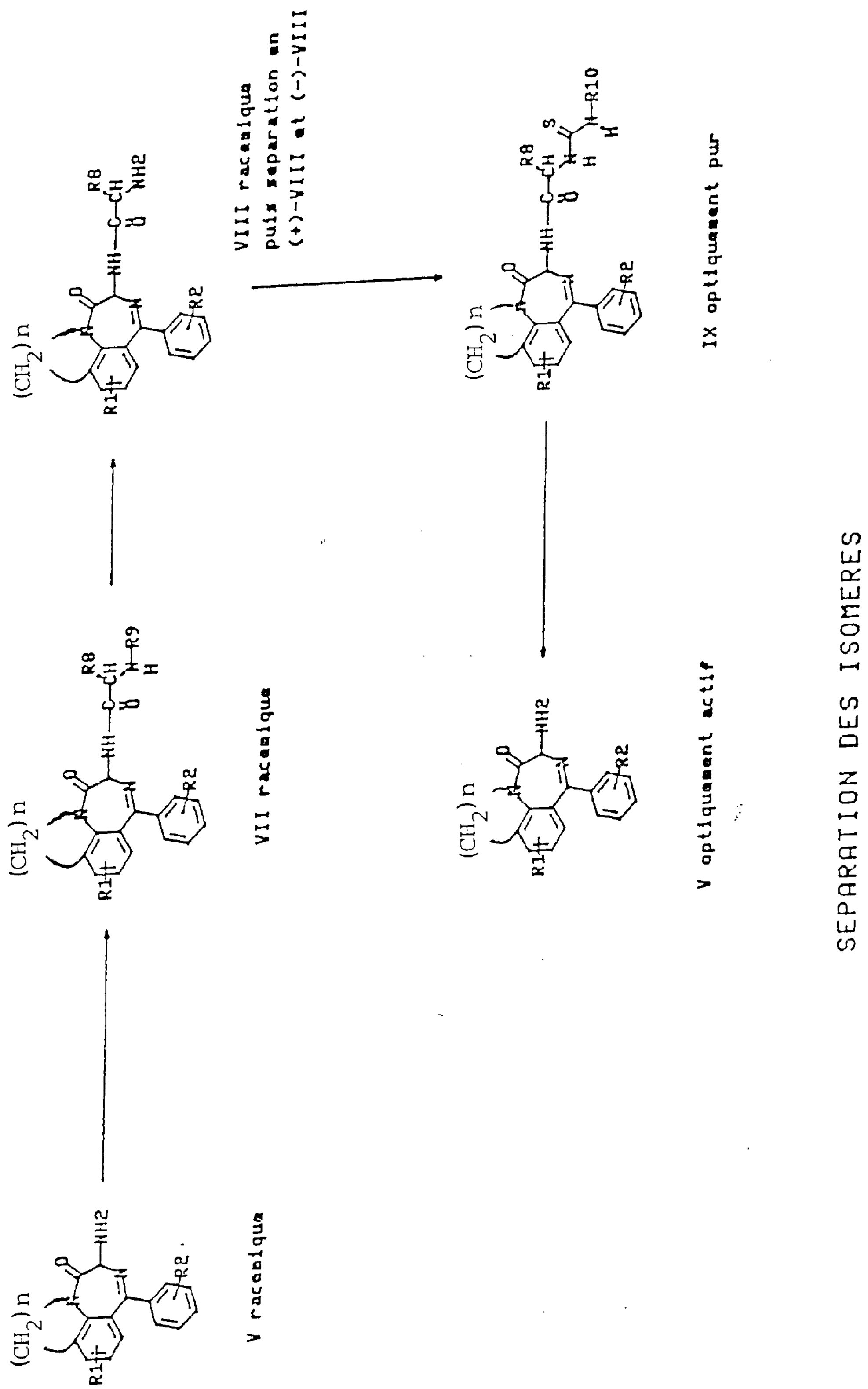


Pour préparer un composé de formule I, optiquement actif, on opère de la même manière que ci-dessus en partant d'une benzodiazépine de formule V, optiquement active, préparée de la manière ci-dessous.

Une benzodiazépine de formule (V), racémique, est condensée avec une molécule ; dérivant d'un amino-acide optiquement actif, naturel ou non, appartenant à la série D ou à la série L, de formule (X) :

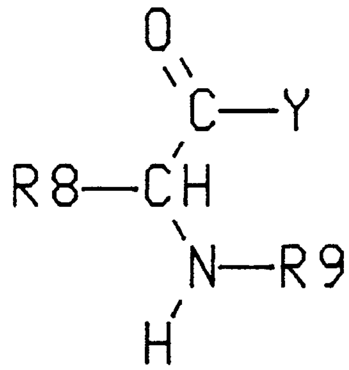

( X )

Y étant un groupe hydroxy, un groupe azido ($-N_3$), un groupe imidazol-1-yle, un groupe $-O-CO-R_3$, $R_3$ pouvant être un radical alkyle encombré comportant de trois à six atomes de carbone, un groupe aryle encombré, de préférence substitué par un ou plusieurs halogènes, ou un groupe $-OR_4$, $R_4$ étant un groupe aromatique comportant un ou deux cycles éventuellement substitué par un ou plusieurs radicaux nitro ou halogènes. De préférence $R_4$ est un groupe benzotriazolyle-1.

$R_8$ étant

- un groupement alkyle ayant de 1 à 6 atomes de carbone éventuellement substitué par un hydroxyle, un groupe thioalkyle ayant de un à six atomes de carbone dans le groupe alkyl ou un groupe carboxyle ou carbonylamino,
- un groupe aryle comportant un ou deux cycles éventuellement substitué par un hydroxyle, notamment phényle ou benzyle
- un groupe aralkyle ayant un ou deux cycles aromatiques et dont la portion alkylique comporte de un à six atomes de carbone, éventuellement substitué sur le cycle par un ou plusieurs groupes halogènes, hydroxy ou méthoxy
- un hétérocycle ayant cinq ou six chainons, et un ou deux hétéroatomes choisis parmi l'azote, l'oxygène et le soufre
- un groupe indolyl-3-méthyle,
- un groupe imidazolyl-4-méthyle. De préférence $R_8$ sera le groupe isobutyle et l'amino-acide appartiendra à la série L,

$R_9$ étant un groupement pouvant être enlevé facilement pour régénérer l'amine libre et peut être

- un radical oxycarbonyle de structure A-O-CO- dans lequel A est un groupe alkyle comportant de un à six atomes de carbone, ou un groupe aryle, éventuellement substitué par un ou plusieurs groupes méthoxy, halogène ou nitro, par exemple benzyle, p-chlorobenzyle, p-bromobenzyle p-nitrobenzyle, fluorènyl-9-méthyle, p-methoxybenzyle, dichloro-2,4-benzyle, dichloro-2,6-benzyle, t-amyle, isopropyle, adamantyle
- un groupe alkanoyle ou alcènoyle comportant de un à six atomes de carbone ou aroyle tel que formyle, trifluoroacétyle, phtalyle
- p-toluènesulfonyle,
- p-nitrosulfényle,

de préférence $R_9$ sera le groupement tertiobutyloxycarbonyle.

Cette condensation est effectuée comme décrit ci-dessus, pour transformer un composé de formule (V) en un composé de formule (I). On obtient un composé possédant la formule générale (VII). Ce composé est déprotégé pour obtenir l'amine libre de formule (VIII). Cette déprotection peut être réalisée par hydrolyse

- soit en milieu acide, en présence d'un acide minéral ou organique fort comme l'acide chlorhydrique, l'acide sulfurique l'acide fluorhydrique, l'acide bromhydrique, un acide sulfonique comme par exemple l'acide para-toluènesulfonique ou l'acide méthanesulfonique, l'acide acétique éventuellement substitué par un à trois atomes de chlore ou de fluor, l'acide formique ou tout autre acide convenable dans un solvant ou un mélange de solvants aqueux ou organique comme un acide carboxylique, un hydrocarbure aliphatique halogéné, un hydrocarbure aromatique éventuellement substitué par un groupe halogène ou hydroxy, un alcool aliphatique éventuellement substitué par un ou plusieurs atomes d'halogènes comme par exemple l'éthanol ou le trifluoro-éthanol ou encore un ether aliphatique linéaire ou cyclique comme par exemple le diméthoxy-1,2-éthane, le dioxanne ou le tétrahydrofuranne. La méthode d'hydrolyse acide préférée consiste à dissoudre le composé dans une solution à environ 10 % d'acide trifluoro-acétique dans le chlorure de méthylène, et à agiter durant une période de quelques minutes à quelques heures à une température comprise entre zéro degrés centigrades et la température de reflux du mélange réactionnel.

- soit en milieu basique dans certains cas comme par exemple le groupe protecteur $R_9$ est OCOA et A est le groupe fluorènyl-9-méthyle, le solvant utilisé étant un solvant ou un mélange de solvants aqueux ou organique comme un hydrocarbure aliphatique halogéné, un solvant dipolaire protique ou aprotique comme par exemple le diméthyl-formamide, le diméthylsulfoxyde, la tétraméthylènesulfone (sulfolane) la N-méthylpyrrolidone, l'acétonitrile ou le N,N-diméthylacétamide ; un alcool aliphatique, un ester d'alcool aliphatique d'acide carboxylique, un ether linéaire ou cyclique ; l'agent basique pouvant être une base minérale comme un hydroxyde de métal alcalin ou organique comme une amine tertiaire aliphatique telle que par exemple la triéthylamine, la diisopropyléthylamine, la N-méthylpyrrolidine ou encore la N-méthylmorpholine.
- soit encore par hydrogènation catalytique, le catalyseur utilisé pouvant être un métal noble comme par exemple du palladium ou encore un oxyde de l'un de ces métaux déposé sur un support ; la nature du catalyseur convenable variant avec la nature du groupe $R_9$ ; quand $R_9$ est AOCO et A est benzyle le catalyseur peut être du palladium sur charbon.

Le dérivé d'amino-acide obtenu de formule (VIII) est séparé en ses distéréoisomères par chromatographie, pour donner les deux isomères de l'amine de formule (VIII).

Par la dégradation d'Edman, on revient aux deux énantiomères (R) et (S) de l'amine (V).

La dégradation d'Edman consiste

- à faire réagir un arylisothiocyanate de formule $R_{10}$-N = C = S sur l'amine libre de formule (VIII) optiquement active pour obtenir la thiourée de formule (IX) ; le groupe $R_{10}$ étant un radical aryle tel que phényle ou naphtyle éventuellement substitué par un ou plusieurs substituants choisis parmi les halogènes, le groupe méthoxy ou un groupement alkyle inférieur ayant de 1 à 6 atomes de carbone. La réaction est effectuée dans un solvant ne pouvant pas réagir avec la fonction isothiocyanate tel qu'un hydrocarbure aliphatique halogéné, un hydrocarbure aromatique éventuellement substitué par un groupe halogène un ester aliphatique d'un alcool aliphatique un solvant dipolaire aprotique comme par exemple le diméthyl-formamide, le diméthylsulfoxyde, la tétraméthylènesulfone (sulfolane) la N-méthylpyrrolidone, l'acétonitrile ou le N,N-diméthylacétamide ou encore un ether aliphatique linéaire ou cyclique comme par exemple le dioxanne ou le tétrahydrofuranne. Habituellement on préfère effectuer cette réaction dans du chlorure de méthylène, à une température comprise entre zéro degré et la température de reflux du mélange réactionnel, la réaction étant complète au bout d'une période de temps comprise entre quelques minutes et quelques heures.
- puis à cycliser et couper la thiourée obtenue de formule (IX) pour donner l'amine de formule (V) optiquement active. Ceci peut se faire en une ou deux étapes, mais on préfère habituellement réaliser les deux étapes sans isoler le composé cyclique intermédiaire ce qui évite une purification. On opère généralement en dissolvant la thiourée dans cinq à cent volumes d'une solution de concentration comprise entre cinq et cent pour cent d'un acide fort comme par exemple l'acide trifluoro-acétique dans un solvant organique comme par exemple un hydrocarbure halogéné, de préférence du chlorure de méthylène et en agitant cette solution à une température comprise entre zéro degré et la température de reflux du mélange réactionnel durant une période comprise entre quelques minutes et quelques heures.

La séparation des isomères de l'amine de formule (V) peut aussi se faire par salification, cristallisation puis filtration du sel obtenu. Le procédé consiste à dissoudre l'amine de formule (V), racémique, dans une solution d'acide optiquement actif, connu pour séparer des bases organiques, comme par exemple l'acide mandèlique, l'acide dibenzoyltartrique, l'acide di-p-toluoyltartrique, l'acide camphosulfonique, l'acide p-nitrobenzoylglutamique, l'acide tartrique, l'acide binaphtylphosphorique, dans un solvant aqueux ou organique comme par exemple un alcool aliphatique inférieur, l'acétone, l'acétonitrile ou tout autre solvant dans lequel un seul des deux sels diastéréoisomères recristallise.

On préfère employer l'acide L-tartrique ou l'acide (+)-binaphtylphosphorique et l'acétone ou l'acétonitrile comme solvant.

Les produits intermédiaires de formule (XI) :

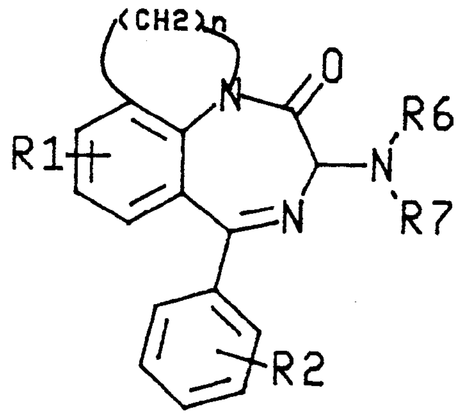

(XI)

n, $R_1$ et $R_2$ ayant les significations indiquées à la revendication 1, $R_6$ étant hydroxy quand $R_7$ représente une liaison supplémentaire entre l'atome d'azote portant $R_6$ et le cycle diazépine et $R_6$ étant l'hydrogène quand $R_7$ est l'hydrogène, sont des intermédiaires utiles pour la préparation des produits actifs suivant l'invention.

L'invention vise également un médicament pour lutter contre les troubles gastro-intestinaux, du pancréas et de la vésicule biliaire, les troubles du système nerveux central et la douleur, caractérisé en ce qu'il comprend une benzodiazépine suivant l'invention.

Les exemples suivants illustrent l'invention.

Sauf mention contraire, les méthodes utilisées lors des synthèses et des analyses sont les suivantes :

Les points de fusion ont été repérés en tube capillaire sur un appareil Mettler et n'ont pas été corrigés. Les spectres de résonance magnétique nucléaire ont été enregistrés sur un spectromètre JEOL FX-90Q (90 Mhz) le tétraméthylsilane étant utilisé comme référence interne. Les spectres sont décrits de la manière suivante : déplacement chimique (exprimé en ppm par rapport à la référence interne), multiplicité, intensité de l'intégration, éventuellement constante découplage et attribution. Les spectres infrarouges ont été enregistrés en pastille de bromure de potassium sur un spectro photomètre Shimadzu IR-435. Les chromatographies flash ont été effectuées telles que décrites par Still sur gel de silice (article E. MERCK 4063) (Still W.C., Kahn M., Mitra A., J. Org. Chem. (1978), 43, 2923). Les chromatographies sur couche mince ont été effectuées sur plaque de silice 60 F-254 de 0.25 mm d'épaisseur (article E. Merck 5714). Les plaques sont examinées sous lumière ultraviolette ou révélées par de l'iode, du réactif de Dragendorf ou du réactif à la toluidine. Les chromatographies sous haute pression (HPLC) sont effectuées sur un appareil Jobin-Yvon.

EXEMPLE 1 :

(Indol-2-ylcarbonylamino)-4-phényl-6-tétrahydro-1,2,3,4-pyrrolo[3,2,1-jk]benzodiazépin-[1,4]-one-3.

Formule I ; Ar = Indol-2-yl, $R_1$ = $R_2$ = Hydrogène. (Ia):

stade-(a)

Transformation d'un composé de formule (III) en un composé de formule (IV).

Dans un ballon tricol maintenu sous atmosphère d'azote, on introduit une solution de Phényl-6-tétrahydro-1,2,3,4-pyrrolo[3,2,1-j,k]benzodiazépin-[1,4]-on-3 (26.3 g, 0.10 mole) dans un mélange de toluène anhydre (500 ml) et de tétrahydrofuranne anhydre (250 ml). La solution est refroidie à -20°C et on y ajoute du tertiobutylate de potassium anhydre (37.1 g, 0.33 mole), la solution devient brun-rouge sombre. Après 30 minutes d'agitation à -20°C on ajoute du nitrite d'isoamyle (20.1 ml, 0.15 mole) et on laisse le mélange revenir à la température ambiante à laquelle on l'agite durant 30 minutes. On ajoute alors une solution d'acide acétique (50 ml) dans de l'eau (500 ml). La phase organique est décantée et la phase aqueuse est réextraite par du chlorure de méthylène (3 fois 200 ml). Les extraits organiques rejoints sont évaporés sous pression réduite et le résidu purifié par chromatographie flash sur une colonne de silice, l'éluant utilisé étant une mélange de polarité croissante d'acétone dans du chlorure de méthylène. On obtient ainsi 23.3 g d'Hydroxyimino-4-phényl-6-tétrahydro-1,2,3,4 pyrrolo[3,2,1-j,k]benzodiazépin-[1,4]-one-3 sous forme d'un solide jaune (rendement 80%). Point de fusion : 215°C (décomposition).
Spectre de résonance magnétique nucléaire.

| | | | | | |
|---|---|---|---|---|---|
| 11.20 ppm | s | 1 proton | | OH |
| 7.80-7.00 ppm | m | 8 protons | | aromatiques |
| 4.35 ppm | t | 2 protons | J = 8 hz | CH2-N |
| 3.20 ppm | t | 2 protons | J = 8 hz | CH2 benzylique |

Spectre infra-rouge.
Bandes à 3300, 3050, 2800, 1665, 1620, 1600, 1570, 1525, 1515, 1340, 1215, 1150 et 1010-950 cm-1.

stade-(b)

transformation d'un composé de formule (IV) en un composé de formule (V).

1° méthode

Dans un ballon tricol relié à un réservoir d'hydrogène maintenu à une pression légèrement supérieure à la pression atmosphérique on introduit une suspension de nickel de Raney (100 g) et d'oxime de l'étape 1a (23.2 g) dans du méthanol (1000 ml). La suspension est agitée à la température ambiante jusqu'à ce que l'absorption d'hydrogène soit terminée (environ 20 heures). La suspension est filtrée et lavée avec cinq fois 200 ml de méthanol. Le méthanol est évaporé sous pression réduite pour donner un résidu huileux visqueux qui est purifié par chromatographie flash sur une colonne de silice, l'éluant utilisé étant un mélange de polarité croissante d'acétone dans du chlorure de méthylène. On obtient ainsi 11 g d'Amino-4-phényl-6-tétrahydro-1,2,3,4-pyrrolo[3,2,1-jk]benzodiazépin-[1,4-]one-3 (rendement 58%) sous forme d'huile qui est engagée immédiatement dans la réaction suivante.
Spectre de résonance magnétique nucléaire.

| | | | | |
|---|---|---|---|---|
| 7.65-7.00 ppm | m | 8 | protons | aromatiques |
| 4.80-4.20 ppm | m | 2 | protons | CH2-N |
| 4.20-3.60 ppm | m | 3 | protons | CH-NH2 |
| 3.40-2.80 ppm | m | 2 | protons | CH2Ar |

2°méthode.

Dans un réacteur pouvant supporter une pression de 12 bars, on introduit sous azote 9,6 g de ruthénium sur charbon à 5%. On hydrogène le ruthénium pendant 2 heures à 20°C sous 10 bars d'hydrogène. On ajoute sous atmosphère d'azote 32g (110 mmoles) d'hydroximino-4-phényl-6-tétrahydro-1,2,3,4-pyrrolo[3,2,1-j,k]benzodiazépin-[1,4]-one-3. On porte à nouveau le réacteur à une pression de 8 bars d'hydrogène et chauffe peu à peu jusqu'à 72°C en deux heures. On maintient cette température pendant encore une heure. On refroidit, on filtre sur silice et rince par du méthanol. Après chromatographie sur silice dans du chlorure de méthylène enrichi en acétone on obtient 29 g (rendement : 94 %) d'amino-4-phényl-6-tétrahydro-1,2,3,4-pyrrolo [3,2,1-j,k] benzodiazépin-[1,4]-one-3.
Formule (V), n = 2, $R_1$ = H, $R_2$ = H ; (Va).
C.C.M. : H2CCl2/MeOH 95/5 Rf = 0,2
R.M.N. :

| | | | | |
|---|---|---|---|---|
| 7.65-7.0 | ppm | 8 | protons | aromatiques |
| 4.80-4.20 | ppm | 2 | protons | CH2N |
| 4.20-3.60 | ppm | 3 | protons | CH-NH2 |
| 3.40-2.80 | ppm | 2 | protons | CH2Ar |

I.R. : 3350, 1675, 1600, 1560, 1440, 1340, 1240, 1100, 730, 690 cm-1.

stade-(1c)

transformation d'un composé de formule (V) en un composé de formule (I).

Dans un ballon sec et maintenu à l'abri des l'humidité par une garde à chlorure de calcium et à l'abri de l'oxygène par un courant d'azote, on introduit une solution de l'amine préparée en 1b (11.0 g, 39.7 mmoles) dans du chlorure de méthylène anhydre (200 ml). On y ajoute du chlorure de l'acide Indolyl-2-carboxylique (7.12 g, 39.6 mmoles). Le mélange est agité à la température ambiante et l'on y ajoute goutte à goutte de la triéthylamine (4.01 g, 39.6 mmoles).

L'avancement de la réaction est suivi par chromatographie sur couche mince. Après disparition du produit de départ (environ une heure de réaction) le milieu réactionnel est dilué par du chlorure de méthylène (200 ml) et lavé successivement par de l'acide chlorhydrique normal (200 ml), une solution saturée de bicarbonate de sodium (200 ml) et de l'eau (200 ml). La phase organique est alors évaporée sous pression réduite et le résidu purifié par chromatographie flash sur une colonne de silice, l'éluant utilisé étant un mélange de polarité croissante d'acétone dans du chlorure de méthylène. On obtient ainsi 12.6 g (rendement 75.5 %) du composé (Ia) sous forme d'un solide blanc. Point de fusion 218°C.

Spectre de résonance magnétique nucléaire (enregistré sur un spectromètre Brücker, à 250 Mhz).

| | | | | | | |
|---|---|---|---|---|---|---|
| 11.66 | ppm | s | 1 | proton | | NH indolique |
| 9.58 | ppm | d | 1 | proton | J = 8 hz | NH amide |
| 7.65 | ppm | m | 2 | protons | | H aromatiques |
| 7.50 | ppm | m | 6 | protons | | H aromatiques |
| 7.20 | ppm | m | 4 | protons | | H aromatiques |
| 7.06 | ppm | t | 1 | proton | J = 7 hz | H aromatiques |
| 5.55 | ppm | d | 1 | proton | J = 8 hz | CH-NH |
| 4.51 | ppm | m | 1 | proton | | CH2-N |
| 3.96 | ppm | m | 1 | proton | | CH2-N |
| 3.41 | ppm | m | 1 | proton | | CH2 benzylique |
| 3.34 | ppm | m | 1 | proton | | CH2 benzylique |

Spectre infrarouge.

Bandes à 3300, 3260, 3040, 2800, 1660, 1635, 1600, 1530, 1395, 1340, 1300, 1250 et 1210 cm-1.

EXEMPLE 2 :

(Chloro-4-benzoylamino)-4-phényl-6-tétrahydro-1,2,3,4-pyrrolo[3,2,1-jk]benzodiazépin-[1,4]-one-3.

Formule I ; Ar = Chloro-4-phényl, $R_1$ = $R_2$ = Hydrogène. N = 2 (Ib) :

Ce composé est préparé à partir de l'amine préparée dans l'exemple-1 stade-(b) de la manière suivante :

Dans un ballon sec et maintenu à l'abri de l'humidité par une garde à chlorure de calcium, on introduit une solution de l'amine préparée dans l'exemple-1 stade-(b) 4.7 g, 16.9 mmoles) dans du chlorure de méthylène anhydre (100 ml). On y ajoute du chlorure de l'acide para-chlorobenzoique (2.97 g, 17.0 mmoles). Le mélange est agité à la température ambiante l'on y ajoute goutte à goutte de la triéthylamine (1.72 g, 17.0 mmoles). L'avancement de la réaction est suivi par chromatographie sur couche mince. Après disparition du produit de départ (environ une heure de réaction) le milieu réactionnel est dilué par du chlorure de méthylène (200 ml) et lavé successivement par de l'acide chlorhydrique normal (100 ml), une solution saturée de bicarbonate de sodium (100 ml) et de l'eau (100 ml). La phase organique est alors évaporée sous pression réduite et le résidu purifié par chromatographie flash sur une colonne de silice, l'éluant utilisé étant un mélange de polarité croissante d'acétone dans du chlorure de méthylène. On obtient ainsi 5.7 g (rendement 81 %) du composé Ib sous forme d'un solide blanc. Point de fusion : 215°C (décomposition).

C.C.M. : H2CCl2/ Acétone 95/5 Rf = 0,64.

Spectre de résonance magnétique nucléaire :

| | | | | | | |
|---|---|---|---|---|---|---|
| 8.08 | ppm | d | 1 | proton | J = 8hz | CH-NH-CO |
| 7.96 | ppm | m | 2 | protons | | H aromatiques |
| 7.70-7.00 | ppm | m | 10 | protons | | H aromatiques |
| 5.60 | ppm | d | 1 | proton | J = 8hz | CH-NH-CO |
| 4.80-4.50 | ppm | m | 1 | proton | | CH2-N |
| 4.30-3.70 | ppm | m | 1 | proton | | CH2-N |
| 3.60-2.80 | ppm | m | 2 | protons | | CH2 benzylique |

Spectre infra-rouge :
Bandes à 3300, 3050, 2900, 1690, 1650, 1590, 1565, 1530, 1490, 1440, 1385, 1340, 1270, 1130, 1080 et 1000 cm-1.

EXEMPLE 3 :

(Iodo-3-benzoylamino)-4-phényl-6-tétrahydro-1,2,3,4-pyrrolo[3,2,1-j,k]benzodiazépin-[1,4]-one-3.

Formule I ; Ar = Iodo-3-phényle, $R_1$ = $R_2$ = Hydrogène. (Ic):

Ce composé est préparé à partir de l'amine préparée dans l'exemple-1 stade-(b) de la manière suivante :
Dans un ballon sec et maintenu à l'abri de l'humidité par une garde à chlorure de calcium, on introduit une solution de l'amine (3g, 10.8 mmoles) dans du chlorure de méthylène anhydre (100 ml). On y ajoute du chlorure de l'acide meta Iodobenzoique (3.14 g, 11.8 mmoles). Le mélange est agité à la température ambiante et l'on y ajoute goutte à goutte de la triéthylamine (1.20 g, 11.9 mmoles). L'avancement de la réaction est suivi par chromatographie sur couche mince. Après disparition du produit de départ (environ une heure de réaction) le milieu réactionnel est dilué par du chlorure de méthylène (200 ml) et lavé successivement par de l'acide chlorhydrique normal (100 ml), une solution saturée de bicarbonate de sodium (100 ml) et de l'eau (100 ml). La phase organique est alors évaporée sous pression réduite et le résidu purifié par chromatographie flash sur une colonne de silice, l'éluant utilisé étant un mélange de polarité croissante d'acétone dans du chlorure de méthylène. On obtient ainsi 4.0 g (rendement 73 %) du composé Ic sous forme d'un solide blanc. Point de fusion : 258°C.
C.C.M. : H2CCl2/Acétone 95/5 Rf = 0,55.
Spectre de résonance magnétique nucléaire :

| | | | | | | |
|---|---|---|---|---|---|---|
| 9.70 | ppm | d | 1 | proton | J = 8hz | CH-NH-CO |
| 8.46 | ppm | s | 1 | proton | | H aromatiques |
| 8.30-7.80 | ppm | m | 3 | protons | | H aromatiques |
| 7.70-7.10 | ppm | m | 8 | protons | | H aromatiques |
| 5.50 | ppm | d | 1 | proton | J = 8hz | CH-NH-CO |
| 4.70-4.40 | ppm | m | 1 | proton | | CH2-N |
| 4.10-3.70 | ppm | m | 1 | proton | | CH2-N |
| 3.60-3.00 | ppm | m | 2 | protons | | CH2 benzylique |

Spectre infrarouge :
Bandes à 3200, 3050, 3000, 1685, 1640, 1600, 1560, 1520, 1465, 1440, 1430, 1380, 1340, 1285, 1210 et 1190 cm-1.

EXEMPLE-4

Phényl-6-(triméthoxy-3,4,5-benzoylamino-)-4-tétrahydro-1,2,3,4-pyrrolo [3,2,1-j,k] benzodiazépin-[1,4]-one-3.

Formule (I) ; Ar = triméthoxy-3,4,5-phényle, $R_1$ = $R_2$ = hydrogène, n = 2. (Id).

Ce composé est préparé à partir de 5 g (18 mmoles) de l'amine décrite dans l'exemple (1) stade (b) de la même façon que dans l'exemple (2) stade (c). A partir de 3,97 g (18 mmoles) de chlorure de l'acide triméthoxy-3,4,5-benzoique et de 1.8 g (18 mmoles) de triéthyle amine dans 200 ml de chlorure de méthylène. Après chromatographie on obtient 8 g (94%) du composé (1d), solide blanc cristallisant sous forme de solvate contenant un tiers de molécule d'eau. F = 158°c.
R.M.N. :

| | | | | | | |
|---|---|---|---|---|---|---|
| 8.0 | ppm | d | 1 | proton | J = 8Hz | CH-NH-CO |
| 7,60-7,0 | ppm | m | 10 | protons | | aromatiques |
| 5,62 | ppm | d | 1 | proton | J = 8Hz | CH-NH-CO |
| 4,55 | ppm | m | 1 | proton | | CH2N |
| 4.15-3,75 | ppm | m | 1 | proton | | CH2N |
| 3,90 | ppm | s | 9 | protons | | CH3O |
| 3.25 | ppm | m | 2 | protons | | CH2Ar |

I.R. : 3320, 2950, 1680, 1640, 1530, 1495, 1310, 1250, 1120, 1000, 760, 730, 700 cm-1.

EXEMPLE-5

Préparation des (-)-(4S) et (+)-(4R)-[indolyl-2-carbonyl-amino]-4-phényl-6-tétrahydro-1,2,3,4-pyrrolo [3,2,1-j, k]benzodiazépin-[1,4]-one-3.

Formule (I) ; Ar = indolyl-2- ; $R_1$ = $R_2$ = H ; n = 2 ; (Ie)-isomère lévogyre et (If)-isomère dextrogyre.

Stade-(a).

Préparation de la Phényl-6-[N-(N-tertiobutyloxy-carbonyl-L-leucyl-)-amino]-4-tétrahydro-1,2,3,4-pyrrolo[3,2,1- j,k]benzodiazépine-[1,4]-one-3.

Formule (VII) ; $R_1$ = $R_2$ = H ; n = 2 ; $R_8$ = isobutyl $R_9$ = tertiobutyloxy-carbonyle.

Dans un ballon tricol équipé d'une garde à chlorure de calcium, d'un thermomètre plongeant et d'une arrivée d'azote, on dissout à 5°C 19 g (68,5 mmoles) de l'amine préparée dans l'exemple (1) stade (b) dans 500 ml de chlorure de méthylène. On ajoute 17,1 g (68.5 mmoles) d'hydrate to Boc-L-Leucine et 10,5 g (68,5 mmoles) d'hydrate d'hydroxy-1-benzotriazole. On ajoute ensuite goutte à goutte une solution de 500 ml de chlorure de méthylène contenant 14.1 g (68,5 mmoles) de dicyclohexyl-carbodiimide. On agite pendant 2 heures à 5°C et on laisse revenir à température ambiante. Après 20 h on filtre l'insoluble et on le rince abondamment au chlorure de méthylène. On évapore et chromatographie le résidu sur une colonne de silice en éluant par du chlorure de méthylène enrichi en acétone. Obtenu 32,5 g (97%) ;
C.C.M. : H2CCl2/Acétone 5 % Rf = 0,8.
R.M.N. :

| | | | | | | |
|---|---|---|---|---|---|---|
| 7.75 | ppm | d | 1 | proton | J = 8Hz | CH-NH-CO |
| 7,60-6,90 | ppm | m | 8 | protons | | aromatiques |
| 5,42 | ppm | d | 1 | proton | J = 8Hz | CH-NH-CO |
| 5,10 | ppm | d | 1 | proton | | NH-CH-CO |
| 4,60 | ppm | m | 1 | proton | | CH2N |
| 3,95 | ppm | m | 1 | proton | | CH2N |
| 3,70-1,70 | ppm | m | 3 | protons | | CH2N,NH-CH-CO |
| 3,25 | ppm | m | 2 | protons | | CH2Ar |
| 1,90-1,20 | ppm | m | 3 | protons | | CHCH2 |
| 1,45 | ppm | s | 9 | protons | | C(CH3) |
| 1,0 | ppm | d | 6 | protons | | (CH3)2 |

I.R. : 3300, 2900, 1710-1650, 1500, 1440, 1160, 1020 cm-1.

Stade-(b)

Préparation de la N-(-L-leucyl-)-amino-4-phényl-6-tétrahydro-1,2,3,4-pyrrolo[3,2,1-j,k]benzodiazépine-[1,4]-one-3.

Formule (VIII) ; $R_1 = R_2 = H$ ; $R_8$ = isobutyl ; n = 2.

32,5 g (66 mmoles) du produit du stade précédent sont dissous dans 300 ml de chlorure de méthylène. On refroidit dans de la glace et ajoute en une minute mais sans laisser monter la température au dessus de 5°C, 300 ml d'acide trifluoroacétique. Après 1/2 h d'agitation, on évapore à sec. On reprend par de l'acétate d'éthyle et de la soude normale, puis par de l'eau saturée de chlorure de sodium. On sèche et évapore. On chromatographie sur silice, en éluant par un mélange de polarité croissante de méthanol dans du chlorure de méthylène. Obtenu : 24 g (72 %).

Pour obtenir la base :

25 g de sel (trifluoroacétate ; 50 mmoles) sont agités violemment avec 200 ml de soude normale et 200 ml d'acétate d'éthyle. On décante, extrait la soude par 50 ml d'acétate d'éthyle, lave l'acétate d'éthyle par 50 ml de solution aqueuse saturée de chlorure de sodium. On sèche, évapore : 19 g (97%).

CCM. : AcOEt/MeOH 90/10 Rf = 0,3;0,4 (2 taches, correspondant aux 2 diastéréoisomères).

R.M.N. :

| | | | | | |
|---|---|---|---|---|---|
| 8,72 | ppm | d | 1 | proton | CH-NH-CO |
| 7,60-6,90 | ppm | m | 8 | protons | aromatiques |
| 5.42 | ppm | d | 1 | proton | CH-NH-CO |
| 4,40 | ppm | m | 1 | proton | CH2N |
| 3,95 | ppm | m | 1 | proton | CH2N |
| 3,60-3.0 | ppm | m | 3 | protons | CO-CH-NH, CH2Ar |
| 2.0 -1,20 | ppm | m | 3 | protons | CHCH2 |
| 1.70 | ppm | s | 2 | protons | NH2 |
| 0.95 | ppm | m | 6 | protons | (CH3)2 |

I.R.: 3350, 2950, 1660, 1600, 1440, 1380, 1240 cm-1.

Stade-(c).

Séparation des isomères optiques.

On monte un dispositif de chromatographie flash avec 400 g de silice, dans une colonne de 5 cm de diamètre. On dissout le produit dans de l'acétate d'éthyle et élue successivement par de l'acétate d'éthyle pur (2,5 l), de l'acétate d'éthyle contenant 5 % de méthanol (2,5 l), de l'acétate d'éthyle contenant 10 % de méthanol (2,5 l), de l'acétate d'éthyle contenant 20 % de méthanol (2,5 l) ; on examine sur plaque de silice, joint les fractions identiques, on évapore.

On recueille :

fractions 8-14 bon produit, isomère A, 11,5 g
fractions 15-16-17 mélange d'isomères, 0,4 g
fractions 18-27 isomère B, 11,5 g

Rendement de la séparation : 96 % (48 % de chacun des deux isomères).

Isomère A : C.C.M. : AcOEt/MeOH 90/10 Rf = 0,5
[αD] = -91°5 (c = 1,0/H2CCl2)
Isomère B : C.C.M. : AcOEt/MeOH 90/10 Rf = 0,3
[αD] = + 56°3 (c = 1,0/H2CCl2)

Stade-(d)

Préparation de la Phényl-6-N-[-(N-phénylthiouréido-)-L-leucyl-]-amino-4-tétrahydro-1,2,3,4-pyrrolo[3,2,1-j,k]benzodiazépine-[1,4]-one-3.

Formule (IX) ; $R_1$ = $R_2$ = H ; $R_8$ = isobutyl ; $R_{10}$ = phényl ; n = 2 ; Isomère A.

Dans une solution agitée de 200 ml de chlorure de méthylène contenant 11,7 g (30 mmoles) de l'isomère A du stade précédent on ajoute goutte à goutte 4,05 g (30 mmoles) d'isothiocyanate de phényle. On suit sur plaque l'avancement de la réaction. Après une heure on évapore : 15,5 g. On reprend le résidu par du chlorure de méthylène contenant 10 % d'acétate d'éthyle et chromatographie sur silice dans le chlorure de méthylène enrichi en acétate d'éthyle. On évapore le solvant des fractions contenant le produit. Obtenu : 15 g (95%).
C.C.M. : H2CCl2/AcOEt 85/15 Rf = 0,4.

Stade-(e).

(-)-(4S)-Amino-4-phényl-6-tétrahydro-1,2,3,4-pyrrolo[3,2,1-j,k]benzodiazépin-[1,4]-one-3.

Formule (Va) ; isomère lévogyre.

On dissout 5,8 g (30 mmoles) de phényl-6-N-(-(N-phénylthiouréido-)-L-leucyl-)-amino-4-tétrahydro-1,2,3,4-pyrrolo[3,2,1- j,k] benzodiazépine-[1,4]-one-3 obtenue au stade (d) dans 200 ml d'acide trifluoroacétique en agitant à température ordinaire. On chauffe une heure à 40°C et évapore. On chasse plusieurs fois du chlorure de méthylène pour éliminer l'excès d'acide trifluoroacétique. On chromatographie sur silice en éluant par de l'acétate d'éthyle enrichi progressivement en méthanol. On obtient 8 g (68 %) d'aiguilles blanches de trifluoroacétate de (-)-(S,4)-amino-4-phényl-6-tétrahydro-1,2,3,4-pyrrolo[3,2,1-j,k] benzodiazépin-[1,4]-one-3.
CCM : H2CCl2/AcOEt 85/15 Rf = 0,4.
R.M.N. :

| | | | | | |
|---|---|---|---|---|---|
| 7,60-6,90 | ppm | m | 8 | protons | aromatiques |
| 4,65 | ppm | m | 1 | proton | CH2N |
| 4,45 | ppm | s | 1 | proton | CHNH3 |
| 3,90 | ppm | m | 1 | proton | CH2N |
| 3,50-2,90 | ppm | m | 5 | protons | NH3,CH2Ar |

Stade-(f).

(-)-(4S)-[(indolyl-2)-carbonylamino]-4-phényl-6-tétrahydro-1,2 ,3 ,4-pyrrolo[3,2,1-j,k]benzodiazépin-[1,4]-one-3.

Formule-(Ie) isomère lévogyre.

Dans un ballon sec maintenu à l'abri de l'humidité par une garde à chlorure de calcium, et à l'abri de l'oxygène par un courant d'azote, on introduit une solution de 11 g l-(S)-amino-4-phényl-6-tétrahydro-1,2,3,4-pyrrolo[3,2,1-j,k]benzodiazépin-[1,4]-one-3 (39,7 mmoles) dans 200 ml de chlorure de méthylène anhydre. On ajoute 7,12 g (39,6 mmoles) de chlorure de l'acide indole-2 carboxylique et le mélange est agité à la température ambiante. On ajoute goutte à goutte la pyridine (3,13 g, 39,6 mmoles) ; et l'avancement de la réaction est suivi par chromatographie sur couche mince.

Après disparition du produit de départ (environ une heure de réaction), le milieu réactionnel est dilué par du chlorure de méthylène (200 ml) et lavé successivement par de l'acide chlorhydrique normal (200 ml), une solution saturée de bicarbonate de sodium (200 ml) et de l'eau (200 ml). La phase organique est séchée et évaporée sous pression réduite, le résidu est purifié par chromatographie sur une colonne de silice, l'éluant utilisé étant un mélange de polarité croissante d'acétone dans de l'acétate d'éthyle. On obtient 12,6 g (75,5 %) de solide blanc. F = 206°C.[αD] = -56°(H2CCl2).
C.C.M. : H2CCl2/Acétone 95/5 Rf = 0,5.
R.M.N. :

| | | | | | | |
|---|---|---|---|---|---|---|
| 11,6 | ppm | s | 1 | proton | | NH indolique |
| 9,58 | | d | 1 | proton | J = 8Hz | CH-NH-CO |
| 7,65-7,06 | ppm | m | 13 | protons | | aromatiques |
| 5,55 | ppm | d | 1 | proton | J = 8Hz | CH-NH-CO |
| 4,51 | ppm | m | 1 | proton | | CH2N |
| 3,96 | ppm | m | 1 | proton | | CH2N |
| 3,41 | | m | 1 | proton | | CH2Ar |
| 3,34 | | m | 1 | proton | | CH2Ar |

I.R. : 3300, 3260, 3040, 2800, 1660, 1635, 1600, 1530, 1395, 1340, 1300, 1250, 1210 cm-1.

Stade-(g).

Préparation de la phényl-6-N-[-(N-phénylthiouréido-)-L-leucyl-]-amino-4-tétrahydro-1,2,3,4-pyrrolo[3,2,1-j,k]benzodiazépine-[1-4]-one-3.

Formule-(IX) isomère B.

Dans une solution agitée de 200 ml de chlorure de méthylène contenant 11,7 g (30 mmoles) de l'isomère B du stade (c) on ajoute goutte à goutte 4,05 g (30 mmoles) d'isothiocyanate de phényle. On suit sur plaque l'avancement de la réaction. Après une heure, on évapore le solvant. Le résidu (15,5 g) est repris par du chlorure de méthylène contenant 10 % d'acétate d'éthyle et chromatographie sur silice dans le chlorure de méthylène enrichi en acétate d'éthyle. On joint les fractions contenant le produit. Obtenu : 15 g (95%).
C.C.M. : H2CCl2/AcOEt 95/15 Rf = 0,4 .

Stade-(h).

(+)-(4R)-amino-4-phényl-6-tétrahydro-1,2,3,4-pyrrolo[3,2,1-j,k]benzodiazépin-1,4-one-3.

Formule (Va) : isomère dextrogyre.

On dissout 5,8 g (30 mmoles) de phényl-6-N-(-N-phénylthiouréido-)-L-leucyl-)-amino-4-tétrahydro-1,2,3,4-pyrrolo [3,2,1-j,k]benzodiazépine-[1,4]-one-3 obtenu au stade (g) dans 200 ml d'acide trifluoroacétique en agitant à température ordinaire. On chauffe une heure à 40°C et évapore. Le résidu est redissous puis tiré à sec plusieurs fois successives pour éliminer l'excès d'acide trifluoroacétique. Il est alors chromatographié sur silice en éluant par de l'acétate d'éthyle enrichi progressivement en méthanol. On obtient 8 g (68 %) d'aiguilles blanches de trifluoroacétate de (+)-(R,4)-amino-4-phényl-6-tétrahydro-1,2,3,4-pyrrolo[3,2,1-j,k]benzodiazépin-[1,4]-one-3.
CCM : H2CCl2/AcOEt 85/15 Rf = 0,4 Stade-(i).
(+)-(4R)-[(indolyl-2)-carbonylamino]-4-phényl-6-tétrahydro-1,2,3,4-pyrrolo[3,2,1-j,k]benzodiazépin-[1,4]-one-3
.

Formule (If) ; isomère dextrogyre.

Dans un ballon sec maintenu à l'abri de l'humidité par une garde à chlorure de calcium, et à l'abri de l'oxygène par un courant d'azote, on introduit une solution de 11 g d-(4R)-amino-4-phényl-6-tétrahydro-1,2,3,4-pyrrolo [3,2,1-j,k]benzodiazépin-[1,4]-one-3 (39,7 mmoles) dans 200 ml de chlorure de méthylène anhydre. On ajoute 7,12 g (39,6 mmoles) de chlorure de l'acide indole-2 carboxylique et le mélange est agité à la température ambiante. On ajoute goutte à goutte la pyridine (3,13 g, 39,6 mmoles) ; et l'avancement de la réaction est suivi par chromatographie sur couche mince .

Après disparition du produit de départ (environ une heure de réaction) le milieu réactionnel est dilué par du chlorure de méthylène (200 ml) et lavé successivement par de l'acide chlorhydrique normal (200 ml), une solution saturée de bicarbonate de sodium (200 ml) et de l'eau (200 ml). La phase organique est séchée et évaporée sous pression réduite, le résidu est purifié par chromatographie sur une colonne de siline, l'éluant utilisé étant un mélange de polarité croissante d'acétone dans de l'acétate d'éthyle. On obtient 12,6 g (75,5 %) de solide blanc. F = 206°C. [αD] = +56°(c = 1,0/H2CCl2).
C.C.M. : H2CCl2/Acétone 95/5 Rf = 0,5.

EXEMPLE-6

Préparation de la(fluoro-2-phényl)-6-[(indolyl-2)-carbonylamino]-4-tétrahydro-1,2,3,4-pyrrolo [3,2,1-j,k]benzodiazépin-[1,4]-one-3.

Formule (I) ; Ar = indolyl-2; $R_1$ = H; $R_2$ = F-2; n = 2; (Ig).

Stade-(a).

Préparation de la (fluoro-2-benzoyl)-7-indoline.

Dans un réacteur muni d'une agitation centrale d'un réfrigérant équipé d'une garde à chlorure de calcium, d'un thermomètre plongeant, d'une arrivée d'azote, d'une ampoule à brome à rétablissement de pression, on dissout 43,03 g (361 mmoles) d'indoline dans 360 ml de tétrachloroéthane. On refroidit dans un bain de glace et ajoute goutte à goutte 46,75 g (399 mmoles) de trichlorure de bore en solution dans 180 ml de tétrachloroéthane. On ajoute 84g (694 mmoles) de fluoro-2-benzonitrile puis 52,2 g (399 mmoles) de trichlorure d'aluminium. On chauffe 8 h à 150°C. Après refroidissement on hydrolyse avec 325 ml d'acide chlorhydrique 4N. On chauffe encore 20 minutes à 80°C pour terminer l'hydrolyse. On laisse refroidir et filtre l'insoluble. On rince par de l'éther, sèche. On reprend le précipité par du chlorure de méthylène et alcalinise par de la soude. On lave la solution organique par une solution concentrée de chlorure de sodium, on sèche sur sulfate de sodium. Après filtration et évaporation on obtient 63 g de résine jaune (rendement = 72%).
C.C.M. : H2CCl2 Rf = 0,5.
R.M.N. :

| | | | | | | |
|---|---|---|---|---|---|---|
| 7,50-7,0 | ppm | m | 7 | protons | | aromatiques |
| 6,40 | ppm | m | 1 | proton | | NH |
| 3,80 | ppm | t | 2 | protons | J = 9Hz | CH2N |
| 3,0 | ppm | t | 2 | protons | J = 9Hz | CH2Ar |

Stade-(b).

Dans un réacteur muni d'une agitation centrale d'un réfrigérant équipé d'une garde à chlorure de calcium, d'un thermomètre plongeant, d'une arrivée d'azote, d'une ampoule à brome à rétablissement de pression, 117 g (485 mmoles) de produit du stade-(a) sont placés en suspension dans 2,5 l d'éther. On refroidit à -20°C et ajoute 41 ml (485 mmoles) de pyridine puis 91,6 g (582 mmoles) de chlorure de bromacétyl goutte à goutte. On laisse revenir à la température ambiante et agite 20 h. On ajoute 2 l d'eau sous agitation et filtre l'insoluble. On rince par de l'eau puis de l'hexane, on sèche. On recristallise dans de l'acétate d'éthyle. On obtient 120 g (rendement 61%) N-bromoacétyl-(fluoro-2-benzoyl-)-7-indoline. F = 136°C.
C.C.M. : H2CCl2/Acétone 95/5 Rf = 0,25.
R.M.N. :

| | | | | | |
|---|---|---|---|---|---|
| 7,90-6,90 | ppm | m | 7 | protons | aromatiques |
| 4,20 | ppm | t | 2 | protons | CH2 N |
| 3,76 | ppm | s | 2 | protons | CO-CH2Br |
| 3,20 | ppm | t | 2 | protons | CH2Ar |

I.R. : 1660, 1610, 1450, 1390, 1210, 1100, 1050, 745 cm-1.

Stade-(c).

Dans un réacteur muni d'une agitation centrale d'un réfrigérant équipé d'une garde à potasse, d'un thermomètre plongeant, d'une arrivée d'azote, d'une ampoule à brome à rétablissement de pression, on verse 1 l de tétrahydrofuranne et 570 ml de méthanol anhydre. Après avoir refroidi à -30°C on ajoute 320 ml d'ammoniac liquide. On ajoute ensuite, peu à peu 114 g (314 mmoles) de produit du stade précédent et laisse sous agitation jusqu'au lendemain, sans refroidir extérieurement. Tout se dissout lentement. Après 20 h on évapore à sec. On lave à l'eau. On obtient 140 g de produit brut que l'on chromatographie sur silice en éluant par du chlorure de méthylène progressivement enrichi en acétone. On obtient 70 g de (fluoro-2-phényl)-6-tétrahydro-1,2,3,4-pyrrolo [3,2,1-j,k]benzodiazépin-[1,4]-one-3 (rendement 79%). F = 148°C.
Formule (III) ; n = 2 ; $R_1$ = H ; $R_2$ = F-2 ; (IIIb).
C.C.M. : H2CCl2/Acétone 95/5 Rf = 0,6.
R.M.N. :

| | | | | | |
|---|---|---|---|---|---|
| 7,60-6,90 | ppm | m | 7 | protons | aromatiques |
| 4,40 | ppm | s | 2 | protons | CO-CH2-N |
| 4,30 | ppm | t | 2 | protons | CH2N |
| 3,20 | ppm | t | 2 | protons | CH2Ar |

I.R. : 1665, 1600, 1445, 1395, 1345, 1210, 740 cm-1.

Stade-(d).

Préparation de l'hydroximino-4-(fluoro-2-phényl)-6-tétrahydro-1,2,3,4-pyrrolo[3,2,1-j,k]benzodiazépin-[1,4]-one-3.

Formule (IV) ; n = 2 ; $R_1$ = H ; $R_2$ = F-2 ; (IVb).

De la même façon que dans l'exemple-1 stade-(a) , à partir de 70 g (249 mmoles) du produit du stade précédent on obtient 67 g (rendement 87 %) d'un solide jaune F = 218°C (dec.)
C.C.M. : H2CCl2/Acétone 95/5 Rf = 0,29.
R.M.N. :

| | | | | | |
|---|---|---|---|---|---|
| 7,80-6,95 | ppm | m | 7 | protons | aromatiques |
| 4,25 | ppm | t | 2 | protons | CH2 N |
| 3,40 | ppm | s | 3 | protons | NOH, H2O |
| 3,20 | ppm | t | 2 | protons | CH2Ar |

I.R. : 3450, 3120, 3000, 2700, 1620, 1600, 1340, 1300, 1000, 740 cm-1.

Stade-(e).

Préparation de l'amino-4-(fluoro-2-phényl)-6-tétrahydro-1,2,3,4-pyrrolo[3,2,1-j,k]benzodiazépin-[1,4]-one-3.

Formule (V) , n = 2, $R_1$ = H, $R_2$ = F-2 ; (Vb).

1° méthode.

De la même façon que dans l'exemple-1 stade-(b), 2° méthode à partir de 30 g (969 mmoles) du produit du stade précédent on obtient 28 g (rendement 98 %) d'une mousse qui est employée directement dans les réactions suivantes.
C.C.M. : H2CCl2/MeOH 95/5 Rf = 0,54.
R.M.N. :

| | | | | | |
|---|---|---|---|---|---|
| 7,70-6,90 | ppm | m | 7 | protons | aromatiques |
| 4,45 | ppm | m | 1 | proton | CH2N |
| 4,3 | ppm | s | 1 | proton | CH-NH2 |
| 3,90 | ppm | m | 1 | proton | CH2N |
| 3,50-3,10 | ppm | m | 2 | protons | CH2Ar |
| 3,20 | ppm | s | 2 | protons | NH2 |

I.R. : 3400, 1680, 1580, 1440, 1200, 740 cm-1.

2° méthode

5,8 g (168 mmoles) de produit du stade-(d) sont placés en suspension dans du méthanol, sous atmosphère d'azote, et on ajoute 3,99 g (168 mmoles) de chlorure de nickel hexahydraté. On refroidit à -20°C et ajoute par petites quantités 1,2 g (336 mmoles) de borohydrure de sodium. La température monte immédiatement et il se forme un précipité noir. On agite à-20°C pendant 30 minutes et évapore à sec. On reprend par de l'acide chlorhydrique concentré, filtre, et alcalinise par une solution d'ammoniaque concentrée, en présence de chlorure de méthylène. On évapore et obtient 6,8 g de produit brut que l'on chromatographie sur silice en éluant par du chlorure de méthylène enrichi progressivement en acétone. On obtient 2,8 g(rendement 48%) d'une mousse identique au produit obtenu par la première méthode.

Stade-(f).

Préparation de la (fluoro-2-phényl)-6-[(indolyl-2)-carbonylamino]-4-tétrahydro-1,2,3,4-pyrrolo[3,2,1- j,k]benzodiazépin-[1,4]-one-3. (Ig).

De la même façon que dans l'exemple-1 stade-(c), à partir de 5 g (169 mmoles) du produit du stade précédent on obtient après chromatographie sur silice dans un gradient de chlorure de méthylène contenant de l'acétone 2,6 g (rendement 36 %) d'un produit dont le point de fusion est 289°C (décomposition).
C.C.M. : H2CCl2/Acétone 95/5 Rf = 0,55.
R.M.N. :

| | | | | | |
|---|---|---|---|---|---|
| 11,65 | ppm | s | 1 | proton | NH indolique |
| 9,58 | | d | 1 | proton | CH-NH-CO |
| 7,80-6,90 | ppm | m | 12 | protons | aromatiques |
| 5,60 | ppm | d | 1 | proton | CH-NH-CO |
| 4,50 | ppm | m | 1 | proton | CH2N |
| 4,0 | ppm | m | 1 | proton | CH2N |
| 3,30 | | m | 2 | proton | CH2Ar |

I.R. : 3250, 1680, 1630, 1525, 1400, 1340, 1340, 1300, 1210, 740 cm-1.

EXEMPLE-7

Préparation de la (fluoro-2-phényl)-6-[(méthoxy-5-indolyl-)-2-carbonyl-amino]-4-tétrahydro-1,2,3,4-pyrrolo [3,2,1-j,k]benzodiazépin-[1,4]-one-3.

Formule (I) ; Ar = méthoxy-5-indolyl-2 ; $R_1$ = H ; $R_2$ = F-2 ; n = 2 ; (Ih).

De la même façon que dans l'exemple-1 stade-(c), à partir de 4,25 g (203 mmoles) du produit de l'exemple-6 stade-(e). Après chromatographie sur silice dans un gradient de chlorure de méthylène contenant de l'acétone, on obtient 4,0 g (rendement 42 %). F = 214°C.
C.C.M. : H2CCl2/Acétone 95/5 Rf = 0,55.
R.M.N. :

| | | | | | |
|---|---|---|---|---|---|
| 11,5 | ppm | s | 1 | proton | NH indolique |
| 9,50 | | d | 1 | proton | CH-NH-CO |
| 7,70-6,80 | ppm | m | 11 | protons | aromatiques |
| 5,60 | ppm | d | 1 | proton | CH-NH-CO |
| 4,50 | ppm | m | 1 | proton | CH2N |
| 4,0 | ppm | m | 1 | proton | CH2N |
| 3,80 | | s | 3 | protons | OCH3 |
| 3,50-3,0 | ppm | m | 2 | protons | CH2Ar |

I.R. : 3250, 1680, 1630, 1525, 1440, 1395, 1340, 1220, 1100, 740 cm-1.

EXEMPLE-8

Préparation de la (fluoro-2-phényl)-6-[(indolyl-3-carbonylamino]-4-tétrahydro-1,2,3,4-pyrrolo[3,2,1- j,k]benzodiazépin-[1,4]-one-3.

Formule (I) ; Ar = indolyl-3 ; $R_1$ = H ; $R_2$ = F-2 ; n = 2 ; (Ii).

De la même façon que dans l'exemple-1 stade-(c), à partir de 4,0 g (135 mmoles) du produit de l'exemple-6 stade-(e) et de 2,425 g (135 mmoles) d'acide indole-3 carboxylique. Après chromatographie sur silice dans un gradient de chlorure de méthylène contenant de l'acétone, on obtient 1,8 g (rendement 30%). F = 206°C.
C.C.M. : H2CCl2/Acétone 85/15 Rf = 0,35.
R.M.N. :

| | | | | | |
|---|---|---|---|---|---|
| 10,1 | ppm | s | 1 | proton | NH indolique |
| 8,20 | | d | 1 | proton | CH-NH-CO |
| 8,00-6,90 | ppm | m | 12 | protons | aromatiques |
| 5.80 | ppm | d | 1 | proton | CH-NH-CO |
| 4,65 | ppm | m | 1 | proton | CH2N |
| 4,0 | ppm | m | 1 | proton | CH2N |
| 3.20 | | m | 2 | proton | CH2Ar |

I.R. : 3230, 1675, 1630, 1530, 1490, 1200, 740 cm-1.

EXEMPLE-9

Préparation de la (fluoro-2-phényl)-6-[(naphtyl-1)-carbonylamino]-4-tétrahydro-1,2,3,4-pyrrolo[3,2,1- j,k]benzodiazépin-[1,4]-one-3.

Formule (I) ; Ar = naphtyl-1 ; $R_1$ = H ; $R_2$ = F-2 ; n = 2 ; (Ij).

De la même façon que dans l'exemple-1 stade-(c), à partir de 4,0 g (135 mmoles) du produit de l'exemple-6 stade-(e) et de 2,59 g (135 mmoles) de chlorure de l'acide naphtyl-1-carboxylique. Après chromatographie sur silice dans un gradient de chlorure de méthylène contenant de l'acétone, on obtient 2,5 g (rendement 41 %). F = 150°C.
C.C.M. : H2CCl2/Acétone 95/5 Rf = 0,75.
R.M.N. :

| | | | | | | |
|---|---|---|---|---|---|---|
| 8,60 | | d | 1 | proton | J = 8Hz | CH-NH-CO |
| 8,0-6,90 | ppm | m | 14 | protons | | aromatiques |
| 5,80 | ppm | d | 1 | proton | J = 8Hz | CH-NH-CO |
| 4,70 | ppm | m | 1 | proton | | CH2N |
| 4,05 | ppm | m | 1 | proton | | CH2N |
| 3.30 | | m | 2 | protons | | CH2Ar |

I.R. : 3400, 1680, 1660, 1600, 1480, 1440, 1385, 750 cm-1.

EXEMPLE-10

Préparation du chloro-8-(fluoro-2-phényl)-6-[(indolyl-2)-carbonylamino]-4-tétrahydro-1,2,3,4-pyrrolo[3,2,1-j,k]benzodiazépin-[1,4]-one-3.

Formule (I) ; Ar = indolyl-2 ; $R_1$ = Cl-8 ; $R_2$ = F-2 ; n = 2 ; (Ik).

Stade-(a).

Préparation de la chloro-5-(fluoro-2-)-benzoyl-7-indoline.

Dans un réacteur muni d'une agitation centrale d'un réfrigérant équipé d'une garde à chlorure de calcium, d'un thermomètre plongeant, d'une arrivée d'azote, d'une ampoule à brome à rétablissement de pression, on verse 44 g (182 mmoles) de (fluoro-2-benzoyl)-7-indoline et 800 ml de chlorure de méthylène. On ajoute peu à peu 27,94 g (182 mmoles) de N-chlorosuccinimide et agite une nuit à température ambiante. On lave avec une solution saturée de bicarbonate de sodium, puis à l'eau, on sèche et évapore. On chromatographie sur silice en éluant par du chlorure de méthylène. On obtient 42 g (rendement 84 %). Le produit est obtenu sous la forme d'une mousse qui n'a pas de point de fusion défini.
C.C.M. : H2CCl2 Rf = 0,7.

R.M.N. :

| | | | | | |
|---|---|---|---|---|---|
| 7,60-7,00 | ppm | m | 6 | protons | aromatiques |
| 6,30 | ppm | m | 1 | proton | NH |
| 3,85 | ppm | m | 2 | protons | CH2N |
| 3,10 | | m | 2 | protons | CH2Ar |

Stade-(b).

Préparation de la N-bromoacétyl-chloro-5-(fluoro-2-benzoyl)-7-indoline.

55 g (199 mmoles) de produit du stade précédent sont traités comme dans l'exemple-6 stade-(b) avec 34,36 g (219 mmoles) de bromure de bromacètyle. On obtient 60 g (rendement 76 %).
C.C.M. : H2CCl2 Rf = 0,25.
R.M.N. :

| | | | | | |
|---|---|---|---|---|---|
| 7,90-6,80 | ppm | m | 6 | protons | aromatiques |
| 4,25 | ppm | t | 2 | proton | CH2N |
| 3,80 | ppm | s | 2 | protons | CH2Br |
| 3,20 | ppm | t | 2 | protons | CH2Ar |

Stade-(c).

Préparation de la chloro-8-(fluoro-2-phényl)-6-tétrahydro-1,2,3,4-pyrrolo [3,2,1-j,k]benzodiazépin-[1,4]-one-3.

Formule (III) ; $R_1$ = Cl-8 ; $R_2$ = F-2 ; n = 2 ; (IIIc).

Selon l'exemple-6 stade-(c), à partir de 90 g (226 mmoles) de produit du stade précédent, on obtient après chromatographie dans un gradient de chlorure de méthylène enrichi en acétone. On obtient 51 g (rendement 65 %). F =148°C. C.C.M. : H2CCl2/Acétone 95/5 Rf = 0,3.
R.M.N. :

| | | | | | |
|---|---|---|---|---|---|
| 7,60-7,00 | ppm | m | 6 | protons | aromatiques |
| 4,45 | ppm | s | 2 | protons | CO-CH2-N |
| 4,32 | ppm | t | 2 | protons | CH2N |
| 4,20 | ppm | t | 2 | protons | CH2Ar |

I.R. : 1660, 1440, 1370, 1340, 1225, 880, 800, 745 cm-1.

Stade-(d).

Préparation du chloro-8-(fluoro-2-phényl)-6-hydroximino-4-tétrahydro-1,2,3,4-pyrrolo[3,2,1-j,k]-benzodiazépin-[1,4]-one-3.

Formule (IV) ; $R_1$ = Cl-8 ; $R_2$ = F-2 ; n = 2 ; (IVc).

De la même façon que dans l'exemple-1 stade-(a), à partir de 45 g (143 mmoles) du produit du stade précédent on obtient 33 g (rendement 67 %) d'un solide jaune F=264°C (dec.) C.C.M. : H2CCl2/Acétone 90/10 Rf = 0,27.

R.M.N. :

| | | | | | |
|---|---|---|---|---|---|
| 7,85-7,0 | ppm | m | 6 | protons | aromatiques |
| 4,32 | ppm | t | 2 | protons | CH2N |
| 3,48 | ppm | s | 1 | proton | NOH |
| 3,28 | ppm | t | 2 | protons | CH2Ar |

I.R. : 3300, 1655, 1615, 1580, 1445, 1370, 1340, 1220, 1160, 1020, 850, 740 cm-1.

Stade-(e).

Préparation de l'amino-4-chloro-8-(fluoro-2-phényl)-6-tétrahydro-1,2,3,4-pyrrolo[3,2,1-j,k]benzodiazépin-[1,4]-one-3.

Formule (VI) ; $R_1$ = Cl-8 ; $R_2$ = F-2 ; n = 2 ; (Vc).

De la même façon que dans l'exemple-1 stade-(b), 2° méthode à partir de 30 g (870 mmoles) du produit du stade précédent on obtient 28 g (rendement 98 %) d'une mousse qui est employée directement dans les réactions suivantes.
C.C.M. : H2CCl2/MeOH 95/5 Rf = 0,55.
R.M.N. :

| | | | | | |
|---|---|---|---|---|---|
| 7,70-6,95 | ppm | m | 6 | protons | aromatiques |
| 4,60 | ppm | m | 1 | proton | CH2N |
| 4,40 | ppm | s | 1 | proton | CH-NH2 |
| 4,0 | ppm | m | 1 | proton | CH2N |
| 3,23 | ppm | m | 2 | protons | CH2Ar |
| 2,50 | ppm | s | 2 | protons | NH2 |

I.R. : 3350, 1670, 1610, 1580, 1440, 1335, 1210, 860, 790, 750 cm-1.

Stade-(f).

Préparation de la chloro-8-(fluoro-2-phényl)-6-[(indolyl-2)-carbonylamino]-4-tétrahydro-1,2,3,4- pyrrolo [3,2,1-j,k]benzodiazépin-[1,4]-one-3 (Ik).

De la même façon que dans l'exemple-1 stade-(c), à partir de 3,2 g (97 mmoles) du produit du stade précédent et 1,75 g (97 mmoles) du chlorure de l'acide indolyl-2-carboxylique. Après chromatographie sur silice dans un gradient de chlorure de méthylène contenant de l'acétone, on obtient 2,7 g (rendement 60 %). F = 248°C (décomposition).
C.C.M. : H2CCl2/Acétone 95/5 Rf = 0,37.
R.M.N. :

| | | | | | | |
|---|---|---|---|---|---|---|
| 10 | ppm | s | 1 | proton | | NH indolique |
| 8,10 | ppm | d | 1 | proton | J = 8Hz | CH-NH-CO |
| 7,75-6,90 | ppm | m | 11 | protons | | aromatiques |
| 5,7 | ppm | d | 1 | proton | J = 8Hz | CH-NH-CO |
| 4,67 | ppm | m | 1 | proton | | CH2N |
| 4,55 | ppm | m | 1 | proton | | CH2N |
| 3.25 | ppm | m | 2 | protons | | CH2Ar |
| 2.15 | ppm | m | 2 | protons | | H2O |

I.R. : 3250, 1680, 1640, 1530, 1480, 1445, 1430, 1100, 795, 740 cm-1 :

EXEMPLE-11

Préparation de la chloro-8-[(chloro-5-indolyl-2)-carbonylamino]-4-(fluoro-2-phényl)-6-tétrahydro-1,2,3,4-pyrrolo[3,2,1- j,k]benzodiazépin-[1,4]-one-3.

Formule (I) ; Ar = chloro-5-indolyl-2 ; $R_1$ = Cl-8 ; $R_2$ = F-2 ; n = 2 ; (II).

De la même façon que dans l'exemple-1 stade-(c), à partir de 5,0 g (152 mmoles) du produit de l'exemple-10 stade-(e) et 3,25 g (152 mmoles) du chlorure de l'acide chloro-5-indolyl-2-carboxylique. Après chromatographie sur silice dans un gradient de chlorure de méthylène contenant de l'acétone, on obtient 2,7 g (rendement 38 %) d'un solide blanc cristallisant avec 0,25 molécule d'eau. F = 246°C (décomposition).
C.C.M. : H2CCl2/Acétone 95/5 Rf = 0,4.
R.M.N. :

| | | | | | | |
|---|---|---|---|---|---|---|
| 11,75 | ppm | m | 1 | proton | | NH indolique |
| 9,65 | ppm | d | 1 | proton | J = 8Hz | CH-NH-CO |
| 7,70-6,90 | ppm | m | 10 | protons | | aromatiques |
| 5,60 | ppm | d | 1 | proton | J = 8Hz | CH-NH-CO |
| 4,5 | ppm | m | 1 | proton | | CH2N |
| 4,0 | ppm | m | 1 | proton | | CH2N |
| 3.2 | | m | 2 | protons | | CH2Ar |
| 3,20 | | m | 2 | protons | | H2O |

I.R. : 3250, 1640, 1530, 1445, 1340, 1200, 790, 750 m-1.

EXEMPLE-12

Préparation de la chloro-8-(dichloro-3,5- benzoylamino)-4-(fluoro-2-phényl)-6-tétrahydro-1,2,3,4-pyrrolo[3,2,1-j,k]benzodiazépin-[1,4]-one-3.

Formule (I) ; Ar = dichloro-3,5-phényl ; $R_1$ = Cl-8 ; $R_2$ = F-2 ; n = 2 ; (Im).

De la même façon que dans l'exemple-1 stade-(c), à partir de 5,0 g (152 mmoles) du produit de l'exemple-10 stade-(e) et 3,42 g (152 mmoles) du chlorure de l'acide dichloro-3,5-benzoïque. Après chromatographie sur silice dans un gradient de chlorure de méthylène contenant de l'acétone, on obtient 4,4 g (rendement 58 %) d'un produit cristallisant avec 0,25 molécule d'eau. F = 291°C (décomposition).
C.C.M. : H2CCl2/Acétone 95/5 Rf = 0,45.
R.M.N. : (acide trifluoroacétique)

| | | | | | |
|---|---|---|---|---|---|
| 8,10-7,30 | ppm | m | 9 | protons | aromatiques |
| 6,25 | ppm | m | 1 | proton | CH-NH-CO |
| 5,0 | ppm | m | 1 | proton | CH2N |
| 4,40 | ppm | m | 1 | proton | CH2N |
| 3,55 | | m | 2 | protons | CH2Ar |

I.R. : 3250,3050, 1680, 1640, 1550, 1530, 1470, 1440, 1280, 1200, 865, 800, 750 cm-1.

EXEMPLE-13

Préparation de l'[(indolyl-2)-carbonylamino]-5-phényl-7-hexahydro-1,2,3,3a,4,5-pyrido[3,2,1-j, k] benzodiazépin-[1,4]-one-4

Formule (I) ; Ar = indolyl-2 ; $R_1$ = H ; $R_2$ = H ; n = 3 ; (In).

Stade-(a).

Préparation de l'hydroximino-5-phényl-7-hexahydro-1,2,3,3a,4,5-pyrido[3,2,1-j,k]benzodiazépin-[1,4]-one-4

Formule (IV) ; $R_1$ = H ; $R_2$ = H ; n = 3 ; (IVd).

De la même façon que dans l'exemple-1 stade-(a), à partir de 26,9 g (97 mmoles) de phényl-7-tétrahydro-1, 2, 3, 4-pipéridino[3,2,1-j,k] benzodiazépin-1,4-one-3. Après chromatographie dans un gradient de chlorure de méthylène enrichi en acétone on obtient 24,3 g (rendement 82 %) d'un solide F = 195°C.
C.C.M. : H2CCl2/Acétone 90/10 Rf = 0,3.
C.C.M. : H2CCl2/MeOH 95/5 Rf = 0,35.
R.M.N. :

| | | | | | |
|---|---|---|---|---|---|
| 9.20 | ppm | s | 1 | proton | NOH |
| 7.90-6.80 | ppm | m | 8 | protons | aromatiques |
| 4.6 | ppm | m | 1 | proton | CH2N |
| 3.20 | ppm | m | 1 | proton | CH2N |
| 2.95 | ppm | m | 2 | protons | CH2Ar |
| 2.0 | ppm | m | 2 | protons | CH2-CH2-CH2 |

I.R. : 3350, 3150, 3050, 1650, 1610, 1440, 1380, 1300, 1000 cm-1.

Stade-(b).

Préparation de l'amino-5-phényl-7-hexahydro-1, 2,3,3a,4,5-pyrido [3,2,1-j,k]benzodiazépin-[1,4]-one-4.

Formule (V) ; $R_1$ = H ; $R_2$ = H ; n = 3 ; (Vd).

De la même façon que dans l'exemple-1 stade-(b), 2° méthode à partir de 28,8 g (94 mmoles) de produit du stade précédent, après chromatographie dans un gradient de chlorure de méthylène enrichi en acétone, on obtient 22 g (rendement 80 %) d'une mousse n'ayant pas de point de fusion défini qui est employée directement dans les réactions suivantes.
C.C.M. : H2CCl2/Acétone 10 % Rf = 0,10
C.C.M. : H2CCl2/MeOH 95/5 Rf = 0,25
R.M.N. :

| | | | | | |
|---|---|---|---|---|---|
| 7,70-7,00 | ppm | m | 8 | protons | aromatiques |
| 5,0 | ppm | m | 2 | protons | CH-NH2, CH2N |
| 3,30 | ppm | m | 1 | proton | CH2N |
| 2,90 | | m | 2 | protons | CH2Ar |
| 2,62 | | s | 2 | protons | NH2 |
| 2,40-1,70 | | m | 2 | protons | CH2-CH2-CH2 |

I.R. : 1670, 1590, 1560, 1440, 1300, 1270 cm-1.

Stade-(c).

De la même façon que dans l'exemple-1 stade-(c), à partir de 2,49 g (85 mmoles) de produit du stade précédent on obtient 3,3 g (rendement 89 %). F = 220°C.
C.C.M. : H2CCl2/MeOH 97/3 Rf = 0,75.
R.M.N. :

| | | | | | |
|---|---|---|---|---|---|
| 11,70 | ppm | s | 1 | proton | NH indolique |
| 9,50 | ppm | d | 1 | proton | CH-NH-CO |
| 7,80-7,00 | ppm | m | 13 | protons | aromatiques |
| 5,68 | ppm | d | 1 | proton | CH-NH-CO |
| 4,4 | ppm | m | 1 | proton | CH2N |
| 3,30 | ppm | m | 1 | proton | CH2N |
| 2,95 | ppm | m | 2 | protons | CH2Ar |
| 2,0 | ppm | m | 2 | protons | CH2-CH2-CH2 |

I.R. : 3270, 1635, 1530, 1440, 1290, 1250 cm-1.

EXEMPLE-14

Préparation du [(chloro-5-indolyl-2)-carbonyl-amino]-5-phényl-7-hexahydro-1,2,3,3a,4,5-pyrido[3,2,1- j,k]benzodiazépin-[1,4]-one-4.

Formule (I) ; Ar = chloro-5-indolyl-2 ; $R_1$ = H ; $R_2$ = H ; n = 3; (Io).

De la même façon que dans l'exemple-1 stade-(c), à partir de 3,2 g (11 mmoles) de produit de l'exemple-13 stade-(b) et de 2,35 g (11 mmoles) de chlorure de l'acide chloro-5-indolyl-2-carboxylique, on obtient 4,5 g (rendement 89 %).
C.C.M. : H2CCl2/MeOh 97/3 Rf = 0,95.
H2CCl2/Acétone 95/5 Rf = 0,55.
R.M.N. :

| | | | | | |
|---|---|---|---|---|---|
| 11,85 | | s | 1 | proton | NH indolique |
| 9,60 | ppm | d | 1 | proton | CH-NH-CO |
| 7,80-7,10 | ppm | m | 12 | protons | aromatiques |
| 5,62 | ppm | d | 1 | proton | CH-NH-CO |
| 4,35 | ppm | m | 1 | proton | CH2N |
| 3,32 | ppm | s | 2 | protons | H2O |
| 3,20 | ppm | m | 1 | proton | CH2N |
| 2,90 | ppm | m | 2 | protons | CH2Ar |
| 1,95 | ppm | m | 2 | proton | CH2-CH2-CH2 |

I.R. : 3380, 3230, 1640, 1530, 1470, 1440, 1390, 760 cm-1.

EXEMPLE-15

Préparation du (trifluorométhyl-3-benzoylamino)-5-phényl-7-hexahydro-1,2,3,3a,4,5-pyrido[3,2,1- j,k]benzodiazépin-[1,4]-one-4.

Formule (I) ; Ar = trifluorométhyl-3-phényl; $R_1$ = H ; $R_2$ = H ; n = 3 ; (Ip).

De la même façon que dans l'exemple-1 stade-(c), à partir de 2,91 g (10 mmoles) de produit de l'exemple-13 stade-(b) et de 2,08 g (10 mmoles) de chlorure de l'acide trifluorométhyll-3-benzoïque, on obtient 4,1 g (rendement 88 %). F = 250°C.

C.C.M. : H2CCl2/MeOH 97/3 Rf = 0,80
H2CCl2/Acétone 95/5 Rf = 0,55.

R.M.N. :

| | | | | | |
|---|---|---|---|---|---|
| 10,05 | ppm | d | 1 | proton | CH-NH-CO |
| 8,50-7,10 | ppm | m | 12 | protons | aromatiques |
| 5,65 | ppm | d | 1 | proton | CH-NH-CO |
| 4,35 | ppm | m | 1 | proton | CH2N |
| 3,30 | ppm | m | 1 | proton | CH2N |
| 2,95 | ppm | m | 2 | protons | CH2Ar |
| 1,95 | ppm | m | 2 | protons | CH2-CH2-CH2 |

I.R. : 3200, 1685, 1650, 1600, 1320, 1260, 1130, 1115 cm-1.

On donne ci-dessous les résultats d'études pharmacologiques et toxicologiques effectuées sur les benzodiazépines de l'invention.

**A. Activité in vitro**

1. L'affinité des composés de formule (I) pour les récepteurs de la cholecystokinine a été étudiée en déterminant la concentration inhibitrice 50 ($CI_{50}$) pour la fixation de la cholecystokinine marquée à l'iode 125 (ci-dessous abrégée en [125I]-CCK8 sulfatée) aux récepteurs

- de membranes plasmiques de pancréas de rat (mâles SD, IFFA CREDO 200-225g)
- de membranes de cerveau de cobayes (mâles, COB LABO, 325-350g) selon le protocole décrit par INNIS R.B. et SNYDER S.M., Eur. J. Pharmacol., 65, 123-124, 1980.

**B. Activité in vivo**

L'activité antagoniste des composés de formule I pour la CCK a été déterminée chez la souris (SWISS

mâle, 18-20 g) sur le modèle d'évacuation gastrique décrit par LOTTI V.J. et coll. (LIFE SCIENCES. 39 ; 1631-1638 ; 1986) en déterminant la dose efficace 50 % ($DE_{50}$) qui protège les animaux de l'inhibition de l'évacuation gastrique provoquée par la CCK-8 sulfatée (80 ug/kg, sc).

## C. Toxicité

La toxicité aigue a été évaluée et les tests d'IRWIN (IRWIN S., Psychopharmacologia, 13, 222-257, 1968) ont été effectués chez la souris mâle (20-21 g). Le tableau I ci-dessous indique les résultats des essais de binding au récepteurs de la CCK du pancréas de rat et du cerveau de cobaye sous forme de la concentration inhibitrice 50 % ($CI_{50}$) ainsi que dans la dernière colonne le rapport de la $CI_{50}$ relative aux récepteurs du pancréas à la $CI_{50}$ relative aux récepteurs du cerveau.

TABLEAU I

| | Liaison $CI_{50}$ [125I]CCK8-SO4 Pancréas | Cerveau | $CI_{50}$ (cerveau) / $CI_{50}$ (pancréas) |
|---|---|---|---|
| IIa | 11.2 | 847 | 75.6 |
| Ia | 5.3 | 1110 | 209.4 |
| Ib | 37 | 3100 | 83.8 |
| Ic | 45 | 8800 | 195.6 |
| Ie | 1.5 | 477 | 318.0 |
| If | 8640 | 24000 | 2.8 |
| Ig | 0.023 | 366 | 15913 |
| Il | 12.3 | 21392 | 2552.2 |
| Im | 30.6 | 25431 | 831.1 |
| In | 3.9 | 407 | 104.4 |

Dans le cas de (IIa), le rapport de la $CI_{50}$ pour les récepteurs du cerveau à la $CI_{50}$ pour les récepteurs du pancréas, est égal à 75,6 alors que, dans le cas des composés de formule (I), ce rapport est en général bien plus élevé. Ceci permet d'obtenir des médicaments présentant une meilleure sélectivité pour les troubles dépendant des récepteurs périphériques et par là moins d'effets secondaires.

Le tableau II, ci-dessous, indique les valeurs obtenues dans le test de l'évacuation gastrique, la toxicité aigue ($DL_{50}$) ainsi que la première dose modifiant un des paramètres du comportement étudié selon la méthode de IRWIN pour les composés de formule (I) ainsi que pour le composé de formule (+/-)-IIa qui est un des produits préférés décrits dans la demande de brevet cités plus haut. La dernière colonne du tableau II indique le rapport de la première dose donnant lieu à l'apparition d'une symptomatologie à la $DE_{50}$ dans le test de l'évacuation gastrique ce qui est interprété comme un index thérapeutique.

TABLEAU II

| | Evacuation gastrique DE50 mg/kg | Toxicité DL50 mg/kg | IRWIN mg/kg | "IRWIN" ------ DE50 |
|---|---|---|---|---|
| IIa | 0.050 | >> 600 | 120 | 2400 |
| Ia | 0.104 | >> 1000 | > 1000 | > 7600 |
| Ie | 0.087 | >> 1000 | > 1000 | > 11000 |
| Ig | 0.076 | >> 1000 | > 300 | > 3900 |

Il apparaît que cet index est en faveur des composés de l'invention, le composé (IIa) provoquant des troubles dès la dose de 120 mg/kg alors qu'aucun trouble n'est observé avec les composés de formule (I) à la dose de 300 voire 1000 mg/kg. Sous forme de médicaments les produits de l'invention apparaissent donc particulièrement utiles à la thérapeutique humaine ou vétérinaire en ce qui concerne :

- principalement les troubles de l'estomac, de l'intestin, du pancréas et de la vésicule biliaire dépendant de la cholecystokinine, comme par exemple les pancréatites, les troubles de la motricité notamment gastrique ou vésiculaire, les ulcères et le syndrome du colon irritable ;
- les dérégulations de l'appétit ;
- la douleur ;
- et éventuellement les troubles dépendant de l'interaction de la cholecystokinine avec les neuromédiateurs du système nerveux central comme par exemple les désordres neuroleptiques, la maladie de Parkinson, la psychose, le syndrome de Gilles de la Tourette, la diskynésie tardive.

Les produits de l'invention sont administrés sous forme de compositions appropriées à la nature et à l'importance de l'affection à traiter. La posologie journalière chez l'homme est habituellement comprise entre 2 milligrammes et 1 gramme de produit qui peut être absorbé en une ou plusieurs prises. Les compositions sont préparées sous des formes compatibles avec la voie d'administration envisagée, comme par exemple les comprimés, dragées, capsules, suppositoires, gels ou suspensions. Ces compositions sont préparées par des méthodes courantes pour l'homme de l'art et comprennent de 1 à 60 % en poids de principe actif (composé de formule I) et 40 à 99 % en poids de véhicule pharmaceutique approprié et compatible avec le principe actif et la forme physique de la composition envisagée. A titre d'exemple, il est présenté ci-dessous la méthode de préparation de comprimés contenant un composé de l'invention.

COMPRIMES

**Formule :**

| | | | | |
|---|---|---|---|---|
| Substance active de formule (I) | 1 | à | 75 | mg |
| Polyvinylpyrrolidone : | 2 | | | mg |
| Carboxyméthylamidon : | 8 | | | mg |
| Stéarate de magnésium : | 3 | | | mg |
| Lactose : | 122 | à | 76 | mg |
| Cellulose monocristalline : | 60 | à | 76 | mg |

pour un comprimé de 200 mg.

Fabrication :

Dissoudre la polyvinylpyrrolidone à raison de 0.1 à 1.0 % en poids dans de l'eau, un alcool aliphatique

inférieur comme par exemple l'éthanol ou un mélange hydroalcoolique. Par ailleurs, mélanger intimement la substance active, le lactose, la moitié de la quantité de cellulose et de carboxyméthylamidon et humidifier ce mélange avec la solution préparée précedemment. Granuler la pate, sécher les granulés sur tamis. Ajouter le reste des composants du mélange puis homogénéiser et comprimer à raison de 200 mg par comprimé.

## Revendications

**Revendications pour les Etats contractants suivants : AT, BE, CH, DE, FR, GB, IT, LI, LU, NL, SE**

1. Benzodiazépines de formule

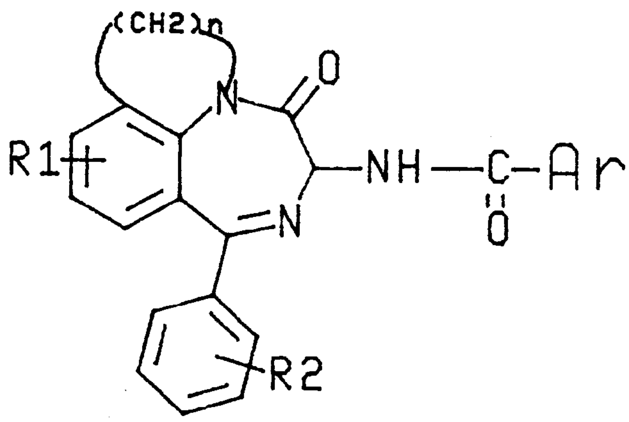

( I )

dans laquelle R1 est H ou halogène, R2 est H ou halogène, Ar est indolyle, phényle, naphtyle, indolyle monosubstitué par un halogène ou par un méthoxy, ou phényle mono di ou tri-substitué par un halogène ou par un méthoxy ou par un groupe trifluorométhyle, n est 2 ou 3 ; et leurs isomères optiques.

2. Benzodiazépines suivant la revendication 1, caractérisée en ce que Ar est le groupe indolyle-2.

3. Benzodiazépines suivant la revendication 1 ou 2, caractérisée en ce que R2 est l'hydrogène ou le fluor en position ortho par rapport à l'atome de carbone reliant le cycle phényle au cycle diazépine.

4. Benzodiazépines suivant l'une quelconque des revendications 1 à 3, caractérisée en ce que R1 est l'hydrogène ou le chlore en position para par rapport à l'atome d'azote commun au cycle diazépine et à l'autre hétérocycle azoté condensé avec le cycle diazépine.

5. Benzodiazépines suivant l'une quelconque des revendications 1 à 4, caractérisée en ce que n est égal à 2.

6. Benzodiazépines suivant l'une quelconque des revendications 1 à 5 caractérisée en ce que l'atome de carbone asymétrique en position alpha par rapport au carbonyle du cycle diazépine possède la configuration S suivant la nomenclature de Cahn, Ingold et Prelog.

7. Benzodiazépines suivant la revendication 1 qui sont :
   a) l'(indolyl-2-carbonylamino)-4-phényl-6-tétrahydro-1,2,3,4-pyrrolo[3,2,1-j,k]benzodiazépin- 1[1,4]-one-3, et de préférence son isomère 4S.
   b) la (fluoro-2-phényl)-6-[(indolyl-2)-carbonylamino]-4-tétrahydro-1,2,3,4-pyrrolo[3,2,1-j,k]-benzodiazépin-[1,4]-one-3, et de préférence son isomère 4S.
   c) la chloro-8-(fluoro-2-phényl)-6-[(indolyl-2)-carbonylamino]-4-tétrahydro-1,2,3,4-pyrrolo[3,2,1-j,k]benzodiazépin-1,4-one-3, et de préférence son isomère 4S.
   d) l'[(indolyl-2)-carbonylamino]-5-phényl-7-hexahydro-1,2,3,3a,4,5-pyrido[3,2,1-j, k] benzodiazépin-[1,4]-one-4, et de préférence son isomère 5S.

8. Procédé de préparation de benzodiazépines de formule (I) à la revendication 1, caractérisé en ce qu'il

consiste

a) pour préparer un benzodiazépine de formule (I) dans sa forme racémique :

à condenser une benzodiazépine-1,4 de formule

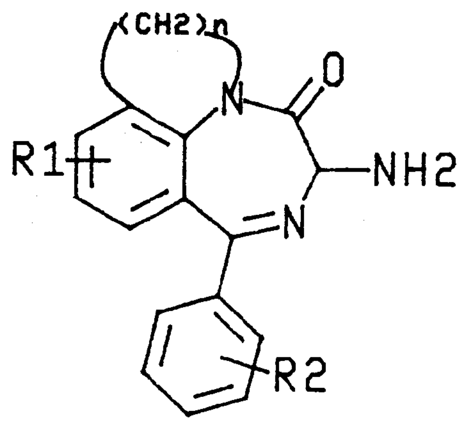

(V)

sur un dérivé d'acide carboxylique de formule Ar-CO-X dans laquelle Ar a la signification indiquée à la revendication 1 et X est un halogène, un groupe azido (-N3), un groupe imidazol-1-yle, un groupe -O-CO-R3, R3 pouvant être, outre de préférence Ar, un radical alkyle encombré comportant de trois à six atomes de carbone, ou aryle plus encombré que le radical Ar, substitué par un ou plusieurs halogènes, ou un groupe -OR4, R4 étant un groupe aromatique comportant un ou deux cycles, substitué par un ou plusieurs groupes nitro ou halogène, ou un groupe hétéroaromatique condensé comportant de un à trois hétéroatomes

b) pour préparer une benzodiazépine de formule (I) dans une forme optiquement active, dextrogyre ou lévogyre

à condenser une benzodiazépine racémique de formule (V) avec une molécule dérivée d'un amino-acide optiquement actif de formule (X) :

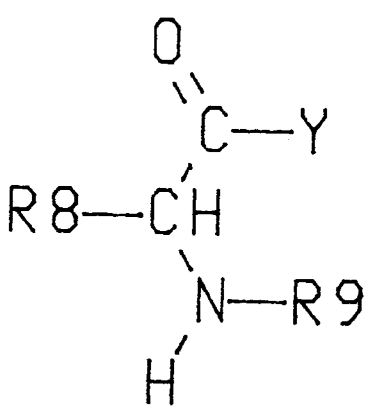

(X)

dans laquelle

Y est un groupe hydroxy, un groupe azido (-N3), un groupe imidazol-1-yl, un groupe O-CO-R3, R3 pouvant être un radical alkyle ramifié comportant de trois à six atomes de carbone, un groupe aryle encombré substitué par un ou plusieurs halogènes, ou un groupe -OR4, R4 étant un groupe aromatique comportant un ou deux cycles éventuellement substitué par un ou plusieurs radicaux nitro ou halogènes, ou un groupe hétéroaromatique condensé comportant de un à trois hétéroatomes.

R8 est

- un groupement alkyle ayant de 1 à 6 atomes de carbone éventuellement substitué par un hydroxyle, un groupe thioalkyle ayant de un à six atomes de carbone dans le groupe alkyl ou un groupe carboxyle ou carbonylamino,

- un groupe aryle comportant ou ou deux cycles éventuellement substitué par un hydroxyle,
- un groupe aralkyle ayant un ou deux cycles aromatiques et dont la portion alkylique comporte de un à six atomes de carbone, éventuellement substitué sur le cycle par un ou plusieurs groupes halogènes, hydroxy ou méthoxy
- un hétérocycle ayant cinq ou six chainons, et un ou deux hétéroatomes choisis parmi l'azote, l'oxygène et le soufre
- un groupe indolyl-3-méthyle,
- un groupe imidazolyl-4-méthyle.

R9 est un groupement pouvant être enlevé facilement pour régénérer l'amine libre et peut être
- un radical oxycarbonyle de structure A-O-CO-dans lequel A est un groupe alkyle comportant de un à six atomes de carbone, ou un groupe aryle, éventuellement substitué par un ou plusieurs groupes méthoxy, halogène ou nitro,
- un groupe alkanoyle ou alkènoyle comportant de un à six atomes de carbone ou aroyle
- p-nitrosulfényle,

pour obtenir un composé optiquement actif de formule

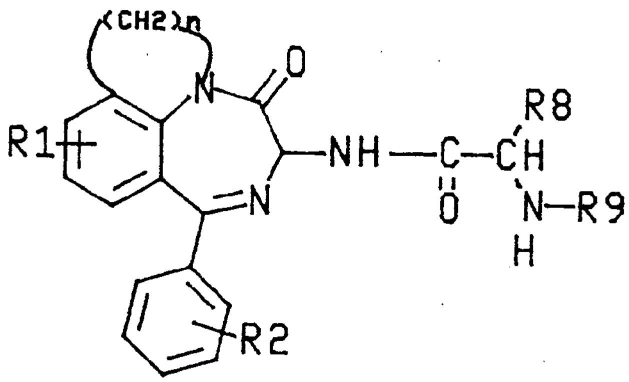

(VII)

que

i) dans le cas où R9 est benzyloxycarbonyl éventuellement substitué, on hydrogène en présence d'un catalyseur choisi parmi les métaux nobles déposés sur un support ou

ii) dans tous les autres cas on hydrolyse en milieu acide ou basique pour obtenir tant dans le cas i) que dans le cas ii) un mélange de deux diastéréoisomères de formule (VIII)

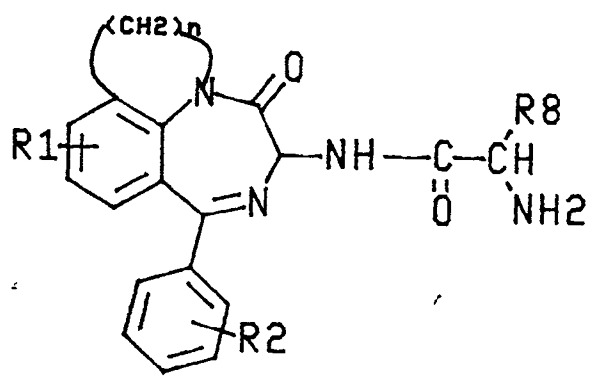

(VIII)

puis à séparer ces distéréoisomères par un procédé physicochimique choisi parmi une méthode chromatographique et/ou une cristallisation fractionnée de la base de formule VIII ou d'un de ses sels d'addition avec un acide, puis à faire réagir un arylisothiocyanate de formule R10-N=C=S dans laquelle R10 est un radical phényle ou naphtyle éventuellement substitué par un ou plusieurs substituants choisis parmi les halogènes, le groupe méthoxy ou un groupement alkyle ayant de un à six atomes de carbone sur l'un au moins des diastéréoisomères pour obtenir une thiourée optiquement active de formule (IX) :

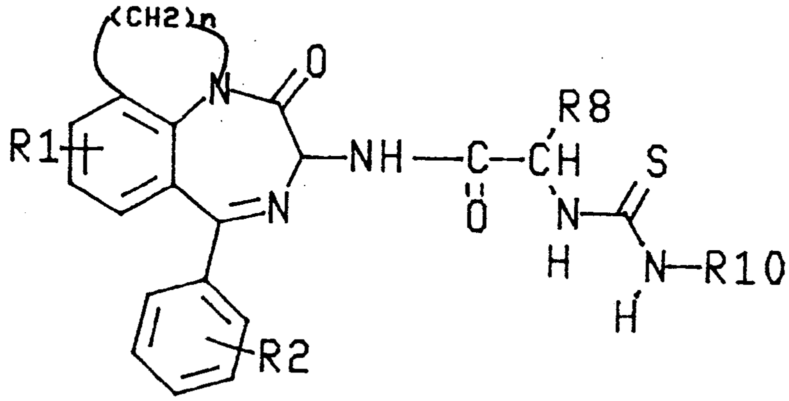

(IX)

puis à effectuer sur la thiourée de formule IX une réaction d'élimination du radical amino acide en la traitant par un acide pour obtenir l'amine (V) optiquement active que l'on met à réagir ensuite comme dans le cas a) de la préparation de la benzodiazépine racémique, pour obtenir la benzodiazépine de formule I optiquement active.

**9.** Procédé suivant la revendication 8, caractérisé en ce qu'il consiste à préparer l'amine de formule (V) en aminant en la position alpha par rapport au carbonyle une benzodiazépine-1,4-one de formule

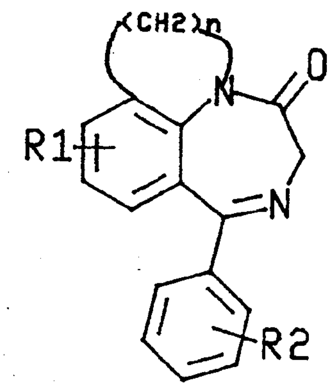

(III)

par un dérivé d'hydroxylamine ou par la chloramine.

**10.** Procédé suivant la revendication 8, caractérisé en ce qu'il consiste à aminer la benzodiazépine-1,4-one de formule (III) en deux stades, le premier stade consistant à la faire réagir sur un réactif d'oximation de formule R5-(N=O)m dans lequel R5 est alcoxy inférieur au chlore, quand m est égal à 1 et est une liaison supplémentaire entre les deux atomes d'azote quand m est égal à 2, pour obtenir l'oxime de formule

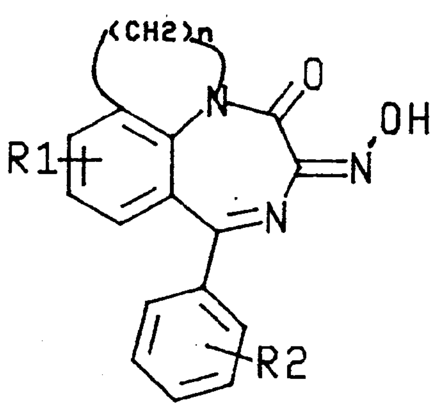

(IV)

que l'on isole, et le second stade consistant à réduire l'oxime catalytiquement par de l'hydrogène en présence d'un catalyseur de réduction ou par réaction avec du zinc en présence d'acide acétique, ou avec du chlorure stanneux en présence d'acide chlorhydrique pour obtenir le dérivé aminé.

11. Procédé suivant la revendication 8, caractérisé en ce qu'il consiste à préparer l'amine de formule (V) en mettant une benzodiazépine-[1,4]-one de formule (III) à réagir en milieu basique sur un réactif susceptible d'introduire une fonction azoture sur un carbanion pour obtenir un azoture de formule

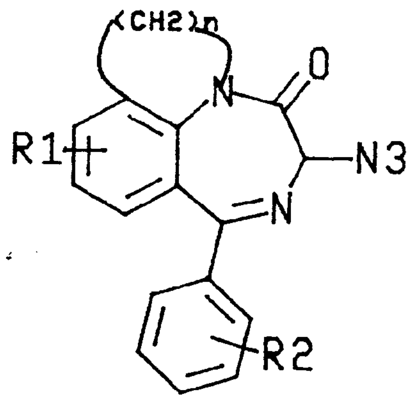

(VI)

12. Médicament pour lutter principalement contre les troubles gastro-intestinaux, du pancréas, de la vésicule biliaire et de l'appétit ; la douleur ; et éventuellement contre les troubles du système nerveux central, caractérisé en ce qu'il comprend une benzodiazépine selon l'une des revendications 1 à 7.

13. Intermédiaires de formule

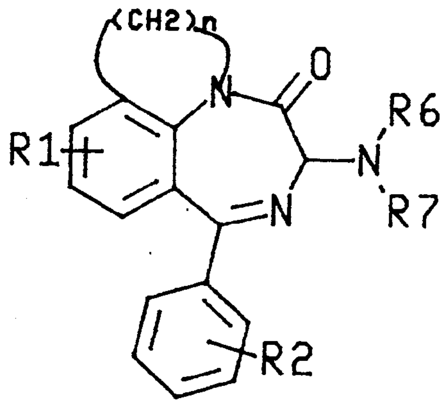

(XI)

n, R1 et R2 ayant les significations indiquées à la revendication 1, R6 étant hydroxy quand R7 représente une liaison supplémentaire entre l'atome d'azote portant R6 et le cycle diazépine et R6 étant l'hydrogène quand R7 est l'hydrogène.

**Revendications pour l'Etat contractant suivant : ES**

1. Procédé de préparation de benzodiazépines de formule

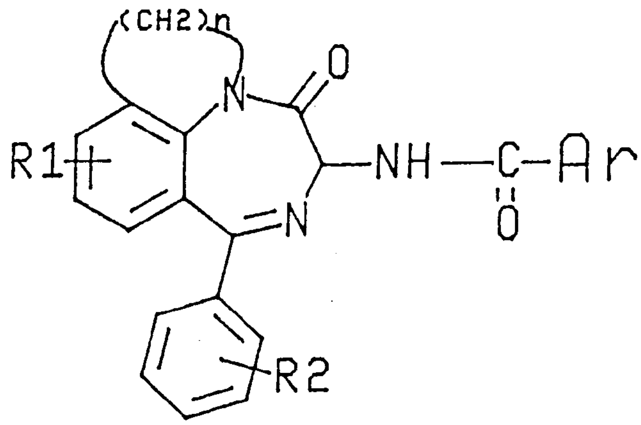

(I)

dans laquelle R1 est H ou halogène, R2 est H ou halogène, Ar est indolyle, phényle, naphtyle, indolyle monosubstitué par un halogène ou par un méthoxy, ou phényle mono di ou tri-substitué par un halogène ou par un méthoxy ou par un groupe trifluorométhyle, n est 2 ou 3 ; et leurs isomères optique, caractérisé en ce qu'il consiste

a) pour préparer une benzodiazépine de formule (I) dans sa forme racémique :

à condenser une benzodiazépine-1,4 de formule

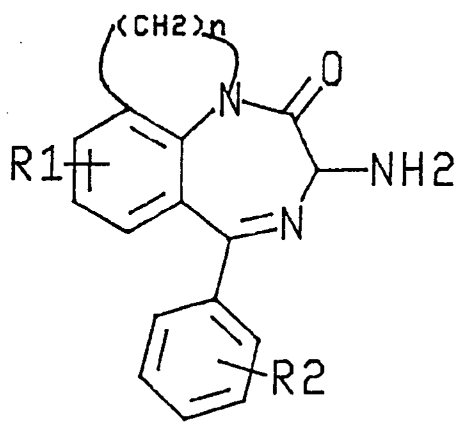

(V)

sur un dérivé d'acide carboxylique de formule Ar-CO-X dans laquelle Ar a la signification indiquée à la revendication 1 et X est un halogène, un groupe azido (-N3), un groupe imidazol-1-yle, un groupe -O-CO-R3, R3 pouvant être, outre de préférence Ar, un radical alkyle encombré comportant de trois à six atomes de carbone, ou aryle plus encombré que le radical Ar, substitué par un ou plusieurs halogènes, ou un groupe -OR4, R4 étant un groupe aromatique comportant un ou deux cycles, substitué par un ou plusieurs groupes nitro ou halogènes, ou un groupe hétéroaromatique condensé comportant de un à trois hétéroatomes

b) pour préparer une benzodiazépine de formule (I) dans une forme optiquement active, dextrogyre ou lévogyre

à condenser une benzodiazépine racémique de formule (V) avec une molécule dérivée d'un amino-acide optiquement actif de formule (X) :

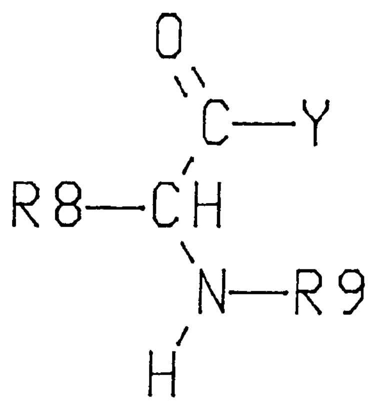

(X)

dans laquelle

Y est un groupe hydroxy, un groupe azido (-N3), un groupe imidazol-1-yl, un groupe O-CO-R3, R3 pouvant être un radical alkyle ramifié comportant de trois à six atomes de carbone, un groupe aryle encombré substitué par un ou plusieurs halogènes, ou un groupe -OR4, R4 étant un groupe aromatique comportant un ou deux cycles éventuellement substitué par un ou plusieurs radicaux nitro ou halogènes, ou un groupe hétéroaromatique condensé comportant de un à trois hétéroatomes.

R8 est

- un groupement alkyle ayant de 1 à 6 atomes de carbone éventuellement substitué par un hydroxyle, un groupe thioalkyle ayant de un à six atomes de carbone dans le groupe alkyl ou un groupe carboxyle ou carbonylamino,
- un groupe aryle comportant un ou deux cycles éventuellement substitué par un hydroxyle,
- un groupe aralkyle ayant un ou deux cycles aromatiques et dont la portion alkylique comporte de un à six atomes de carbone, éventuellement substitué sur le cycle par un ou plusieurs groupes halogènes, hydroxy ou méthoxy
- un hétérocycle ayant de cinq à six chainons, et un ou deux hétéroatomes choisis parmi l'azote, l'oxygène et le soufre
- un groupe indolyl-3-méthyle,
- un groupe imidazolyl-4-méthyle.

R9 est un groupement pouvant être enlevé facilement pour régénérer l'amine libre et peut être

- un radical oxycarbonyle de structure A-O-CO-dans lequel A est un groupe alkyle comportant de un à six atomes de carbone, ou un groupe aryle, éventuellement substitué par un ou plusieurs groupes méthoxy, halogène ou nitro,
- un groupe alkanoyle ou alkènoyle comportant de un à six atomes de carbone ou aroyle
- p-nitrosulfényle,

pour obtenir un composé optiquement actif de formule

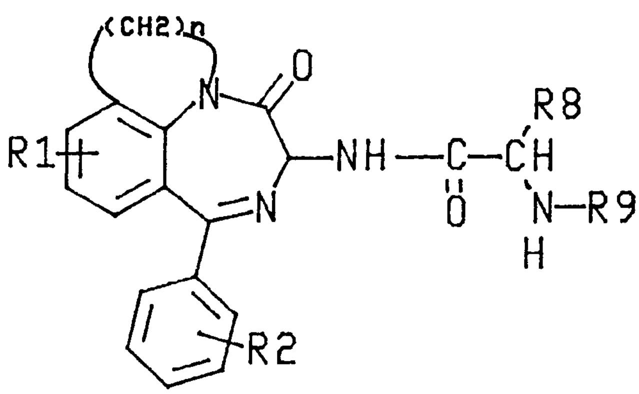

(VII)

que

i) dans le cas où R9 est benzyloxycarbonyl éventuellement substitué, on hydrogène en présence d'un catalyseur choisi parmi les métaux nobles déposés sur un support ou

ii) dans tous les autres cas on hydrolyse en milieu acide ou basique pour obtenir tant dans le cas i) que dans le cas ii) un mélange de deux diastéréoisomères de formule (VIII)

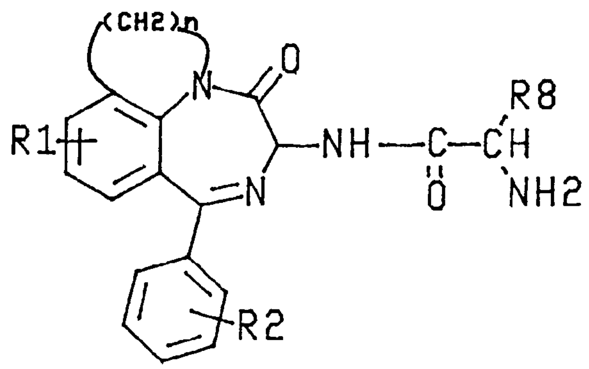

(VIII)

puis à séparer ces diastéréoisomères par un procédé physicochimique choisi parmi une méthode chromatographique et/ou une cristallisation fractionnée de la base de formule VIII ou d'un de ses sels d'addition avec un acide, puis à faire réagir un arylisothiocyanate de formule R10-N=C=S dans laquelle R10 est un radical phényle ou naphtyle éventuellement substitué par un ou plusieurs substituants choisis parmi les halogènes, le groupe méthoxy ou un groupement alkyle ayant de un à six atomes de carbone sur l'un au moins des diastéréoisomères pour obtenir une thiourée optiquement active de formule (IX) :

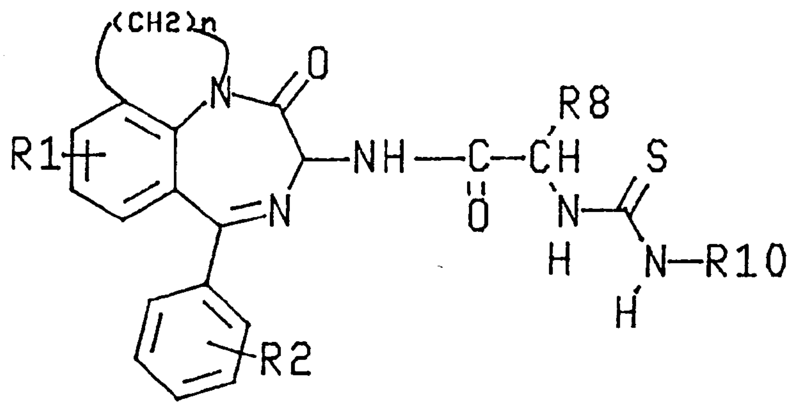

(IX)

puis à effectuer sur la thiourée de formule IX une réaction d'élimination du radical amino acide en la traitant par un acide pour obtenir l'amine (V) optiquement active que l'on met à réagir ensuite comme dans le cas a) de la préparation de la benzodiazépine racémique, pour obtenir la benzodiazépine de formule I optiquement active.

**2.** Procédé suivant la revendication 1, caractérisé en ce que Ar est le groupe indolyle-2.

**3.** Procédé suivant la revendication 1 ou 2, caractérisé en ce que R2 est l'hydrogène ou le fluor en position ortho par rapport à l'atome de carbone reliant le cycle phényle au cycle diazépine.

**4.** Procédé suivant l'une quelconque des revendications 1 à 3, caractérisé en ce que R1 est l'hydrogène ou le chlore en position para par rapport à l'atome d'azote commun au cycle diazépine et à l'autre hétérocycle azoté condensé avec le cycle diazépine.

**5.** Procédé suivant l'une quelconque des revendications 1 à 4, caractérisé en ce que n est égal à 2.

**6.** Procédé suivant l'une quelconque des revendications 1 à 5, caractérisé en ce que l'atome de carbone asymétrique en position alpha par rapport au carbonyle du cycle diazépine possède la configuration S suivant la nomenclature de Cahn, Ingold et Prelog.

7. Procédé selon la revendication 1 qui consiste à préparer
 a) l'(indolyl-2-carbonylamino)-4-phényl-6-tétrahydro-1,2,3,4-pyrrolo[3,2,1-j,k]benzodiazépin-[1,4]-one-3 , et de préférence son isomère 4S.
 b) la (fluoro-2-phényl)-6-[(indolyl-2)-carbonylamino]-4-tétrahydro-1,2,3,4-pyrrolo[3,2, 1-j,k]-benzo-diazépin-[1,4]-one-3, et de préférence son isomère 4S.
 c) la chloro-8-(fluoro-2-phényl)-6-[(indolyl-2)-carbonylamino]-4-tétrahydro-1,2,3,4-pyrrolo[3,2,1- j,k]benzo-diazépin-1,4-one-3, et de préférence son isomère 4S.
 d) l'[(indolyl-2)-carbonylamino]-5-phényl-7-hexahydro-1,2,3,3a,4,5-pyrido[3,2, 1-j,k]benzodiazépin-[1,4-one-4, et de préférence son isomère 5S.

8. Procédé suivant la revendication 1, caractérisé en ce qu'il consiste à préparer l'amine de formule (V) en aminant en la position alpha par rapport au carbonyle une benzodiazépine-1,4-one de formule

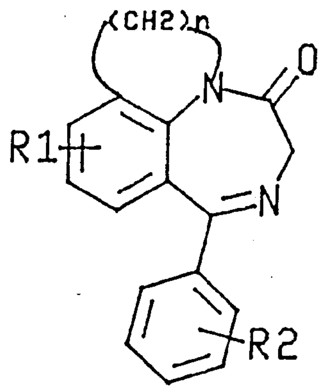

(III)

par un dérivé d'hydroxylamine ou par la chloramine.

9. Procédé suivant la revendication 1, caractérisé en ce qu'il consiste à aminer la benzodiazépine-1,4-one de formule (III) en deux stades, le premier stade consistant à la faire réagir sur un réactif d'oximation de formule R5-(N=O)m dans lequel R5 est alcoxy inférieur au chlore, quand m est égal à 1 et est une liaison supplémentaire entre les deux atomes d'azote quand m est égal à 2, pour obtenir l'oxime de formule

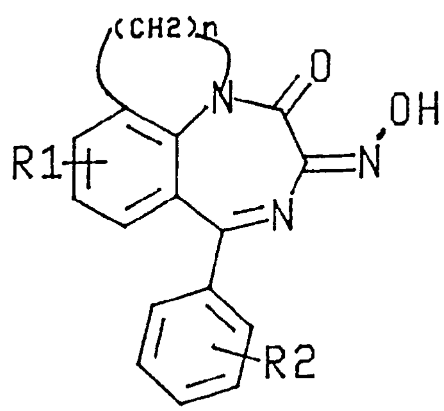

(IV)

que l'on isole, et le second stade consistant à réduire l'oxime catalytiquement par de l'hydrogène en présence d'un catalyseur de réduction ou par réaction avec du zinc en présence d'acide acétique, ou avec du chlorure stanneux en présence d'acide chlorhydrique pour obtenir le dérivé aminé.

10. Procédé suivant la revendication 1, caractérisé en ce qu'il consiste à préparer l'amine de formule (V) en mettant une benzodiazépine-[1,4]-one de formule (III) à réagir en milieu basique sur un réactif susceptible d'introduire une fonction azoture sur un carbanion pour obtenir un azoture de formule

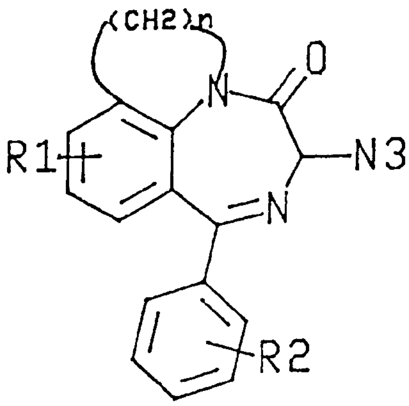

(VI)

11. Procédé de préparation d'un médicament pour lutter principalement contre les troubles gastro-intestinaux, du pancréas, de la vésicule biliaire et de l'appétit ; la douleur ; et éventuellement contre les troubles du système nerveux central, caractérisé en ce qu'il consiste à mélanger une benzodiazépine telle que définie à l'une des revendications 1 à 7 à un excipient pharmaceutiquement acceptable.

12. L'utilisation d'une benzodiazépine telle que définie aux revendications 1 à 7 pour l'obtention d'un médicament destiné à lutter principalement contre les troubles gastro-intestinaux, du pancréas, de la vésicule biliaire et de l'appétit, la douleur, et éventuellement contre les troubles du système nerveux central.

13. Procédé de préparation d'intermédiaires de formule

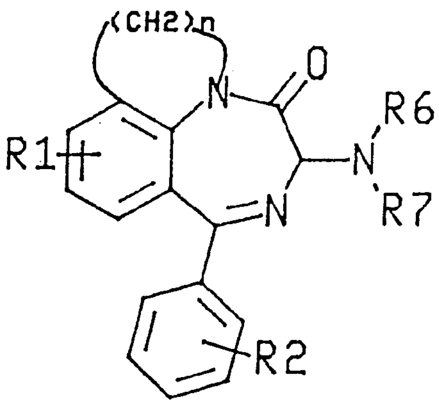

(XI)

n, R1 et R2 ayant les significations indiquées à la revendication 1, R6 étant hydroxy quand R7 représente une liaison supplémentaire entre l'atome d'azote portant R6 et le cycle diazépine et R6 étant l'hydrogène quand R7 est l'hydrogène, caractérisé en ce qu'il consiste à faire réagir une benzodiazépine-[1,4]-one de formule

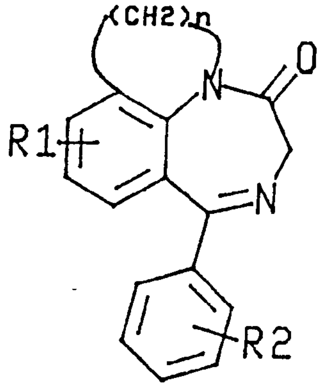

(III)

sur un réactif d'oximation de formule $R_5$-(N=O)$_m$ dans lequel $R_5$ est alcoxy inférieur ou chlore quand m est égal à 1 et est une liaison supplémentaire entre les atomes d'azote quand m est égal à 2 pour obtenir les intermédiaires de formule XI dans lesquels $R_6$ est hydroxy et pour obtenir ceux dans lesquels $R_6$ et $R_7$ sont l'hydrogène à réduire ceux dans lesquels $R_5$ est hydroxy par de l'hydrogène en présence d'un catalyseur de réduction ou par réaction avec du zinc en présence d'acide acétique ou avec du chlorure stanneux en présence d'acide chlorhydrique ou en amenant en la position alpha par rapport au carbonyle une benzodiazépine-[1,4]-one de formule (III) par un dérivé d'hydroxylamine ou par la chloramine.

**Revendications pour l'Etat contractant suivant : GR**

1. Benzodiazépines de formule

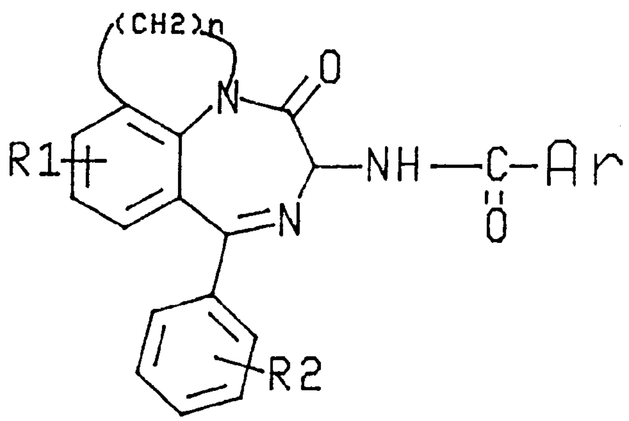

(I)

dans laquelle R1 est H ou halogène, R2 est H ou halogène, Ar est indolyle, phényle, naphtyle, indolyle monosubstitué par un halogène ou par un méthoxy, ou phényle mono di ou tri-substitué par un halogène ou par un méthoxy ou par un groupe trifluorométhyle, n est 2 ou 3 ; et leurs isomères optiques.

2. Benzodiazépines suivant la revendication 1, caractérisée en ce que Ar est le groupe indolyle-2.

3. Benzodiazépines suivant la revendication 1 ou 2, caractérisée en ce que R2 est l'hydrogène ou le fluor en position ortho par rapport à l'atome de carbone reliant le cycle phényle au cycle diazépine.

4. Benzodiazépines suivant l'une quelconque des revendications 1 à 3, caractérisée en ce que R1 est l'hydrogène ou le chlore en position para par rapport à l'atome d'azote commun au cycle diazépine et à l'autre hétérocycle azoté condensé avec le cycle diazépine.

5. Benzodiazépines suivant l'une quelconque des revendications 1 à 4, caractérisée en ce que n est égal à 2.

6. Benzodiazépines suivant l'une quelconque des revendications 1 à 5 caractérisée en ce que l'atome de carbone asymétrique en position alpha par rapport au carbonyle du cycle diazépine possède la configuration S suivant la nomenclature de Cahn, Ingold et Prelog.

7. Benzodiazépines suivant la revendication 1 qui sont :
   a) l'(indolyl-2-carbonylamino)-4-phényl-6-tétrahydro-1,2,3,4-pyrrolo[3,2,1-j,k]benzodiazépin- [1,4]-one-3, et de préférence son isomère 4S.
   b) la (fluoro-2-phényl)-6-[(indolyl-2)-carbonylamino]-4-tétrahydro-1,2,3,4-pyrrolo[3,2,1- j,k]-benzodiazépin-[1,4]-one-3, et de préférence son isomère 4S.
   c) la chloro-8-(fluoro-2-phényl)-6-[(indolyl-2)-carbonylamino]-4-tétrahydro-1,2,3,4-pyrrolo[3,2,1- j,k]benzodiazépin-1,4-one-3, et de préférence son isomère 4S.
   d) l'[(indolyl-2)-carbonylamino]-5-phényl-7-hexahydro-1,2,3,3a,4,5-pyrido[3,2,1-j,k] benzodiazépin-[1,4]-one-4, et de préférence son isomère 5S.

8. Procédé de préparation de benzodiazépines de formule (I) à la revendication 1, caractérisé en ce qu'il consiste

a) pour préparer un benzodiazépine de formule (I) dans sa forme racémique :
à condenser une benzodiazépine-1,4 de formule

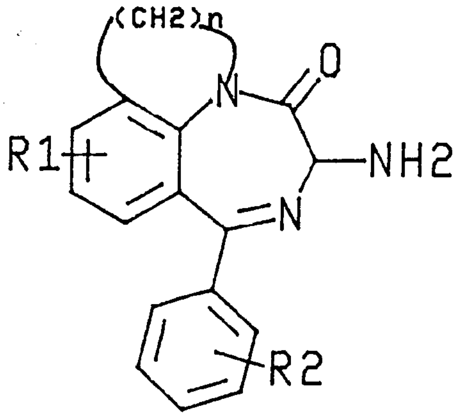

(V)

sur un dérivé d'acide carboxylique de formule Ar-CO-X dans laquelle Ar a la signification indiquée à la revendication 1 et X est un halogène, un groupe azido (-N3), un groupe imidazol-1-yle, un groupe -O-CO-R3, R3 pouvant être, outre de préférence Ar, un radical alkyle encombré comportant de trois à six atomes de carbone, ou aryle plus encombré que le radical Ar, substitué par un ou plusieurs halogènes, ou un groupe -OR4, R4 étant un groupe aromatique comportant un ou deux cycles, substitué par un ou plusieurs groupes nitro ou halogène, ou un groupe hétéroaromatique condensé comportant de un à trois hétéroatomes

b) pour préparer une benzodiazépine de formule (I) dans une forme optiquement active, dextrogyre ou lévogyre
à condenser une benzodiazépine racémique de formule (V) avec une molécule dérivée d'un amino-acide optiquement actif de formule (X) :

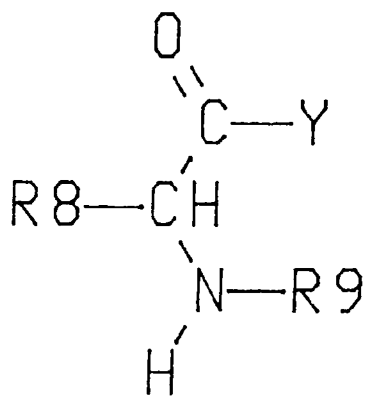

(X)

dans laquelle

Y est un groupe hydroxy, un groupe azido (-N3), un groupe imidazol-1-yl, un groupe O-CO-R3, R3 pouvant être un radical alkyle ramifié comportant de trois à six atomes de carbone, un groupe aryle encombré substitué par un ou plusieurs halogènes, ou un groupe -OR4, R4 étant un groupe aromatique comportant un ou deux cycles éventuellement substitué par un ou plusieurs radicaux nitro ou halogènes, ou un groupe hétéroaromatique condensé comportant de un à trois hétéroatomes.

R8 est

- un groupement alkyle ayant de 1 à 6 atomes de carbone éventuellement substitué par un hydroxyle, un groupe thioalkyle ayant de un à six atomes de carbone dans le groupe alkyl ou un groupe carboxyle ou carbonylamino,
- un groupe aryle comportant un ou deux cycles éventuellement substitué par un hydroxyle,
- un groupe aralkyle ayant un ou deux cycles aromatiques et dont la portion alkylique comporte de un à six atomes de carbone, éventuellement substitué sur le cycle par un ou plusieurs groupes halogènes, hydroxy ou méthoxy

- un hétérocycle ayant cinq ou six chainons, et un ou deux hétéroatomes choisis parmi l'azote, l'oxygène et le soufre
- un groupe indolyl-3-méthyle,
- un groupe imidazolyl-4-méthyle.

R9 est un groupement pouvant être enlevé facilement pour régénérer l'amine libre et peut être
- un radical oxycarbonyle de structure A-O-CO-dans lequel A est un groupe alkyle comportant de un à six atomes de carbone, ou un groupe aryle, éventuellement substitué par un ou plusieurs groupes méthoxy, halogène ou nitro,
- un groupe alkanoyle ou alkènoyle comportant de un à six atomes de carbone ou aroyle
- p-nitrosulfényle,

pour obtenir un composé optiquement actif de formule

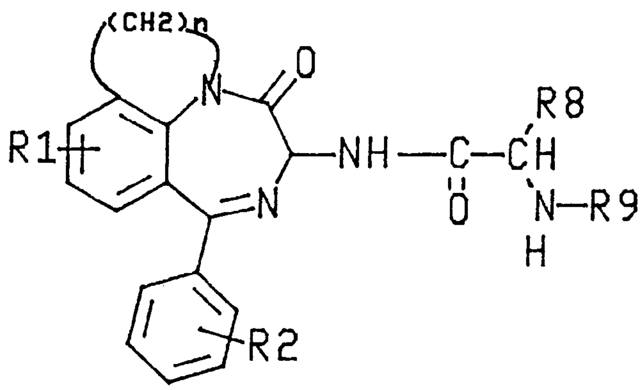

(VII)

que

i) dans le cas où R9 est benzyloxycarbonyl éventuellement substitué, on hydrogène en présence d'un catalyseur choisi parmi les métaux nobles déposés sur un support ou

ii) dans tous les autres cas on hydrolyse en milieu acide ou basique pour obtenir tant dans le cas i) que dans le cas ii) un mélange de deux diastéréoisomères de formule (VIII)

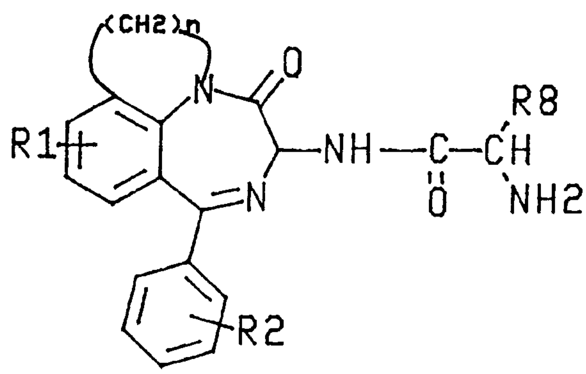

(VIII)

puis à séparer ces distéréoisomères par un procédé physicochimique choisi parmi une méthode chromatographique et/ou une cristallisation fractionnée de la base de formule VIII ou d'un de ses sels d'addition avec un acide, puis à faire réagir un arylisothiocyanate de formule R10-N=C=S dans laquelle R10 est un radical phényle ou naphtyle éventuellement substitué par un ou plusieurs substituants choisis parmi les halogènes, le groupe méthoxy ou un groupement alkyle ayant de un à six atomes de carbone sur l'un au moins des diastéréoisomères pour obtenir une thiourée optiquement active de formule (IX) :

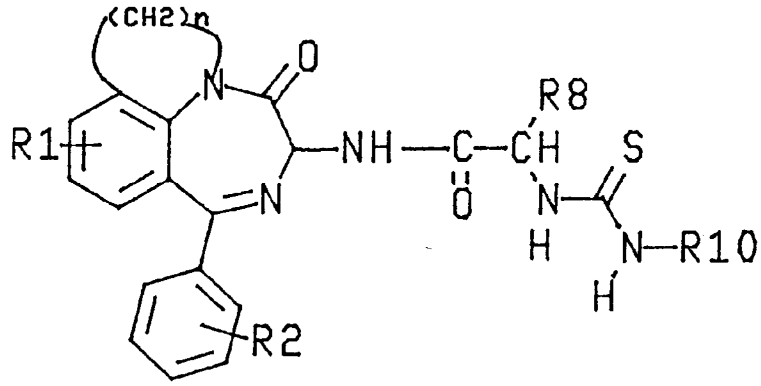

(IX)

puis à effectuer sur la thiourée de formule IX une réaction d'élimination du radical amino acide en la traitant par un acide pour obtenir l'amine (V) optiquement active que l'on met à réagir ensuite comme dans le cas a) de la préparation de la benzodiazépine racémique, pour obtenir la benzodiazépine de formule I optiquement active.

**9.** Procédé suivant la revendication 8, caractérisé en ce qu'il consiste à préparer l'amine de formule (V) en aminant en la position alpha par rapport au carbonyle une benzodiazépine-1,4-one de formule

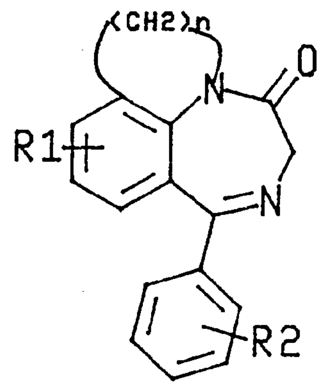

(III)

par un dérivé d'hydroxylamine ou par la chloramine.

**10.** Procédé suivant la revendication 8, caractérisé en ce qu'il consiste à aminer la benzodiazépine-1,4-one de formule (III) en deux stades, le premier stade consistant à la faire réagir sur un réactif d'oximation de formule R5-(N=O)m dans lequel R5 est alcoxy inférieur ou chlore, quand m est égal à 1 et est une liaison supplémentaire entre les deux atomes d'azote quand m est égal à 2, pour obtenir l'oxime de formule

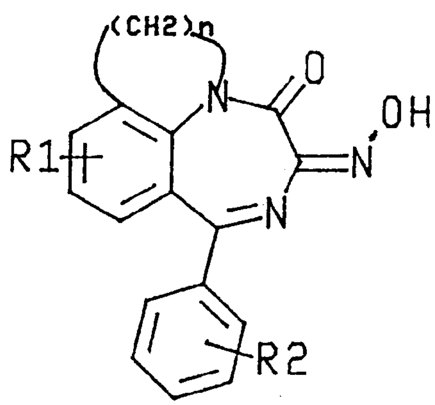

(IV)

que l'on isole, et le second stade consistant à réduire l'oxime catalytiquement par de l'hydrogène en présence d'un catalyseur de réduction ou par réaction avec du zinc en présence d'acide acétique, ou avec

du chlorure stanneux en présence d'acide chlorhydrique pour obtenir le dérivé aminé.

**11.** Procédé suivant la revendication 8, caractérisé en ce qu'il consiste à préparer l'amine de formule (V) en mettant une benzodiazépine-[1,4]-one de formule (III) à réagir en milieu basique sur un réactif susceptible d'introduire une fonction azoture sur un carbanion pour obtenir un azoture de formule

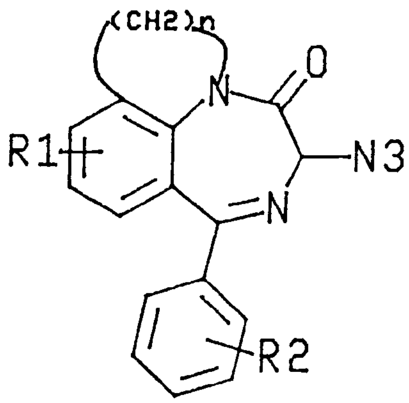

(VI)

**12.** Procédé de préparation d'un médicament pour lutter principalement contre les troubles gastro-intestinaux, du pancréas, de la vésicule biliaire et de l'appétit; la douleur ; et éventuellement contre les troubles du système nerveux central, caractérisé en ce qu'il consiste à mélanger une benzodiazépine selon l'une des revendications 1 à 7 à un excipient pharmaceutiquement acceptable.

**13.** L'utilisation d'une benzodiazépine telle que définie aux revendications 1 à 7 pour l'obtention d'un médicament destiné à lutter principalement contre les troubles gastro-intestinaux, du pancréas, de la vésicule biliaire et de l'appétit, la douleur, et éventuellement contre les troubles du système nerveux central.

**14.** Intermédiaires de formule

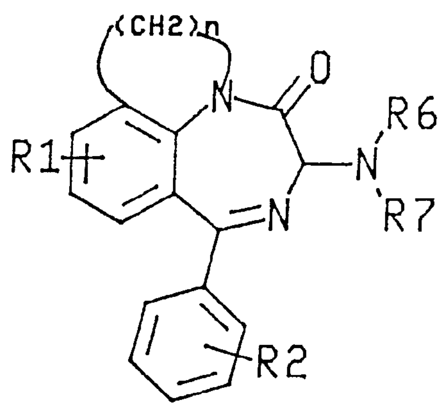

(XI)

n, R1 et R2 ayant les significations indiquées à la revendication 1, R6 étant hydroxy quand R7 représente une liaison supplémentaire entre l'atome d'azote portant R6 et le cycle diazépine et R6 étant l'hdrogène quand R7 est l'hdrogène, caractérisée en ce qu'il consiste à faire réagir une benzodiazépine -[1,4]-one de formule

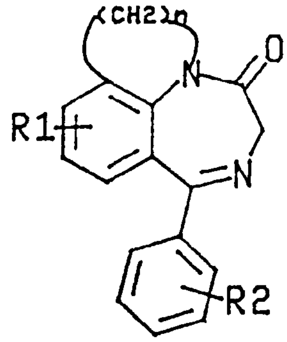

(III)

sur un réactif d'oximation de formule $R_5$-(N=O)$_m$ dans lequel $R_5$ est alcoxy inférieur ou chlore quand m est égal à 1 et est une liaison supplémentaire entre les atomes d'azote quand m est égal à 2 pour obtenir les intermédiaires de formule XI dans lesquels $R_6$ est hydroxy et pour obtenir ceux dans lesquels $R_6$ et $R_7$ sont l'hydrogène à réduire ceux dans lesquels $R_5$ est hydroxy par de l'hydrogène en présence d'un catalyseur de réduction ou par réaction avec du zinc en présence d'acide acétique ou avec du chlorure stanneux en présence d'acide chlorhydrique ou en amenant en la position alpha par rapport au carbonyle une benzodiazépine-[1,4]-one de formule (III) par un dérivé d'hydroxylamine ou par la chloramine.

**Patentansprüche**

**Patentansprüche für folgende Vertragsstaaten : AT, BE, CH, DE, FR, GB, IT, LI, LU, NL, SE**

1. Benzodiazepine der nachstehenden Formel (I)

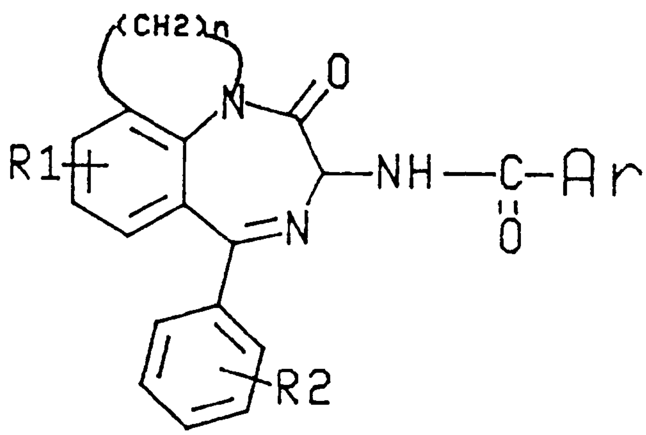

(I)

wobei
der Rest R1 steht für Wasserstoff oder Halogen,
der Rest R2 steht für Wasserstoff oder Halogen,
die Gruppe Ar steht für eine Indolylgruppe, für eine Phenylgruppe, für eine Naphtylgruppe, für eine monosubstituierte Indolylgruppe, die mit einem Halogenrest oder mit einem Methoxyrest substitutiert ist, oder für eine mono, di- oder trisubstituierte Phenylgruppe, die mit einem oder mehreren Halogenrest(en), mit einem oder mehreren Methoxyrest(en) oder mit einer oder mehreren Trifluormethylgruppe(n) substituiert ist,
n hat den Wert "2" oder "3";
sowie deren optische Isomere.

2. Benzodiazepine nach Anspruch 1,
<u>dadurch gekennzeichnet,</u> daß
die Gruppe Ar für eine Indolyl-2-Gruppe steht.

3. Benzodiazepine nach Anspruch 1 oder 2,

dadurch gekennzeichnet, daß der Rest R2 Wasserstoff oder Fluor in Orthostellung ist zu dem C-Atom am Phenylring, das mit dem Diazepinring verbunden ist.

4. Benzodiazepine nach einem der Ansprüche 1 bis 3, dadurch gekennzeichnet, daß der Rest R1 Wasserstoff oder Chlor in Parastellung ist zu dem gemeinsamen N-Atom im Diazepinring und im heterozyklischen Ring, der mit dem Diazepinring kondensiert ist.

5. Benzodiazepine nach einem der Ansprüche 1 bis 4, dadurch gekennzeichnet, daß n den Wert "2" hat.

6. Benzodiazepine nach einem der Ansprüche 1 bis 5, dadurch gekennzeichnet, daß das asymmetrische C-Atom in $\alpha$-Stellung zur Carbonylgruppe am Diazepinring S-Konfiguration einnimmt entsprechend der Nomenklatur von Cahn, Ingold und Prelog.

7. Benzodiazepine nach Anspruch 1, nämlich
   a) (Indolyl-2-carbonylamino)4-phenyl-6-tetrahydro-1,2,3,4-pyrrolo[3,2,1-j,k]benzodiazepin-[1,4]-on-3, und hier vorzugsweise das 4S-Isomere;
   b (Fluor-2-phenyl)-6-[(indolyl-2)-carbonylamino]4-tetrahydro-1,2,3,4-pyrrolo [3,2,1-j,k]-benzodiazepin-[1,4]-on-3, und hier vorzugsweise das 4S-Isomere;
   c) Chlor-8-(fluor-2-phenyl)-6-[(indolyl-2)-carbonylamino]4-tetrahydro-1 ,2,3,4-pyrrolo[3,2,1-j,k]-benzodia zepin-1,4-on-3, und hier vorzugsweise das 4S-Isomere;
   d) [(Indolyl-2)-carbonylamino]-5-phenyl-7-hexahydro-1,2,3,3a,4,5-pyrido[3,2,1-j,k]-benzodiazepin-[1,4]-o n-4, und hier vorzugsweise das 5S-Isomere.

8. Verfahren zur Herstellung eines Benzodiazepin der Formel (I) nach Anspruch 1, gekennzeichnet durch die Verfahrensschritte:
   a) zur Erzeugung eines Benzodiazepin der Formel (I) in Form des Racemates wird ein Benzodiazepin-1,4 der nachstehenden Formel (V)

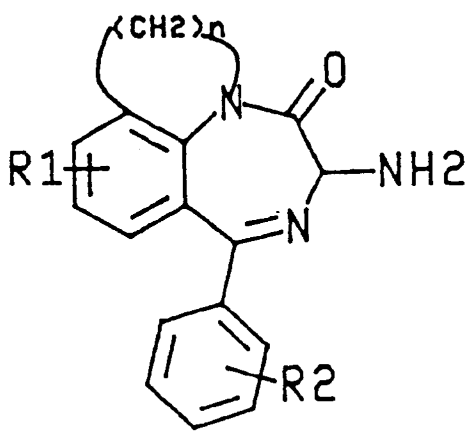

(V)

mit einem Carbonsäurederivat Ar-CO-X kondensiert,
wobei
die Gruppe Ar hat die in Anspruch 1 angegebene Bedeutung,
X steht für Halogen, eine Azidogruppe ($-N_3$), eine Imidazol-1-yl-Gruppe, oder für ei ne Gruppe -O-CO-R3,
wobei
R3 über die bevorzugte Gruppe Ar hinaus sein kann ein sperriger $C_{3-6}$-Alkylrest, oder eine Arylgruppe, welche noch sperriger als die Gruppe Ar ist und welche zusätzlich mit einem oder mehreren Halogenatom(en) substituiert sei n kann, oder ei ne Gruppe -OR4,
wobei

R4 steht für eine aromatische Gruppe, die einen Ring oder zwei Ringe aufweist und die mit einem oder mehreren Nitro- oder Halogenrest (en) substituiert ist, oder für eine kondensierte heteroaromatische Gruppe, die ein bis drei Heteroatom(e) enthält;

b) zur Erzeugung eines Benzodiazepin der Formel (I) in optisch aktiver Form
wird ein Benzodiazepin-Racemat der Formel (V) rechtsdrehend oder linksdrehend kondensiert mit einem Molekül, das abgeleitet ist von einer optisch aktiven Aminosäure der nachstehenden Formel (X)

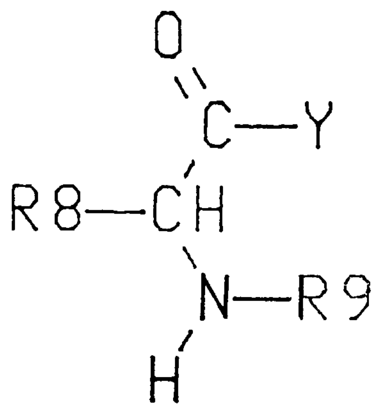

(X)

wobei
Y steht für einen Hydroxyrest, für einen Azidorest (-$N_3$), für eine Imidazol-1-yl-Gruppe, oder für eine Gruppe -O-CO-R3,
wobei
R3 sein kann eine verzweigte $C_{3-6}$-Alkylgruppe, eine sperrige Arylgruppe, die mit einem oder mehreren Halogenatomen substituiert sein kann, oder eine Gruppe -OR4,
wobei
R4 für eine aromatische Gruppe steht, die einen Ring oder zwei Ringe aufweist, und die wahlweise mit einem oder mehreren Nitro- oder Halogenrest(en) substituiert sei n kann, oder für ei ne kondensierte heteroaromatische Gruppe, die ein bis drei Heteroatom(e) enthält;
R8 steht für

. eine $C_{1-6}$-Alkylgruppe, die wahlweise substituiert ist mit einem Hydroxylrest, mit einer Thioalkylgruppe mit einer $C_{1-6}$-Alkylgruppe, mit einer Carboxylgruppe oder mit einer Carbonylaminogruppe,

. eine Arylgruppe, die einen Ring oder zwei Ringe aufweist, die wahlweise mit einem Hydroxylrest substituiert sind,

. eine Arylalkylgruppe mit einem oder zwei aromatischen Ring(en) mit einer $C_{1-6}$-Alkylgruppe, wobei der/die Ring(e) wahlweise substituiert sein können mit einem oder mehreren Halogenrest(en), Hydroxylrest(en) oder Methoxyrest(en),

. einen heterozyklischen Ring mit fünf oder sechs Ringgliedern und mit ein oder zwei Heteroatomen, nämlich Stickstoff, Sauerstoff oder Schwefel,

. eine Indolyl-3-methyl-Gruppe,

. eine Imidazolyl-4-methyl-Gruppe.

R9 steht für
eine Gruppe, die sich leicht von einem freien Amin ablöst und dieses gegebenenfalls auch wieder bildet, und sein kann

. eine Oxycarbonylgruppe der Struktur A-O-CO-, wobei A steht für eine $C_{1-6}$Alkylgruppe oder für eine Arylgruppe, die wahlweise mit einem oder mehreren Methoxy-, Halogen- oder Nitro-Rest(en) substituiert sein kann,

. eine $C_{1-6}$-Alkanoylgruppe, oder für eine $C_{1-6}$-Alkenoylgruppe oder für ei ne Aroylgruppe,

. eine p-Nitrosulfenyl-Gruppe;

zur Erzeugung einer optisch aktiven Verbindung der nachstehenden Formel (VII)

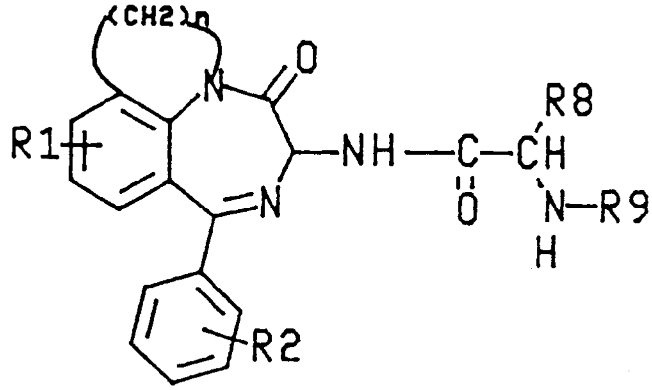

(VII)

wobei
i) wird für den Fall, daß R9 für einen Benzyloxycarbonyl-Rest steht, der wahlweise substituiert sein kann, in Gegenwart eines Edelmetall-Träger-Katalysators hydriert,
ii) wird im Fall aller anderen Substituenten in saurem oder basischem Milieu hydrolysiert, wobei sowohl im Falle (i) wie im Fall (ii) ein Gemisch der beiden Diastereo-isomeren der nachstehenden Formel (VIII) anfällt:

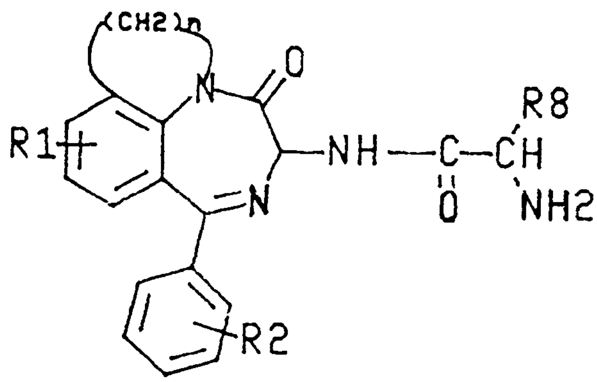

(VIII)

das erhaltene Diastereo-isomeren-Gemisch (VIII) wird nach einem physikalisch/chemischen Verfahren aufgetrennt, nämlich mit Hilfe der Chromatographie und/oder mit Hilfe der fraktionierten Kristallisation einer Verbindung (VIII) oder eines Additionssalzes der Verbindung (VIII) mit einer Säure;

danach wird mit einem Arylisothiocyanat der Formel R10-N=C=S umgesetzt, wobei
R10 steht für einen Phenylrest oder für einen Naphtylrest, die wahlweise mit einem oder mehreren Halogen-, Methoxy- oder $C_{1-6}$-Alkyl-Rest(en) substituiert sein können,
um mindestens ein Diastereo-isomeres zu erhalten in Form eines optisch aktiven Thioharnstoffes mit der nachstehenden Formel (IX):

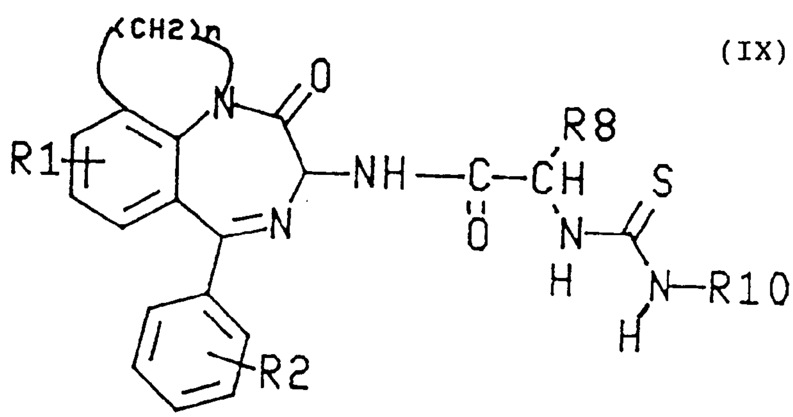

(IX)

daraufhin wird der optisch aktive Thioharnstoff (IX) umgesetzt, um die Aminosäuregruppe zu beseitigen

und um eine Säurebehandlung durchzuführen, um das optisch aktive Amin (V) zu erhalten, das nunmehr mit einem geeigneten Reagens umgesetzt wird, um im Falle (a) das Benzodiazepin-Racemat zu erhalten, oder um das optisch aktive Benzodiazepin (I) zu erhalten.

**9.** Verfahren nach Anspruch 8,
dadurch gekennzeichnet, daß
zur Erzeugung eines Amin (V) wird ein Benzodiazepin-1,4-on der nachstehenden Formel (III)

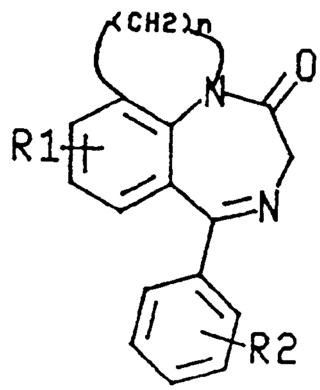

(III)

in α-Stellung zur Carbonylgruppe aminiert mit einem Hydroxylamin-Derivat oder mit Chloramin.

**10.** Verfahren nach Anspruch 8,
dadurch gekennzeichnet, daß
eine Aminierung des Benzodiazepin-1,4-on (III) in zwei Stufen durchgeführt wird, wobei
in einer ersten Stufe mit einem Oximbildungsmittel R5-$(N=O)_m$ umgesetzt wird, wobei
R5 steht für einen niederen Alkoxyrest oder für Chlor,
m hat den Wert "1 ", oder m hat den Wert "2", wenn eine zusätzliche Bindung zwischen den beiden Stickstoffatomen vorhanden ist, um ein Oxim der nachstehenden Formel (IV) zu erhalten, das isoliert wird:

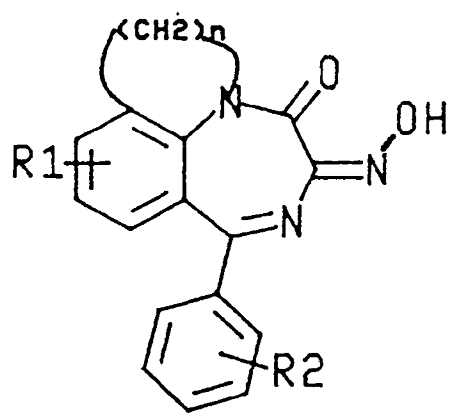

(IV)

in der zweiten Stufe wird das Oxim (IV) katalytisch in Gegenwart eines Reduktionskatalysators mit Wasserstoff reduziert, oder es wird mit Zink in Gegenwart von Essigsäure umgesetzt, oder es wird mit Zinn(II)chlorid in Gegenwart von Salzsäure umgesetzt, um das Aminderivat zu erhalten.

**11.** Verfahren nach Anspruch 8,
dadurch gekennzeichnet, daß
zur Erzeugung des Amin (V) ein Benzodiazepin-[1,4]-on (III) in basischem Milieu mit einem Reagens umgesetzt wird, das in das Carbanion eine Stickstoff-Funktion ($N_3$) einführt, um ein Stickstoffderivat der nachstehenden Formel (VI) zu erhalten:

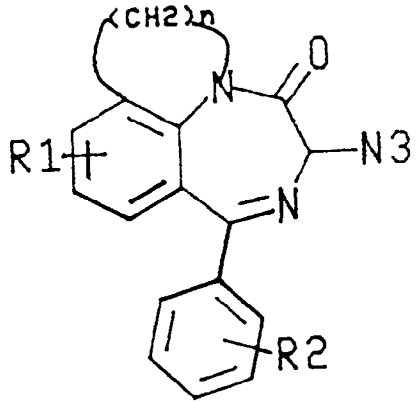

(VI)

12. Arzneimittel, insbesondere zur Bekämpfung von Beschwerden des Magen-Darm-Traktes, der Bauchspeicheldrüse, der Gallenblase, bei Appetitstörungen, als Schmerzmittel und wahlweise zur Bekämpfung von Störungen des zentralen Nervensystems,
dadurch gekennzeichnet, daß
das Arzneimittel als Wirkstoff ein Benzodiazepin nach einem der Ansprüche 1 bis 7 enthält.

13. Zwischenprodukte der nachstehenden Formel (XI)

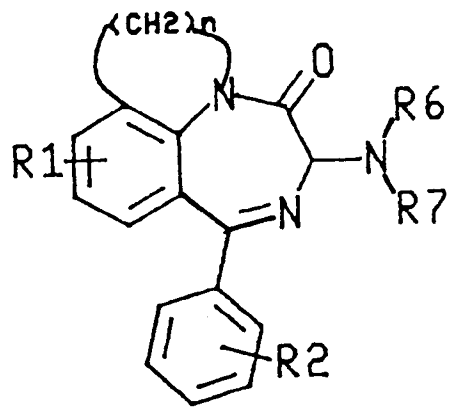

(XI)

wobei
n, R1 und R2 die in Anspruch 1 angegebene Bedeutung haben,
R6 für den Hydroxylrest steht, wenn R7 eine zusätzliche Bindung zwischen dem, den Rest R6 tragenden N-Atom und dem Diazepinring bildet, und R6 für den Wasserstoffrest steht, wenn R7 für Wasserstoff steht.

**Patentansprüche für folgenden Vertragsstaat : ES**

1. Verfahren zur Herstellung von Benzodiazepinen der nachstehenden Formel (I)

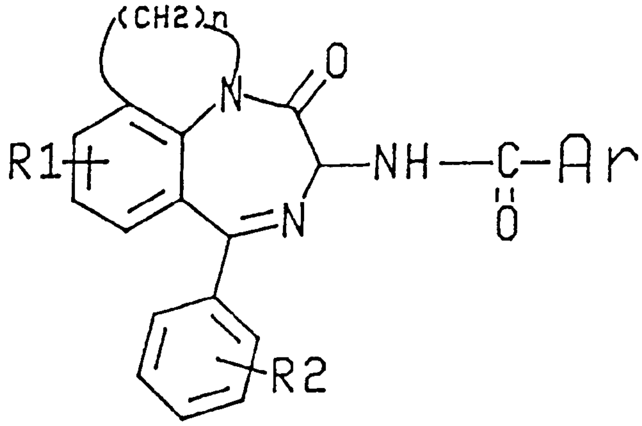

(I)

wobei
der Rest R1 steht für Wasserstoff oder Halogen,
der Rest R2 steht für Wasserstoff oder Halogen,
die Gruppe Ar steht für eine Indolylgruppe, für eine Phenylgruppe, für eine Naphtylgruppe, für eine monosubstituierte Indolylgruppe, die mit einem Halogenrest oder mit einem Methoxyrest substitutiert ist, oder für eine mono, di- oder trisubstituierte Phenylgruppe, die mit einem oder mehreren Halogenrest(en), mit einem oder mehreren Methoxyrest(en) oder mit einer oder mehreren Trifluormethylgruppe(n) substituiert ist,
n hat den Wert "2" oder "3";
sowie deren optische Isomere,
gekennzeichnet durch die Verfahrensschritte:

a) zur Erzeugung eines Benzodiazepin der Formel (I) in Form des Racemates
wird ein Benzodiazepin-1,4 der nachstehenden Formel (V)

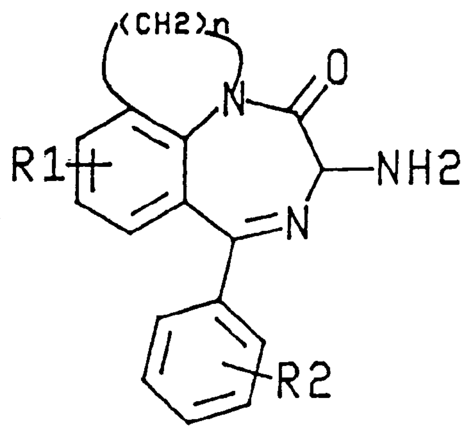

(V)

mit einem Carbonsäurederivat Ar-CO-X kondensiert,
wobei
die Gruppe Ar hat die in Anspruch 1 angegebene Bedeutung,
X steht für Halogen, eine Azidogruppe ($-N_3$), eine Imidazol-1-yl-Gruppe, oder für ei ne Gruppe -O-CO-R3,
wobei
R3 über die bevorzugte Gruppe Ar hinaus sein kann ein sperriger $C_{3-6}$-Alkylrest, oder eine Arylgruppe, welche noch sperriger als die Gruppe Ar ist und welche zusätzlich mit einem oder mehreren Halogenatom(en) substituiert sein kann, oder eine Gruppe -OR4,
wobei
R4 steht für eine aromatische Gruppe, die einen Ring oder zwei Ringe aufweist und die mit einem oder mehreren Nitro- oder Halogenrest(en) substituiert ist, oder für eine kondensierte heteroaromatische Gruppe, die ein bis drei Heteroatom(e) enthält;

b) zur Erzeugung eines Benzodiazepin der Formel (I) in optisch aktiver Form

wird ein Benzodiazepin-Racemat der Formel (V) rechtsdrehend oder linksdrehend kondensiert mit einem Molekül, das abgeleitet ist von einer optisch aktiven Aminosäure der nachstehenden Formel (X)

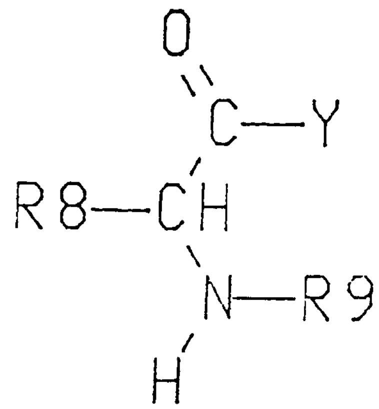

(X)

wobei
Y steht für einen Hydroxyrest, für einen Azidorest ($-N_3$), für eine Imidazol-1-yl-Gruppe, oder für eine Gruppe -O-CO-R3,
wobei
R3 sein kann eine verzweigte $C_{3-6}$-Alkylgruppe, eine sperrige Arylgruppe, die mit einem oder mehreren Halogenatomen substituiert sein kann, oder eine Gruppe -OR4,
wobei
R4 steht für eine aromatische Gruppe, die einen Ring oder zwei Ringe aufweist, und die wahlweise mit einem oder mehreren Nitro-oder Halogenrest(en) substituiert sein kann, oder für eine kondensierte heteroaromatische Gruppe, die ei n bis drei Heteroatom(e) enthält;
R8 steht für

. eine $C_{1-6}$-Alkylgruppe, die wahlweise substituiert ist mit einem Hydroxylrest, mit einer Thioalkylgruppe mit einer $C_{1-6}$-Alkylgruppe, mit einer Carboxylgruppe oder mit einer Carbonylaminogruppe,
. eine Arylgruppe, die einen Ring oder zwei Ringe aufweist, die eventuell mit einem Hydroxylrest substituiert sind,
. eine Arylalkylgruppe mit einem oder zwei aromatischen Ring(en) mit einer $C_{1-6}$-Alkylgruppe, wobei der/die Ring(e) wahlweise substituiert sein können mit einem oder mehreren Halogenrest(en), Hydroxylrest(en) oder Methoxyrest(en),
. einen heterozyklischen Ring mit fünf oder sechs Ringgliedern und mit ein oder zwei Heteroatomen, nämlich Stickstoff, Sauerstoff oder Schwefel,
. eine Indolyl-3-methyl-Gruppe,
. eine Imidazolyl-4-methyl-Gruppe.

R9 steht für
eine Gruppe, die sich leicht von einem freien Amin ablöst und dieses gegebenenfalls auch wieder bildet, und sein kann

. eine Oxycarbonylgruppe der Struktur A-O-CO-, wobei A steht für eine $C_{1-6}$-Alkylgruppe oder für eine Arylgruppe, die wahlweise mit einem oder mehreren Methoxy-, Halogen- oder Nitro-Rest(en) substituiert sein kann,
. eine $C_{1-6}$-Alkanoylgruppe, oder für eine $C_{1-6}$-Alkenoylgruppe oder für ei ne Aroylgruppe,
. eine p-Nitrosulfenyl-Gruppe;

zur Erzeugung einer optisch aktiven Verbindung der nachstehenden Formel (VII)

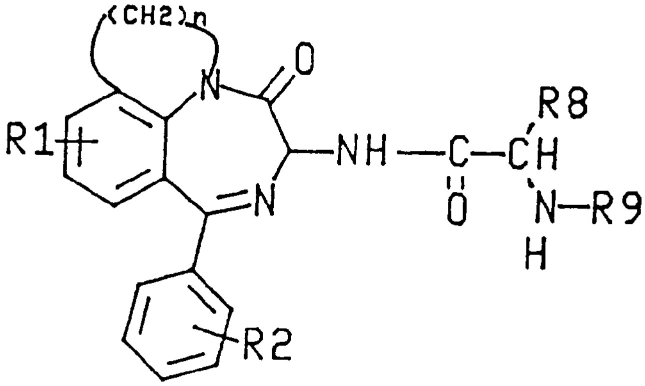

(VII)

wobei

i) wird für den Fall, daß R9 für einen Benzyloxycarbonyl-Rest steht, der wahlweise substituiert sein kann, in Gegenwart eines Edelmetall-Träger-Katalysators hydriert,

ii) wird im Fall aller anderen Substituenten in saurem oder basischem Milieu hydrolysiert, wobei sowohl im Falle (i) wie im Fall (ii) ein Gemisch der beiden Diastereo-isomeren der nachstehenden Formel (VIII) anfällt:

(VIII)

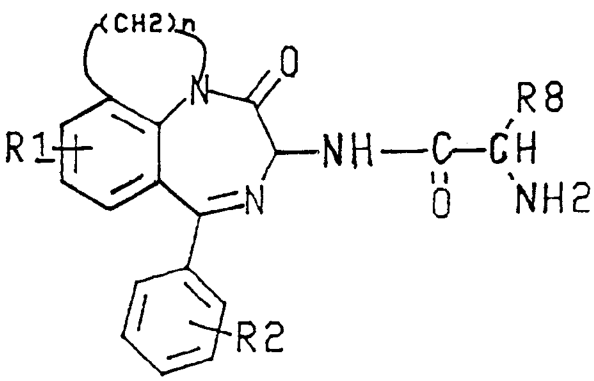

das erhaltene Diastereo-isomeren-Gemisch (VIII) wird nach einem physikalisch/chemischen Verfahren aufgetrennt, nämlich mit Hilfe der Chromatographie und/oder mit Hilfe der fraktionierten Kristallisation einer Verbindung (VIII) oder eines Additionssalzes der Verbindung (VIII) mit einer Säure;

danach wird mit einem Arylisothiocyanat der Formel R10-N=C=S umgesetzt,

wobei

R10 steht für einen Phenylrest oder für einen Naphtylrest, die wahlweise mit einem oder mehreren Halogen-, Methoxy- oder $C_{1-6}$-Alkyl-Rest(en) substituiert sein können,

um mindestens ein Diastereo-isomeres zu erhalten in Form eines optisch aktiven Thioharnstoffes mit der nachstehenden Formel (IX):

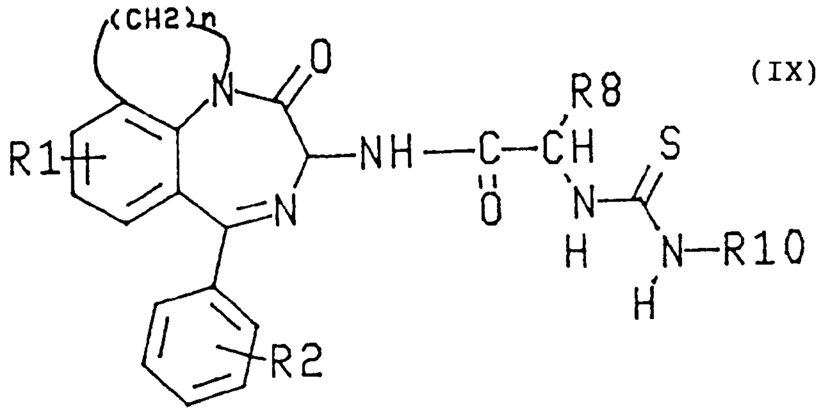

(IX)

darauf wird der optisch aktive Thioharnstoff (IX) umgesetzt, um die Aminosäuregruppe zu beseitigen und um eine Säurebehandlung durchzuführen, um das optisch aktive Amin (V) zu erhalten, das nunmehr mit einem geeigneten Reagens umgesetzt wird, um im Falle (a) das Benzodiazepin-Racemat zu erhalten, oder um das optisch aktive Benzodiazepin (I) zu erhalten.

2. Verfahren nach Anspruch 1,
dadurch gekennzeichnet, daß
die Gruppe Ar für eine Indolyl-2-Gruppe steht.

3. Verfahren nach Anspruch 1 oder 2,
dadurch gekennzeichnet, daß
der Rest R2 Wasserstoff oder Fluor in Orthostellung ist zu dem C-Atom am Phenylring, das mit dem Diazepinring verbunden ist.

4. Verfahren nach einem der Ansprüche 1 bis 3,
dadurch gekennzeichnet, daß
der Rest R1 Wasserstoff oder Chlor in Parastellung ist zu dem gemeinsamen N-Atom im Diazepinring und im heterozyklischen Ring, der mit dem Diazepi nri ng kondensiert ist.

5. Verfahren nach einem der Ansprüche 1 bis 4,
dadurch gekennzeichnet, daß
n den Wert "2" hat.

6. Verfahren nach einem der Ansprüche 1 bis 5,
dadurch gekennzeichnet, daß
das asymmetrische C-Atom in $\alpha$-Stellung zur Carbonylgruppe am Diazepinring S-Konfiguration einnimmt entsprechend der Nomenklatur von Cahn, Ingold und Prelog.

7. Verfahren zur Herstellung eines Benzodiazepin nach Anspruch 1,
nämlich
a) (Indolyl-2-carbonylamino)-4-phenyl-6-tetrahydro-1,2,3,4-pyrrolo[3,2,1-j,k]benzodiazepin-[1,4]-on-3, und hier vorzugsweise das 4S-Isomere;
b) (Fluor-2-phenyl)-6-[(indolyl-2)-carbonylamino]-4-tetrahydro-1,2,3,4-pyrrolo [3,2,1-j,k]-benzodiazepin-[1,4]-on-3, und hier vorzugsweise das 4S-Isomere;
c) Chlor-8-(fluor-2-phenyl)-6-[(indolyl-2)-carbonylamino]4-tetrahydro-1,2,3,4-pyrrolo[3,2,1-j,k]-benzodiazepin-1,4-on-3, und hier vorzugsweise das 4S-Isomere;
d) [(Indolyl-2)-carbonylamino]-5-phenyl-7-hexahydro-1,2,3,3a,4,5-pyrido[3,2,1-j,k]-benzodiazepin-[1,4]-on-4, und hier vorzugsweise das 5S-Isomere.

8. Verfahren nach Anspruch 1,
dadurch gekennzeichnet, daß
zur Erzeugung eines Amin (V) ein Benzodiazepin-1,4-on der nachstehenden Formel (III)

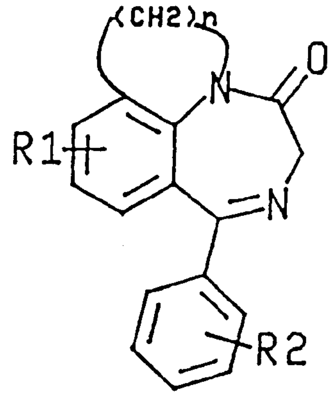

(III)

in α-Stellung zur Carbonylgruppe mit einem Hydroxylamin-Derivat oder mit Chloramin aminiert wird.

**9.** Verfahren nach Anspruch 1,
dadurch gekennzeichnet, daß
eine Aminierung des Benzodiazepin-1,4-on (III) in zwei Stufen durchgeführt wird, wobei
in einer ersten Stufe mit einem Oximbildungsmittel R5-$(N=O)_m$ umgesetzt wird, wobei
R5 steht für einen niederen Alkoxyrest oder für Chlor,
m hat den Wert "1", oder m hat den Wert "2", wenn eine zusätzliche Bindung zwischen den beiden Stickstoffatomen vorhanden ist, um ein Oxim der nachstehenden Formel (IV) zu erhalten, das isoliert wird:

(IV)

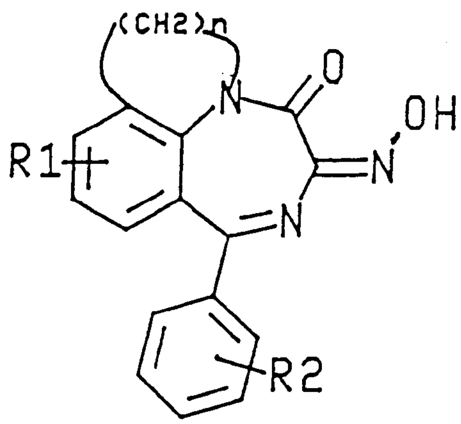

in der zweiten Stufe wird das Oxim (IV) katalytisch in Gegenwart eines Reduktionskatalysators mit Wasserstoff reduziert, oder es wird mit Zink in Gegenwart von Essigsäure umgesetzt, oder es wird mit Zinn(II)chlorid in Gegenwart von Salzsäure umgesetzt, um das Aminderivat zu erhalten.

**10.** Verfahren nach Anspruch 1,
dadurch gekennzeichnet, daß
zur Erzeugung des Amin (V) ein Benzodiazepin-[1,4]-on (III) in basischem Milieu mit einem Reagens umgesetzt wird, das in das Carbanion eine Stickstoff-Funktion ($N_3$) einführt, um ein Stickstoffderivat der nachstehenden Formel (VI) zu erhalten:

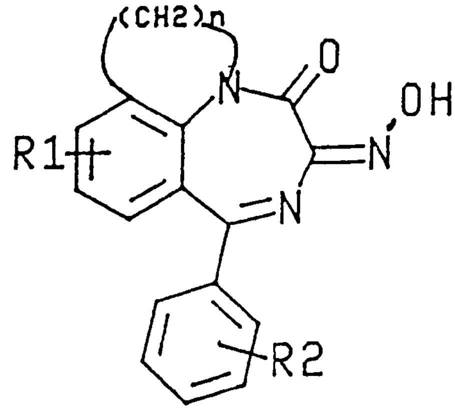

(VI)

11. Verfahren zur Herstellung eines Arzneimittels, insbesondere zur Bekämpfung von Beschwerden des Magen-Darm-Traktes, der Bauchspeicheldrüse, der Gallenblase, bei Appetitstörungen, als Schmerzmittel und wahlweise zur Bekämpfung von Störungen des zentralen Nervensystems,
dadurch gekennzeichnet, daß
ein nach einem der Ansprüche 1 bis 7 erhältliches Benzodiazepin mit einem pharmazeutischen Träger vermischt wird.

12. Die Verwendung eines Benzodiazepin, das nach einem der Ansprüche 1 bis 7 erhältlich ist, zur Herstellung eines Arzneimittels, insbesondere zur Bekämpfung von Beschwerden des Magen-Darm-Traktes, der Bauchspeicheldrüse, der Gallenblase, bei Appetitstörungen, als Schmerzmittel und wahlweise zur Bekämpfung von Störungen des zentralen Nervensystems.

13. Verfahren zur Herstellung von Zwischenprodukten der nachstehenden Formel (XI)

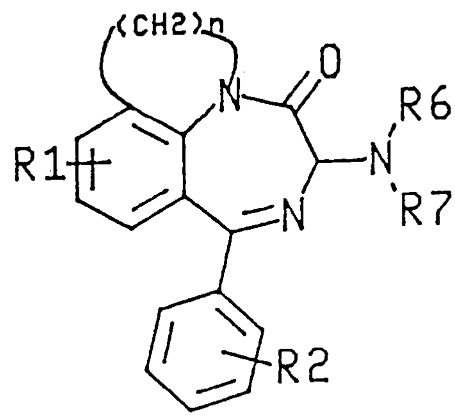

(XI)

wobei
n, R1 und R2 die in Anspruch 1 angegebene Bedeutung haben,
R6 für den Hydroxylrest steht, wenn R7 eine zusätzliche Bindung zwischen dem, den Rest R6 tragenden N-Atom und dem Diazepinring bildet,
und R6 für den Wasserstoffrest steht, wenn R7 für Wasserstoff steht,
dadurch gekennzeichnet, daß
zur Erzeugung von Zwischenprodukten der Formel (XI), wobei R6 für den Hydroxylrest steht,
ein Benzodiazepin-[1,4]-on der nachstehenden Formel (III)

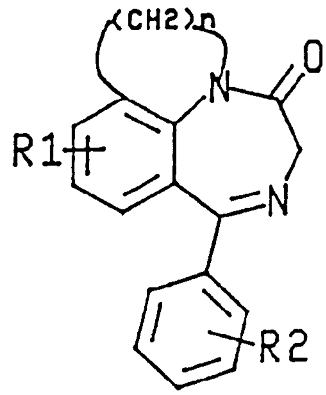

(III)

mit einem Oximbildungsmittel R5-(N = O)$_m$ umgesetzt wird, wobei R5 steht für einen niederen Alkoxyrest oder für Chlor, wenn m den Wert "1" hat, und eine zusätzlichen Bindung zwischen den beiden Stickstoffatomen vorhanden ist, wenn m den Wert "2" hat,

und daß ferner zur Erzeugung von Zwischenprodukten der Formel (XI), wobei R6 und R7 für Wasserstoff stehen,

jene Zwischenprodukte, in denen R6 für den Hydroxylrest steht, katalytisch in Gegenwart eines Reduktionskatalysators mit Wasserstoff reduziert werden oder mit Zink in Gegenwart von Essigsäure umgesetzt werden oder mit Zinn(II)chlorid in Gegenwart von Salzsäure umgesetzt werden, oder ein Benzodiazepin-[1,4]-on der Formel (III) mit einem Hydroxylamin-Derivat oder mit Chloramin in n-Stellung zur Carbonylgruppe aminiert wird.

**Patentansprüche für folgenden Vertragsstaat : GR**

1. Benzodiazepine der nachstehenden Formel (I)

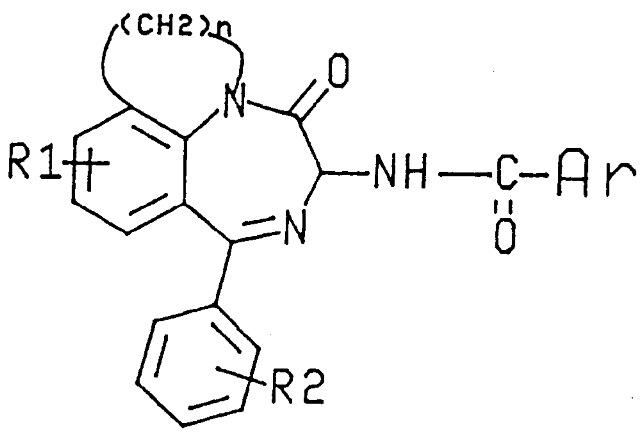

(I)

wobei
der Rest R1 steht für Wasserstoff oder Halogen,
der Rest R2 steht für Wasserstoff oder Halogen,
die Gruppe Ar steht für eine Indolylgruppe, für eine Phenylgruppe, für eine Naphtylgruppe, für eine monosubstituierte Indolylgruppe, die mit einem Halogenrest oder mit einem Methoxyrest substitutiert ist, oder für eine mono, di- oder trisubstituierte Phenylgruppe, die mit einem oder mehreren Halogenrest(en), mit einem oder mehreren Methoxyrest(en) oder mit einer oder mehreren Trifluormethylgruppe(n) substituiert ist,
n hat den Wert "2" oder "3";
sowie deren optische Isomere.

2. Benzodiazepine nach Anspruch 1,
<u>dadurch gekennzeichnet,</u> daß
die Gruppe Ar für eine Indolyl-2-Gruppe steht.

3. Benzodiazepine nach Anspruch 1 oder 2,
dadurch gekennzeichnet, daß
der Rest R2 Wasserstoff oder Fluor in Orthostellung ist zu dem C-Atom am Phenylring, das mit dem Diazepinring verbunden ist.

4. Benzodiazepine nach einem der Ansprüche 1 bis 3,
dadurch gekennzeichnet, daß
der Rest R1 Wasserstoff oder Chlor in Parastellung ist zu dem gemeinsamen N-Atom im Diazepinring und im heterozyklischen Ring, der mit dem Diazepinring kondensiert ist.

5. Benzodiazepine nach einem der Ansprüche 1 bis 4,
dadurch gekennzeichnet, daß
n den Wert "2" hat.

6. Benzodiazepine nach einem der Ansprüche 1 bis 5,
dadurch gekennzeichnet, daß
das asymmetrische C-Atom in $\alpha$-Stellung zur Carbonylgruppe am Diazepinring S-Konfiguration einnimmt entsprechend der Nomenklatur von Cahn, Ingold und Prelog.

7. Benzodiazepine nach Anspruch 1,
nämlich
a) (Indolyl-2-carbonylamino)-4-phenyl-6-tetrahydro-1,2,3,4-pyrrolo[3,2,1-j,k]benzodiazepin-[1,4]-on-3 , und hier vorzugsweise das 4S-Isomere;
b) (Fluor-2-phenyl)-6-[(indolyl-2)-carbonylamino]-4-tetrahydro-1,2,3,4-pyrrolo [3,2,1-j,k]-benzodiazepin-[1,4]-on-3, und hier vorzugsweise das 4S-Isomere;
c) Chlor-8-(fluor-2-phenyl)-6-[(indolyl-2)-carbonylamino]-4-tetrahydro-1,2,3,4-pyrrolo[3,2,1-j,k]-ben zodiazepin-1,4-on-3, und hier vorzugsweise das 4S-Isomere;
d) [(Indolyl-2)-carbonylami no]-5-phenyl-7-hexahydro-1,2,3,3a,4,5-pyrido[3,2,1-j,k]-benzodiazepin-[1,4]-on-4, und hier vorzugsweise das 5S-Isomere.

8. Verfahren zur Herstellung eines Benzodiazepin der Formel (I) nach Anspruch 1,
gekennzeichnet durch die Verfahrensschritte:
a) zur Erzeugung eines Benzodiazepin der Formel (I) in Form des Racemates
wird ein Benzodiazepin-1,4 der nachstehenden Formel (V)

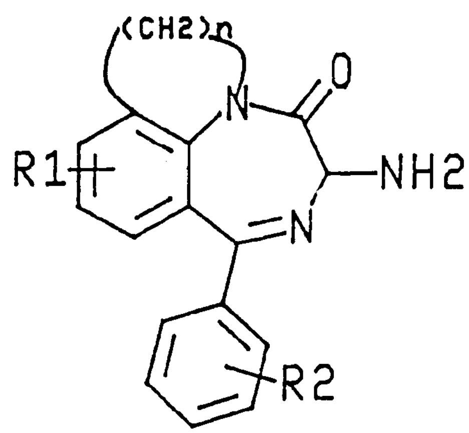

(V)

mit einem Carbonsäurederivat Ar-CO-X kondensiert,
wobei
die Gruppe Ar hat die in Anspruch 1 angegebene Bedeutung,
X steht für Halogen, eine Azidogruppe (-$N_3$), eine Imidazol-1-yl-Gruppe, oder für eine Gruppe -O-CO-R3,
wobei
R3 über die bevorzugte Gruppe Ar hinaus sein kann ein sperriger $C_{3-6}$-Alkylrest, oder eine Arylgruppe, welche noch sperriger als die Gruppe Ar ist und welche zusätzlich mit einem oder mehreren Halogenatom(en) substituiert sein kann, oder eine Gruppe -OR4,

wobei
R4 steht für eine aromatische Gruppe, die einen Ring oder zwei Ringe aufweist und die mit einem oder mehreren Nitro- oder Halogenrest(en) substituiert ist, oder für ei ne kondensierte heteroaromatische Gruppe, die ein bis drei Heteroatom(e) enthält;
b) zur Erzeugung eines Benzodiazepin der Formel (I) in optisch aktiver Form
wird ein Benzodiazepin-Racemat der Formel (V) rechtsdrehend oder linksdrehend kondensiert mit einem Molekül, das abgeleitet ist von einer optisch aktiven Aminosäure der nachstehenden Formel (X)

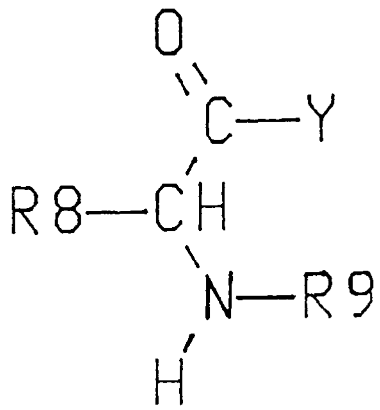

(X)

wobei
Y steht für einen Hydroxyrest, für einen Azidorest (-$N_3$), für eine Imidazol-1-yl-Gruppe, oder für ei ne Gruppe -O-CO-R3,
wobei
R3 sein kann eine verzweigte $C_{3-6}$-Alkylgruppe, eine sperrige Arylgruppe, die mit einem oder mehreren Halogenatomen substituiert sein kann, oder eine Gruppe -OR4,
wobei
R4 steht für eine aromatische Gruppe, die einen Ring oder zwei Ringe aufweist, und die wahlweise mit einem oder mehreren Nitro- oder Halogenrest(en) substituiert sein kann, oder für eine kondensierte heteroaromatische Gruppe, die ein bis drei Heteroatom(e) enthält;

R8 steht für:

. eine $C_{1-6}$-Alkylgruppe, die wahlweise substituiert ist mit einem Hydroxylrest, mit einer Thioalkylgruppe mit einer $C_{1-6}$-Alkylgruppe, mit einer Carboxylgruppe oder mit einer Carbonylaminogruppe,
. eine Arylgruppe, die einen Ring oder zwei Ringe aufweist, die wahlweise mit einem Hydroxylrest substituiert sind,
. eine Arylalkylgruppe mit einem oder zwei aromatischen Ring(en) mit einer $C_{1-6}$-Alkylgruppe, wobei der/die Ring(e) wahlweise substituiert sein können mit einem oder mehreren Halogenrest(en), Hydroxylrest(en) oder Methoxyrest(en),
. einen heterozyklischen Ring mit fünf oder sechs Ringgliedern und mit ein oder zwei Heteroatomen, nämlich Stickstoff, Sauerstoff oder Schwefel,
. eine Indolyl-3-methyl-Gruppe,
. eine Imidazoly-4-methyl-Gruppe.

R9 steht für

eine Gruppe, die sich leicht von einem freien Amin ablöst und dieses gegebenenfalls auch wieder bildet, und sein kann

. eine Oxycarbonylgruppe der Struktur A-O-CO-, wobei A steht für eine $C_{1-6}$-Alkylgruppe oder für eine Arylgruppe, die wahlweise mit einem oder mehreren Methoxy-, Halogen- oder Nitro-Rest(en) substituiert sein kann,
. eine $C_{1-6}$-Alkanoylgruppe, oder für eine $C_{1-6}$-Alkenoylgruppe oder für eine Aroylgruppe,
. eine p-Nitrosulfenyl-Gruppe;

zur Erzeugung einer optisch aktiven Verbindung der nachstehenden Formel (VII)

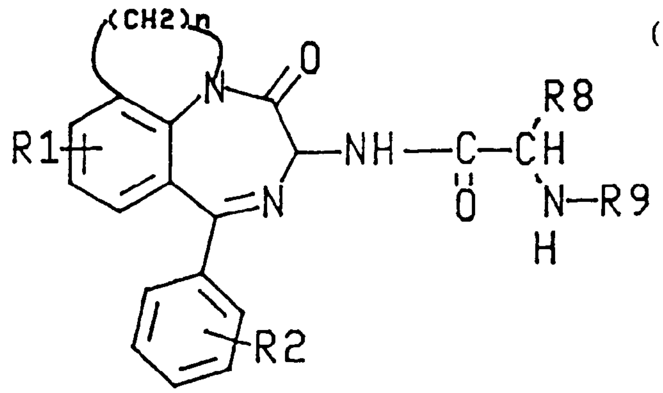

(VII)

wobei

i) wird für den Fall, daß R9 für einen Benzyloxycarbonyl-Rest steht, der wahlweise substituiert sein kann, in Gegenwart eines Edelmetall-Träger-Katalysators hydriert,

ii) wird im Fall aller anderen Substituenten in saurem oder basischem Milieu hydrolysiert, wobei sowohl im Falle (i) wie im Fall (ii) ein Gemisch der beiden Diastereo-isomeren der nachstehenden Formel (VIII) anfällt:

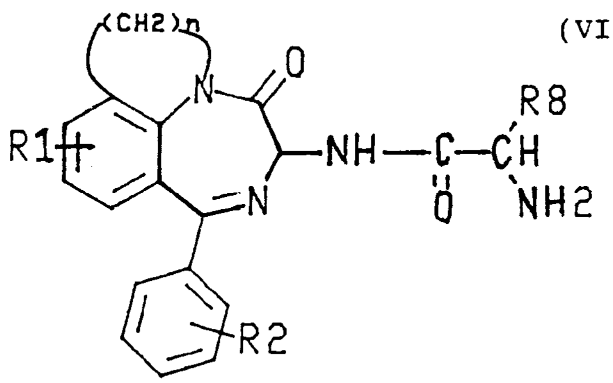

(VIII)

das erhaltene Diastereo-isomeren-Gemisch (VIII) wird nach einem physikalisch/chemischen Verfahren aufgetrennt, nämlich mit Hilfe der Chromatographie und/oder mit Hilfe der fraktionierten Kristallisation einer Verbindung (VIII) oder eines Additionssalzes der Verbindung (VIII) mit einer Säure;

danach wird mit einem Arylisothiocyanat der Formel R10-N=C=S umgesetzt, wobei

R10 steht für einen Phenylrest oder für einen Naphtylrest, die wahlweise mit einem oder mehreren Halogen-, Methoxy- oder $C_{1-6}$-Alkyl-Rest(en) substituiert sein können,

um mindestens ein Diastereo-isomeres zu erhalten in Form ei nes optisch aktiven Thioharnstoffes mit der nachstehenden Formel (IX):

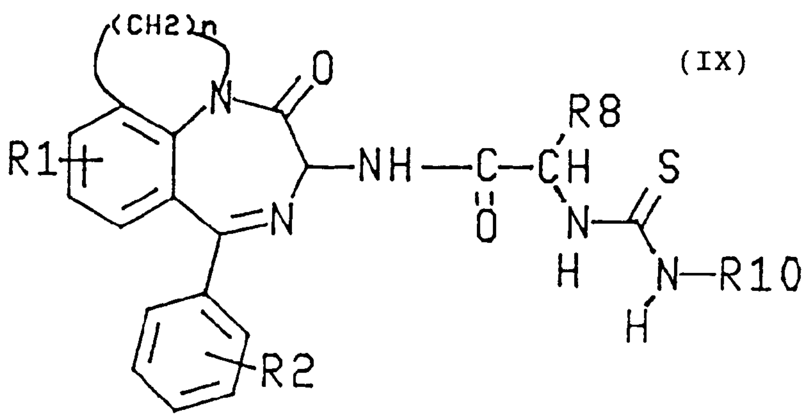

(IX)

daraufhin wird der optisch aktive Thioharnstoff (IX) umgesetzt, um die Aminosäuregruppe zu beseitigen und um eine Säurebehandlung durchzuführen, um das optisch aktive Amin (V) zu erhalten, das nunmehr mit einem geeigneten Reagens umgesetzt wird, um im Falle (a) das Benzodiazepin-Racemat zu erhalten, oder um das optisch aktive Benzodiazepin (I) zu erhalten.

**9.** Verfahren nach Anspruch 8,
dadurch gekennzeichnet, daß
zur Erzeugung eines Amin (V) ein Benzodiazepin-1,4-on der nachstehenden Formel (III)

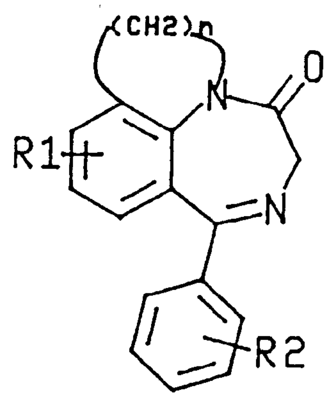

(III)

in α-Stellung zur Carbonylgruppe mit einem Hydroxylamin-Derivat oder mit Chloramin aminiert wird.

**10.** Verfahren nach Anspruch 8,
dadurch gekennzeichnet, daß
eine Aminierung des Benzodiazepin-1,4-on (III) in zwei Stufen durchgeführt wird, wobei
in einer ersten Stufe mit einem Oximbildungsmittel R5-$(N=O)_m$ umgesetzt wird, wobei
R5 steht für einen niederen Alkoxyrest oder für Chlor,
m hat den Wert "1", oder m hat den Wert "2", wenn eine zusätzliche Bindung zwischen den beiden Stickstoffatomen vorhanden ist, um ein Oxim der nachstehenden Formel (IV) zu erhalten, das isoliert wird:

(IV)

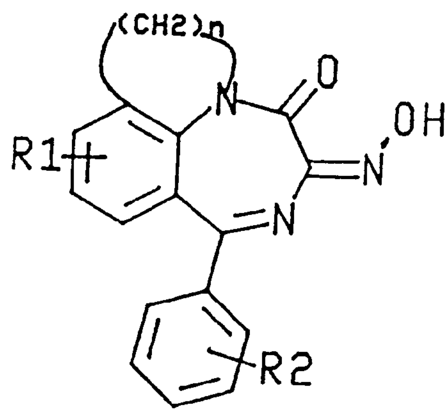

in der zweiten Stufe wird das Oxim (IV) katalytisch in Gegenwart eines Reduktionskatalysators mit Wasserstoff reduziert, oder es wird mit Zink in Gegenwart von Essigsäure umgesetzt, oder es wird mit Zinn(II)chlorid in Gegenwart von Salzsäure umgesetzt, um das Aminderivat zu erhalten.

**11.** Verfahren nach Anspruch 8,
dadurch gekennzeichnet, daß
zur Erzeugung des Amin (V) ein Benzodiazepin-[1,4]-on (III) in basischem Milieu mit einem Reagens umgesetzt wird, das in das Carbanion eine Stickstoff-Funktion ($N_3$) einführt, um ein Stickstoffderivat der nachstehenden Formel (VI) zu erhalten:

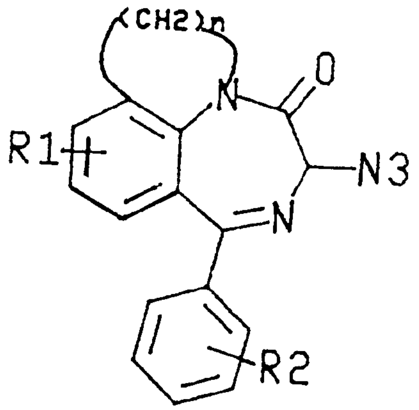

(VI)

12. Verfahren zur Herstellung eines Arzneimittels, insbesondere zur Bekämpfung von Beschwerden des Magen-Darm-Traktes, der Bauchspeicheldrüse, der Gallenblase, bei Appetitstörungen, als Schmerzmittel und wahlweise zur Bekämpfung von Störungen des zentralen Nervensystems,
dadurch gekennzeichnet, daß
ein Benzodiazepin nach einem der Ansprüche 1 bis 7 mit einem pharmazeutischen Träger vermischt ist.

13. Die Verwendung eines Benzodiazepin nach einem der Ansprüche 1 bis 7 zur Herstellung eines Arzneimittels, insbesondere zur Bekämpfung von Beschwerden des Magen-Darm-Traktes, der Bauchspeicheldrüse, der Gallenblase, bei Appetitstörungen, als Schmerzmittel und wahlweise zur Bekämpfung von Störungen des zentralen Nervensystems.

14. Zwischenprodukte der nachstehenden Formel (XI)

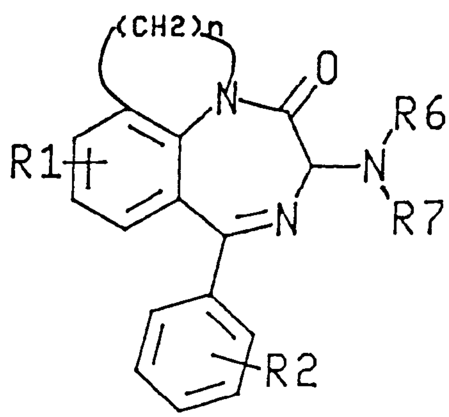

(XI)

wobei
n, R1 und R2 die in Anspruch 1 angegebene Bedeutung haben, R6 für den Hydroxylrest steht, wenn R7 eine zusätzliche Bindung zwischen dem, den Rest R6 tragenden N-Atom und dem Diazepinring bildet, und R6 für den Wasserstoffrest steht, wenn R7 für Wasserstoff steht,
dadurch gekennzeichnet, daß
zur Erzeugung von Zwischenprodukten der Formel (XI), wobei
R6 für den Hydroxylrest steht,
ein Benzodiazepin-1,4-on der nachstehenden Formel (III)

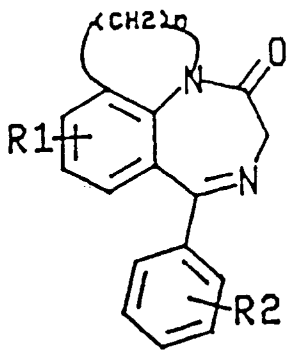

(III)

mit einem Oximbildungsmittel $R5\text{-}(N{=}O)_m$ umgesetzt wird, wobei
R5 steht für einen niederen Alkoxyrest oder für Chlor, wenn m den Wert "1" hat, und eine zusätzlichen Bindung zwischen den beiden Stickstoffatomen vorhanden ist, wenn m den Wert "2" hat,

und daß ferner zur Erzeugung von Zwischenprodukten der Formel (XI), wobei R6 und R7 für Wasserstoff stehen,
jene Zwischenprodukte, in denen R6 für den Hydroxylrest stehen, katalytisch in Gegenwart eines Reduktionskatalysators mit Wasserstoff reduziert werden oder mit Zink in Gegenwart von Essigsäure umgesetzt werden oder mit Zinn(II)chlorid in Gegenwart von Salzsäure umgesetzt werden, oder ein Benzodiazepin-[1,4]-on der Formel (III) mit einem Hydroxylamin-Derivat oder mit Chloramin in n-Stellung zur Carbonylgruppe aminiert wird.

## Claims

**Claims for the following Contracting States : AT, BE, CH, DE, FR, GB, IT, LI, LU, NL, SE**

1. Benzodiazepines of formula

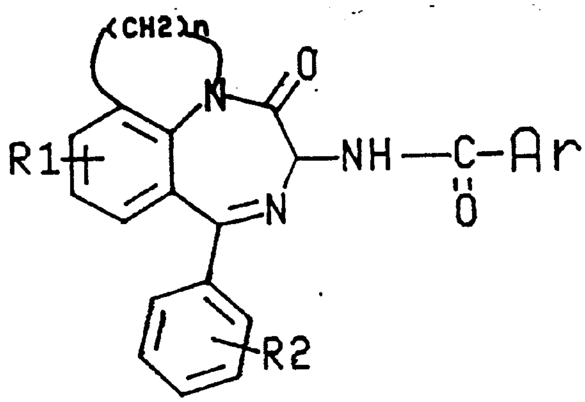

(I)

in which $R_1$ is H or halogen, $R_2$ is H or halogen, Ar is indolyl, phenyl, naphthyl, indolyl monosubstituted with a halogen or with a methoxy or pnenyl mono-, di- or trisubstituted with a halogen or with a methoxy or with a trifluoromethyl group, and n is 2 or 3; and their optical isomers.

2. Benzodiazepines according to Claim 1, characterised in that Ar is a 2-indolyl group.

3. Benzodiazepines according to Claim 1 or 2, characterised in that $R_2$ is hydrogen or fluorine at the ortho position with respect to the carbon atom linking the phenyl ring to the diazepine ring.

4. Benzodiazepines according to any one of Claims 1 to 3, characterised in that $R_1$ is hydrogen or chlorine at the para-position with respect to the nitrogen atom common to the diazepine ring and to the other nitrogen heterocycle fused to the diazepine ring.

5. Benzodiazepines according to any one of Claims 1 to 4, characterised in that n is equal to 2.

6. Benzodiazepines according to any one of Claims 1 to 5, characterised in that the asymmetric carbon atom at the alpha-position with respect to the carbonyl of the diazepine ring possesses the S configuration according to the nomenclature of Cahn, Ingold and Prelog.

7. Benzodiazepines according to Claim 1, which are:
a) 4-(2-indolylcarbonylamino)-6-phenyl-1,2,3,4-tetrahydropyrrolo [3,2,1-jk][1-4]benzodiazepin-3-one, and preferably its 4S isomer.
b) 6-(2-fluorophenyl)-4-(2-indolylcarbonylamino)-1,2,3,4-tetrahydropyrrolo [3,2,1-jk][1-4]benzodiazepin-3-one, and preferably its 4S isomer.
c) 8-chloro-6-(2-fluorophenyl)-4-(2-indolylcarbonylamino)-1,2,3,4-tetrahydropyrrolo [3,2,1-jk][1,4]benzodiazepin-3-one and preferably its 4S isomers.
d) 5-(2-indolylcarbonylamino)-4-phenyl-1,2,3,3a,4,5-hexahydropyrido [3,2,1-jk][1,4]benzodiazepin-4-one and preferably its 5S isomer.

8. Process for preparing benzodiazpines of formula (I) in Claim 1, characterised in that it consists
a) for preparing a benzodiazepine of formula (I) in its racemic form:
in condensing a 1,4-benzodiazepine of formula

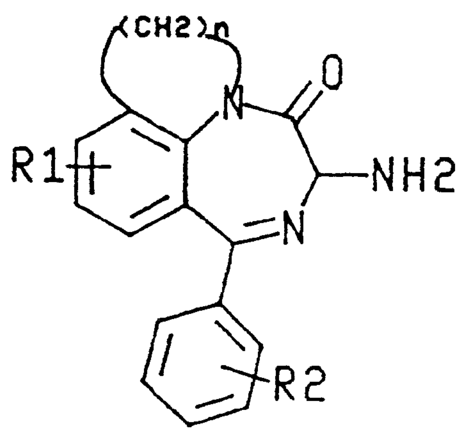

(V)

with a carboxylic acid derivative of formula Ar-CO-X, in which Ar has the meaning stated in Claim 1 and X is a halogen, an azido ($-N_3$) group, a 1-imidazolyl group, a group $-O-CO-R_3$, it being possible for $R_3$ to be, besides preferably Ar, a hindered alkyl radical containing from three to six carbon atoms, or an aryl radical more hindered than the radical Ar, substituted with one or more halogens, or a group $-OR_4$, $R_4$ being an aromatic group containing one or two rings and substituted with one or more nitro or halogen groups, or a condensed heteroaromatic group containing from one to three hetero atoms
b) for preparing a benzodiazepine of formula (I) in a dextrorotary or laevorotary optically active form in condensing a racemic benzodiazepine of formula (V) with a molecule derived from an optically active amino acid of formula (X):

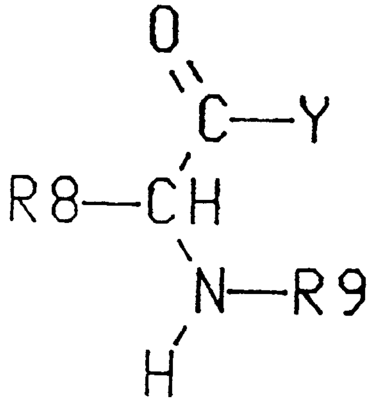

(X)

in which

Y is a hydroxy group, an azido ($-N_3$) group, a 1-imidazolyl group, a group $-O-CO-R_3$, it being possible for $R_3$ to be a branched alkyl radical containing from three to six carbon atoms, a hindered aryl group substituted with one or more halogens, or a group $-OR_4$, $R_4$ being an aromatic group containing one or two rings, optionally substituted with one or more nitro or halogen radicals or a condensed heteroaromatic group containing from one to three hetero atoms,

$R_8$ is

- an alkyl group having from 1 to 6 carbon atoms, optionally substituted with a hydroxyl, a thioalky group having from one to six carbon atoms in the alkyl group or a carboxyl or carbonylamido group,
- an aryl group containing one or two rings, optionally substituted with a hydroxyl,
- an aralkyl group having one or two aromatic rings and in which the alkyl portion contains from one to six carbon atoms, optionally substituted on the ring with one or more halogen, hydroxy or methoxy groups
- a five- or six-membered heterocycle having one or two hetero atoms chosen form nitrogen, oxygen and sulphur
- a 3-indolylmethyl group
- a 4-imidazolylmethyl group.

R9 is a group which is readily removable to regenerate to free amine, and can be

- an oxycarbonyl radical of structure A-O-CO-, in which A is an alkyl group containing from one to six carbon atoms, or an aryl group, optionally substituted with one or more methoxy, halogen or nitro groups,
- an alkanoyl or alkenyl group containing from one to six carbon atoms or an aroyl group
- p-nitrosulphenyl,

to obtain an optically active compound of formula

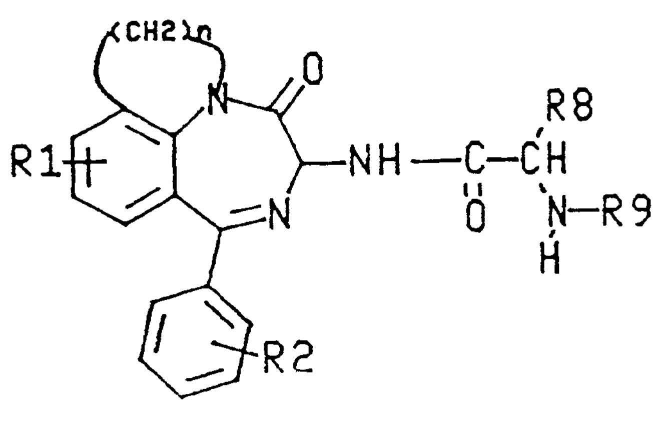

(VII)

which

i) in the case where $R_9$ is benzoyloxycarbonyl optionally substituted, is hydrogenated in the presence of a catalyst chosen from noble metals deposited on a support, or

ii) in all other cases, is hydrolysed in an acid or basic medium, to obtain, both in case i) and in case ii),

a mixture of two diastereoisomers of formula (VIII)

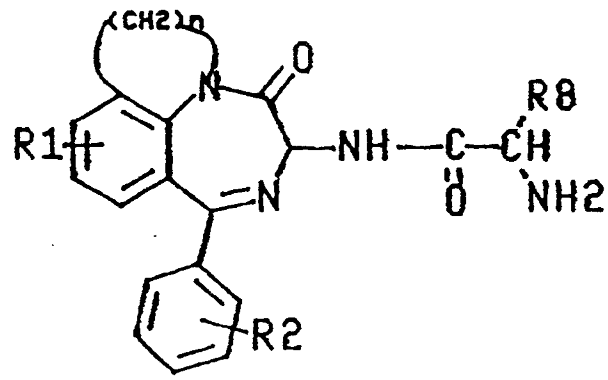

(VIII)

then in separating these diastereoisomers by a physicochemical process chosen from a chromatographic method and/or a fractional crystallisation of the base of formula VIII or of one of its addition salts with an acid

then in reacting an aryl isothiocyanate of formula $R_{10}$-N=C=S, in which $R_{10}$ is a phenyl or naphthyl radical optionally substituted with one or more substituents chosen from halogens, a methoxy group or an alkyl group having from one to six carbon atoms, with at least one of the diastereoisomers to obtain an optically active thiourea of formula (IX):

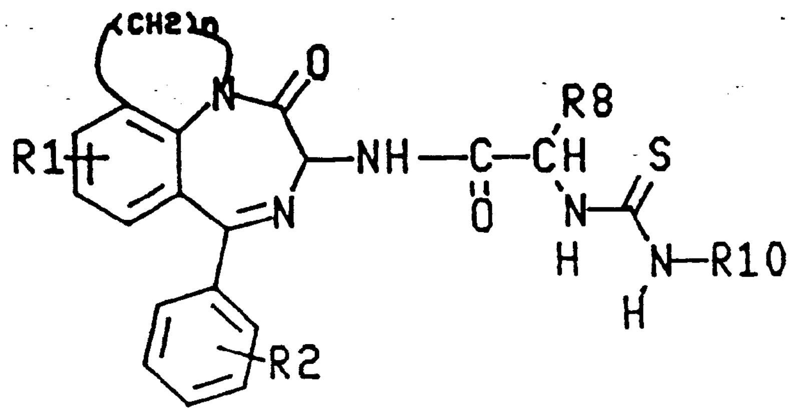

(IX)

and then in performing of the thiourea of formula IX a reaction of removal of the amino acid radical by treating it with an acid, to obtain the optically active amine (V) which is thereafter reacted as in the case of the preparation of the racemic benzodiazepine, to obtain the optically active benzodiazepine of formula I.

**9.** Process according to Claim 8, characterised in that it consists in preparing the amine of formula (V) by aminating, at the alpha position with respect to the carbonyl, a 1,4-benzodiazepinone of formula

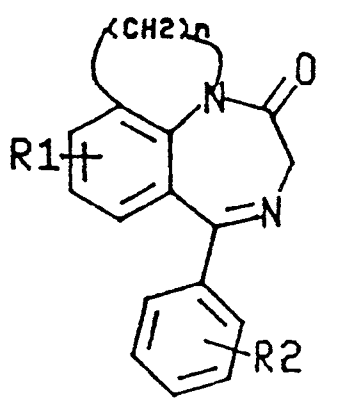

(III)

with a hydroxylamine derivative or with chloramine.

**10.** Process according to Claim 8, characterised in that it consists in aminating the 1,4-benzodiazepinone of formula (III) in two stages, the first stage consisting in reacting it with an oximation reagent of formula $R_5$-(N=O)$_m$, in which $R_5$ is lower alkoxy or chlorine when m is equal to 1 and is an additional bond between the nitrogen atoms when m is equal to 2, to obtain the oxime of formula (IV):

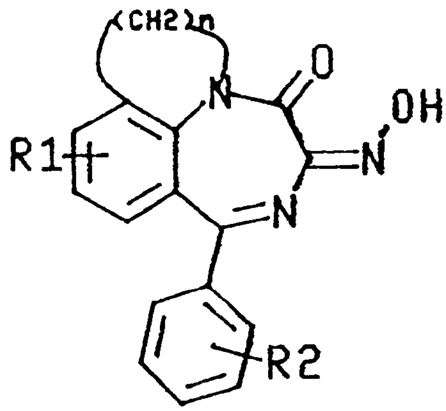

(IV)

which is isolated, and the second stage consisting in reducing the oxime catalytically with hydrogen in the presence of a reduction catalyst or by reaction with zinc in the presence of acetic acid or with stannous chloride in the presence of hydrochloric acid, to obtain the amino derivative.

**11.** Process according to Claim 8, characterised in that it consists in preparing the amine of fo rmula (V) by reacting a 1,4-benzodiazepinone of formula (III) in a basic medium with a reagent capable of introducing an azide group onto a carbanion, to obtain an azide of formula

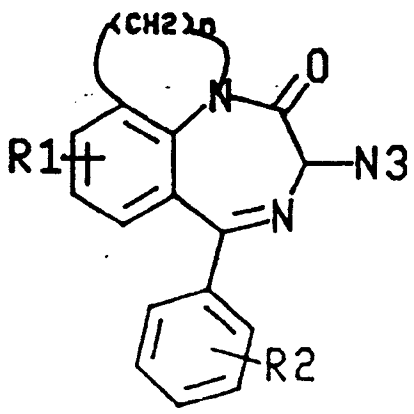

(VI)

**12.** Medicinal product, chiefly for combating gastrointestinal disorders and disorders of the pancreas, the gallbladder and the appetite; pain; and possibly disorders of the central nervous system; characterised in that it comprises an benzodiazepine according to one of Claims 1 to 7.

**13.** Intermediates of formula

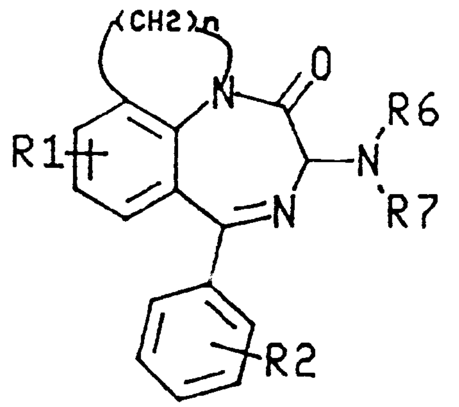

(XI)

n, $R_1$ and $R_2$ having the meanings stated in Claim 1, $R_5$ being hydroxy when $R_7$ denotes an additional bond between the nitrogen atom bearing $R_6$ and the diazepine ring, and $R_6$ being hydrogen when $R_7$ is hydrogen.

**Claims for the following Contracting State : ES**

1. Process for preparing benzodiazepines of formula

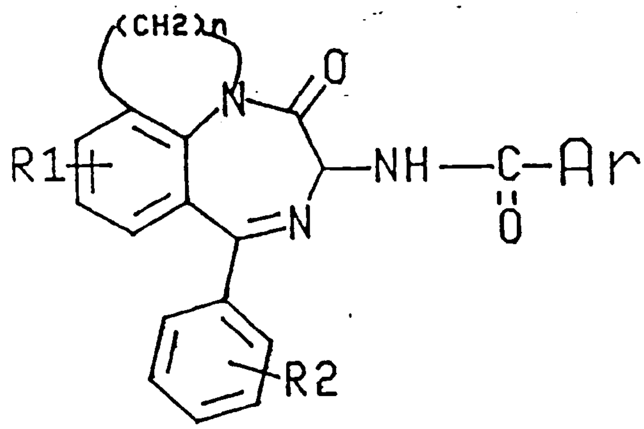

(I)

in which $R_1$ is H or halogen, $R_2$ is H or halogen, Ar is indolyl, phenyl, naphtyl, indolyl monosubstituted with a halogen or with a methoxy of phenyl mono-, di- or trisubstituted with a halogen or with a methoxy or with a tirfluoromethyl group, and n is 2 or 3; and their optical isomers, characterized in that it comprises

a) for preparing a benzodiazepine of formula (I) in its racemic form:

in condensing a 1,4-benzodiazepine of formula

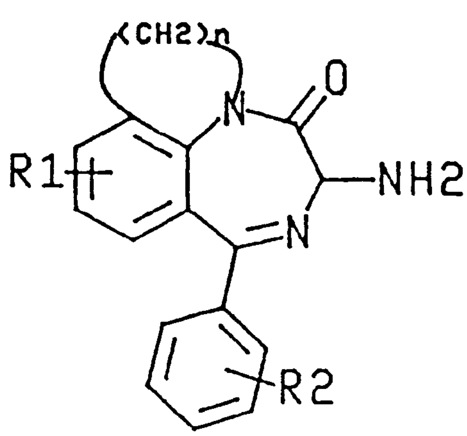

(V)

with a carboxylic acid derivative of formula Ar-CO-X, in which Ar has the meaning stated in Claim 1 and X is a halogen, an azido ($-N_3$) group, a 1-imidazolyl group, a group $-O-CO-R_3$, it being possible for $R_3$ to be, besides preferably Ar, a hindered alkyl radical containing from three to six carbon atom, or an aryl radical more hindered than the radical Ar, substituted with one or more halogens, or a group $-OR_4$, $R_4$ being an aromatic group containing one or two rings and substituted with one or more nitro or halogen groups, or a condensed heteroaromatic group containing from one to three hetero atoms

b) for preparing a benzodiazepine of formula (I) in a dextrorotary or laevorotary optically active form

in condensing a racemic benzodiazepine of formula (v) with a molecule derived from an optically active amino acid of formula (X):

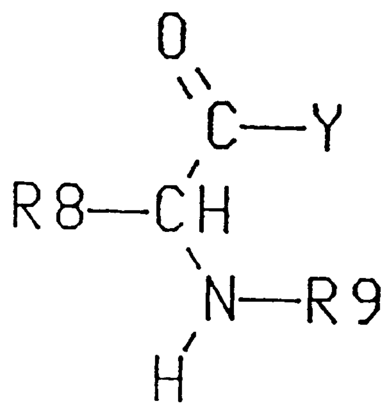

(X)

in which

Y is a hydroxy group, an azido ($-N_3$) group, a 1-imidazolyl group, a group $-O-CO-R_3$, it being possible for $R_3$ to be a branched alkyl radical containing from three to six carbon atoms, a hindered aryl group substituted with one or more halogens, or a group $-OR_4$, $R_4$ being an aromatic group containing one or two rings, optionally substituted with one or more nitro or halogen radicals or a condensed heteroaromatic group containing from one to three hetero atoms,

$R_6$ is

- an alkyl group having from 1 to 6 carbon atoms, optionally substituted with a hydroxyl, a thioalkyl group having from one to six carbon atoms in the alkyl group or a carboxyl or carbonylamido group,
- an aryl group containing one or two rings, optionally substituted with a hydroxyl,
- an aralkyl group having one or two aromatic rings and in which the alkyl portion contains from one to six carbon atoms, optionally substituted on the ring with one or more halogen, hydroxy or methoxy groups
- a five- or six-membered heterocycle having one or two hetero atoms chosen from nitrogen, oxygen and sulphur
- a 3-indolylmethyl group
- a 4-imidazolylmethyl group.

R9 is a group which is readily removable to regenerate the free amine, and can be

- an oxycarbonyl radical of structure A-O-CO-, in which A is an alkyl group containing from one to six carbon atoms, or an aryl group, optionally substituted with one or more methoxy, halogen or nitro groups,
- an alkanoyl or alkenyl group containing from one to six carbon atoms or an aroyl group
- p-nitrosulphenyl,

to obtain an optically active compound of formula

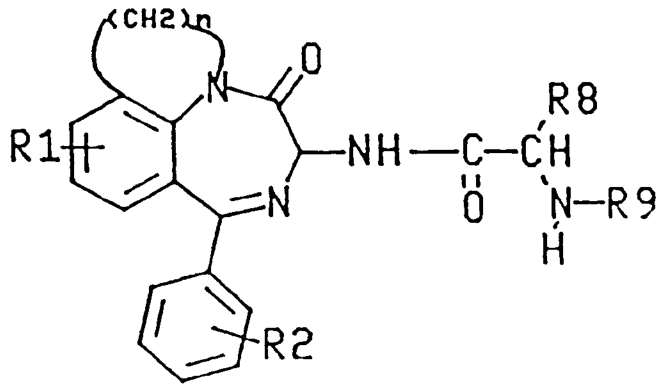

(VII)

which

i) in the case where $R_9$ is benzoyloxycarbonyl, optionally substituted, is hydrogenated in the presence of a catalyst chosen from noble metals deposited on a support, or

ii) in all other cases, is hydrolysed in an acid or basic medium, to obtain, both in case i) and in case ii), a mixture of two diastereoisomers of formula (VIII)

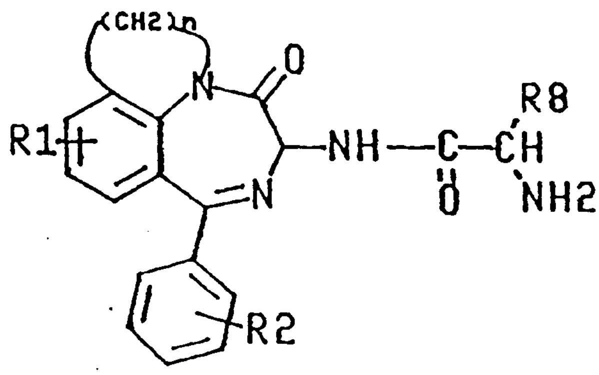

(VIII)

then in separating these diastereoisomers by a physicochemical process chosen from a chromatographic method and/or a fractional crystallisation of the base of formula VIII or of one of its addition salts with an acid

then in reacting an aryl isothiocyanate of formula $R_{10}$-N=C=S, in which $R_{10}$ is a phenyl or naphthyl radical optionally substituted with one or more substituents chosen from halogens, a methoxy group or an alkyl group having from one to six carbon atoms, with at least one of the diastereoisomers to obtain an optically active thiourea of formula (IX):

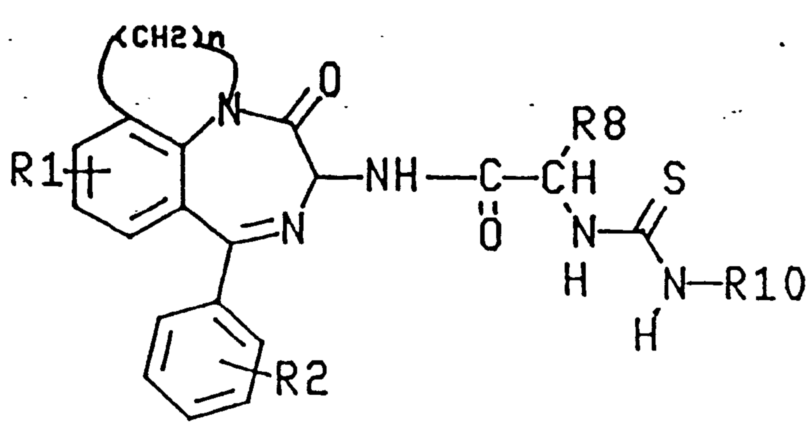

(IX)

and then in performing of the thiourea of formula IX a reaction of removal of the amino acid radical by treating it with an acid, to obtain the optically active amine (V) which is thereafter reacted as in the case of the preparation of the racemic benzodiazepine, to obtain the optically active benzodiazepine of formula I.

2. Process according to claim 1, characterised in that Ar is a 2-indolyl group.

3. Process according to claim 1 or 2, characterised in that $R_2$ is hydrogen or fluorine at the ortho position with respect to the carbon atom linking the phenyl ring to the diazepine ring.

4. Process according to any one of claims 1 to 3, characterized in that $R_1$ is hydrogen or chlorine at the para-position with respect to the nitrogen atom common to the diazepine ring and to the other nitrogen heterocycle fused to the diazepine ring.

5. Process according to any one of claims 1 to 4, characterised in that n is equal to 2.

6. Process according to any one of claims 1 to 5, characterised in that the asymmetric carbon atom at the alpha-position with respect to the carbonyl of the diazepine ring possesses the S configuration according to the nomenclature of Cahn, Ingold and Prelog.

7. Process according to claim 1, which comprises preparing:
   a) 4-(2-indolylcarbonylamio)-6-phenyl-1,2,3,4-tetrahydropyrrolo [3,2,1-jk][1,4] benzodiazepin-3-one, and preferably its 4S isomer.
   b) 6-(2-fluorophenyl)-4-(2-indolylcarbonylamino)-1,2,3,4-tetrahydropyrrolo[3,2,1,-jk][1,4]benzodiazepin-3-one, and preferably its 4S isomer.
   c) 8-chloro-6-(2-fluorophenyl)-4-(2-indolylcarbonylamino)-1,2,3,4-tetrahydropyrrolo[3,2,1- jk][1,4] benzodiazepin-3-one and preferably its 4S isomers.
   d) 5-(2-indolylcarbonylamino)-7-phenyl-1,2,3,3a,4,5-hexahydropyrido [3,2,1-jk][1,4]benzodiazepin-4-one and preferably its 5S isomer.

8. Process according to claim 1, characterized in that it comprises preparing the amine of formula (V) by aminating, at the alpha position with respect to the carbonyl, a 1,4-benzodiazepinone of formula

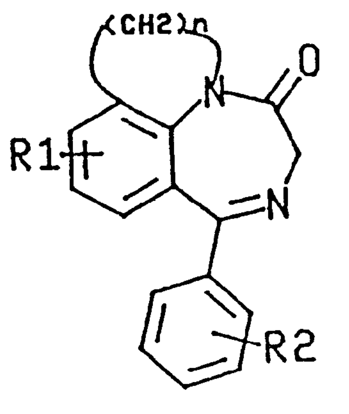

(III)

with a hydroxylamine derivative or with chloramine.

9. Process according to claim 1, characterized in that it comprises aminating the 1,4-benzodiazepinone of formula (III) in two stages, the first stage consisting in reacting it with an oximation reagent of formula $R_5$-$(N{=}O)_m$, in which $R_5$ is lower alkoxy or chlorine when m is equal to 1 and is an additional bond between the nitrogen atoms when m is equal to 2, to obtain the oxime of formula (IV):

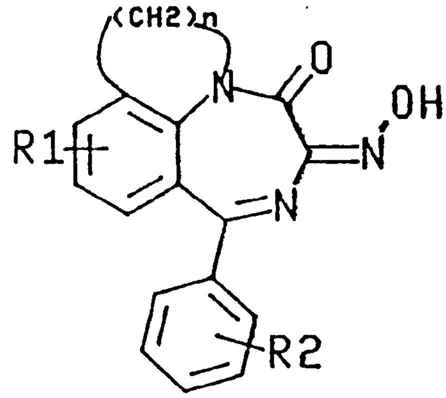

(IV)

which is isolated, and the second stage consisting in reducing the oxime catalytically with hydrogen in the presence of a reduction catalyst or by reaction with zinc in the presence of acetic acid or with stannous chloride in the presence of hydrochloric acid, to obtain the amino derivative.

**10.** Process according to claim 1, characterised in that it comprises preparing the amine of formula (V) by reacting a 1,4-benzodiazepinone of formula (III) in a basic medium with a reagent capable of introducing an azide group onto a carbanion, to obtain an azide of formula

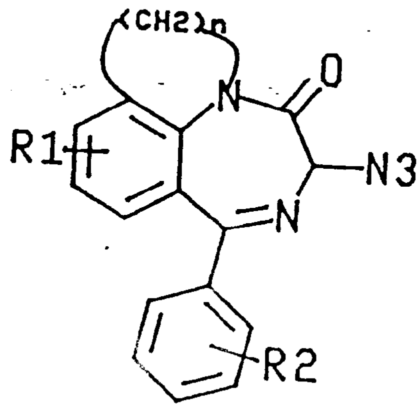

(VI)

**11.** Process for preparing a medicinal product, chiefly for combating gastrointestinal disorders and disorders of the pancreas, the gallbladder and the appetite; pain; and possibly disorders of the central nervous system; characterised in that it comprises mixing a benzodiazepine as defined in one of claims 1 to 7 with a pharmaceutical carrier.

**12.** The use of a benzodiazepine as defined in claims 1 to 7 for preparing a medicinal product for combating gastrointestinal disorders and disorders of the pancreas, the gallbladder and the appetite, pain; and possibly disorders of the central nervous system.

**13.** Process for preparing intermediates of formula

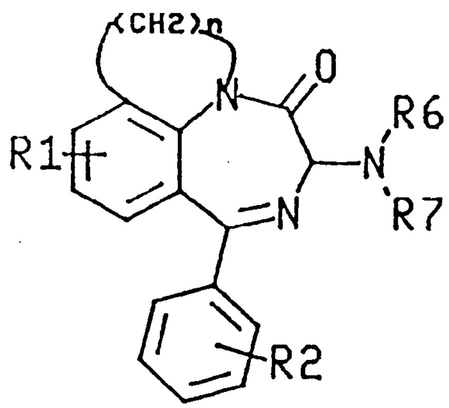

(XI)

n, $R_1$ and $R_2$ having the meanings stated in claim 1, $R_6$ being hydroxy when $R_7$ denotes an additional bond between the nitrogen atom bearing $R_6$ and the diazepine ring, and $R_6$ being hydrogen when $R_7$ is hydrogen, characterized in that it comprises reacting a benzodiazepine-[1,4]one of formula

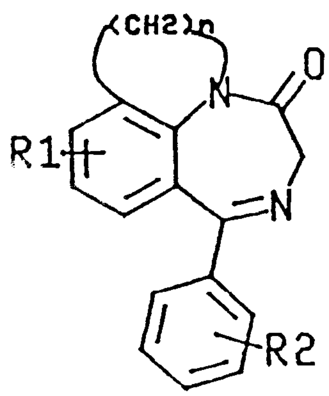

(III)

with an oximation reagent of formula $R_6$-(N=O)$_m$ in which $R_6$ is lower alkoxy or chlorine when m is equal to 1 and is an additional bond between the nitrogen atoms when m is equal to 2 to obtain the intermediates of formula XI in which $R_6$ is hydroxy and, for preparing those in which $R_6$ is hydroxy and, for preparing those in which $R_6$ and $R_7$ are hydrogen, reducing those in which $R_6$ is hydroxy with hydrogen in the presence of a reduction catalyst or by reaction with zinc in the presence of acetic acid or with stannous choride in the presence of hydrochloric acid or aminating in the alpha position relative to the carbonyl a benzodiazepine -[1,4]-one of formula (III) with a derivative of hydroxylamine or with chloramine.

**Claims for the following Contracting State : GR**

1. Benzodiazepines of formula

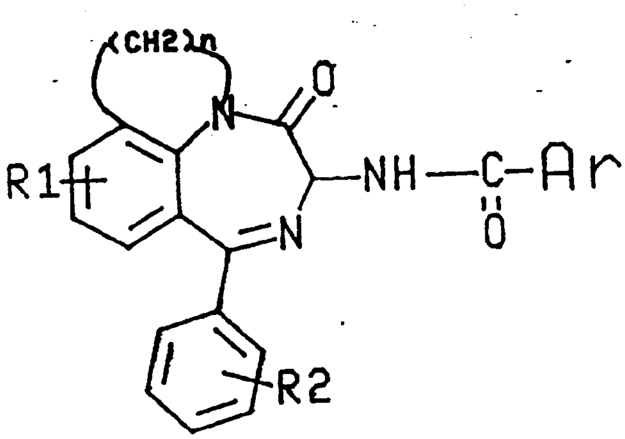

( I )

in which $R_1$ is H or halogen, $R_2$ is H or halogen, Ar is indolyl, phenyl, naphthyl, indolyl monosubstituted with a halogen or with a methoxy or phenyl mono-, di- or trisubstituted with a halogen or with a methoxy or with a trifluoromethyl group, and n is 2 or 3; and their optical isomers.

2. Benzodiazepines according to Claim 1, characterised in that Ar is a 2-indolyl group.

3. Benzodiazepines according to Claim 1 or 2, characterised in that $R_2$ is hydrogen or fluorine at the ortho position with respect to the carbon atom linking the phenyl ring to the diazepine ring.

4. Benzodiazepines according to any one of Claims 1 to 3, characterised in that $R_1$ is hydrogen or chlorine at the para-position with respect to the nitrogen atom common to the diazepine ring and to the other nitrogen heterocycle fused to the diazepine ring.

5. Benzodiazepines according to any one of Claims 1 to 4, characterised in that n is equal to 2.

6. Benzodiazepines according to any one of Claims 1 to 5, characterised in that the asymmetric carbon atom at the alpha-position with respect to the carbonyl of the diazepine ring possesses the S configuration according to the nomenclature of Cahn, Ingold and Prelog.

7. Benzodiazepines according to Claim 1, which are:
a) 4-(2-indolylcarbonylamino)-6-phenyl-1,2,3,4-tetrahydropyrrolo [3,2,1-jk][1,4]benzodiazepin-3-one, and preferably its 4S isomer.
b) 6-(2-fluorophenyl)-4-(2-indolylcabonylamino)-1,2,3,4-tetrahydropyrrolo[3,2,1-jk][1,4]benzodiazepin-3-one, and preferably its 4S isomer.
c) 8-chloro-6-(2-fluorophenyl)-4-(2-indolylcarbonylamino)-1,2,3,4-tetrahydropyrrolo [3,2,1-jk][1,4]benzodiazepin-3-one and preferably its 4S isomers.
d) 5-(2-indolylcarbonylamino)-7-phenyl-1,2,3,3a,4-5-hexahydropyrido [3,2,1-jk]-[1,4]benzodiazepin-4-one and preferably its 5S isomer.

8. Process for preparing benzodiazepines of formula (I) in Claim 1, characterised in that it comprises
a) for preparing a benzodiazepine of formula (I) in its racemic form:
in condensing a 1,4-benzodiazepine of formula

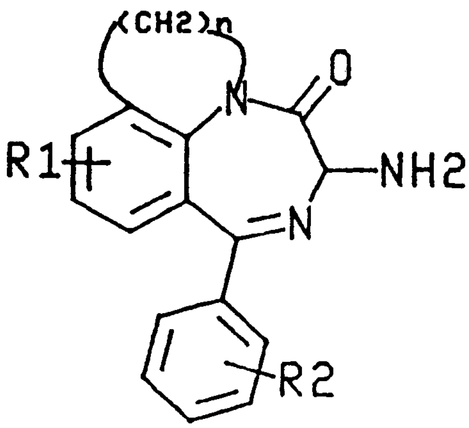

(V)

with a carboxylic acid derivative of formula Ar-CO-X, in which Ar has the meaning stated in Claim 1 and X is a halogen, an azido ($-N_3$) group, a 1-imidazolyl group, a group $-O-CO-R_3$, it being possible for $R_3$ to be, besides preferably Ar, a hindered alkyl radical containing from three to-six carbon-atoms, or an aryl radical more hindered than the radical Ar, substituted with one or more halogens, or a group $-OR_4$, $R_4$ being an aromatic group containing one or two rings and substituted with one or more nitro or halogen groups, or a condensed heteroaromatic group containing from one to three hetero atoms
b) for preparing a benzodiazepine of formula (I) in a dextrorotary or laevorotary optically active form
in condensing a racemic benzodiazepine of formula (V) with a molecule derived from an optically active amino acid of formula (X):

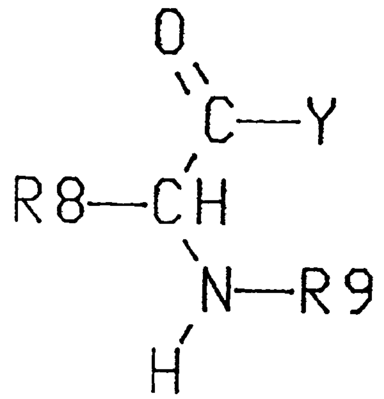

(X)

in which

Y is a hydroxy group, an azido ($-N_3$) group, a 1-imidazolyl group, a group $-O-CO-R_3$, it being possible for $R_3$ to be a branched alkyl radical containing from three to six carbon atoms, a hindered aryl group substituted with one or more halogens, or a group $-OR_4$, $R_4$ being an aromatic group containing one or two rings, optionally substituted with one or more nitro or halogen radicals or a condensed heteroaromatic group containing from one to three hetero atoms,

$R_6$ is

- an alkyl group having from 1 to 6 carbon atoms, optionally substituted with a hydroxyl, a thioalkyl group having from one to six carbon atoms in the alkyl group or a carboxyl or carbonylamido group,
- an aryl group containing one or two rings, optionally substituted with a hydroxyl,
- an aralkyl group having one or two aromatic rings and in which the alkyl portion contains from one to six carbon atoms, optionally substituted on the ring with one or more halogen, hydroxy or methoxy groups
- a five- or six-membered heterocycle having one or two hetero atoms chosen from nitrogen, oxygen and sulphur
- a 3-indolylmethyl group
- a 4-imidazolylmethyl group.

$R_9$ is a group which is readily removable to regenerate the free amine, and can be

- an oxycarbonyl radical of structure A-O-CO-, in which A is an alkyl-group containing from one to six carbon atoms, or an aryl group, optionally substituted with one or more methoxy, halogen or nitro groups,
- an alkanoyl or alkenyl group containing from one to six carbon atoms or an aroyl group
- p-nitrosulphenyl,

to obtain an optically active compound of formula

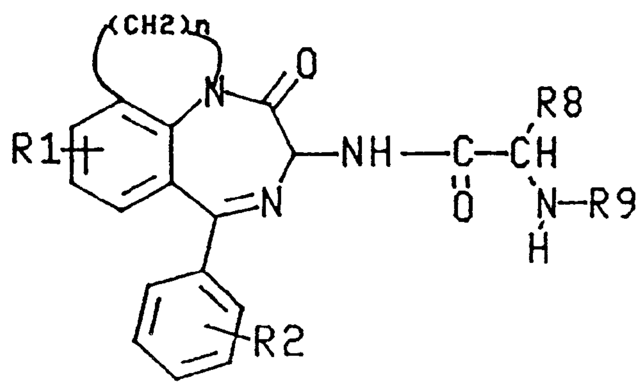

(VII)

which

i) in the case where $R_9$ is benzoyloxycarbonyl, optionally substituted, is hydrogenated in the presence of a catalyst chosen from noble metals deposited on a support, or

ii) in all other cases, is hydrolysed in an acid or basic medium, to obtain, both in case i) and in case ii), a mixture of two diastereoisomers of formula (VIII)

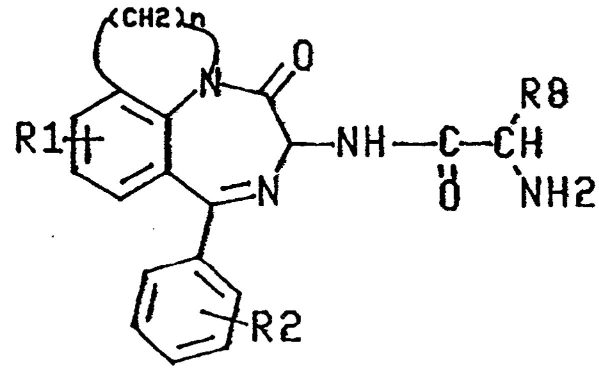

(VIII)

then in separating these diastereoisomers by a physicochemical process chosen from a chromatographic method and/or a fractional crystallisation of the base of formula VIII or of one of its addition salts with an acid

then in reacting an aryl isothiocyanate of formula $R_{10}$-N=C=-S, in which $R_{10}$ is a phenyl or naphthyl radical optionally substituted with one or more substituents chosen from halogens, a methoxy group or an alkyl group having from one to six carbon atoms, with at least one of the diastereoisomers to obtain an optically active thiourea of formula (IX):

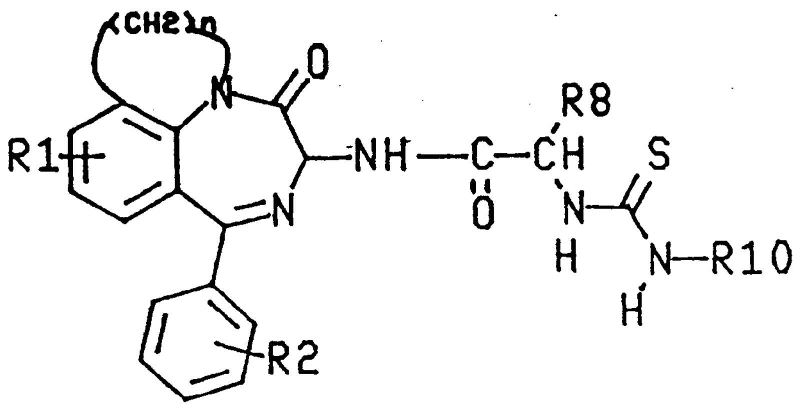

(IX)

and then in performing of the thiourea of formula IX a reaction of removal of the amino acid radical by treating it with an acid, to obtain the optically active amine (V) which is thereafter reacted as in the case of the preparation of the racemic benzodiazepine, to obtain the optically active benzodiazepine of formula I.

9. Process according to Claim 8, characterised in that it comprises preparing the amine of formula (V) by aminating, at the alpha position with respect to the carbonyl, a 1,4-benzodiazepinone of formula

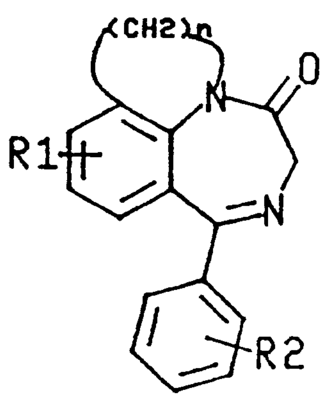

(III)

with a hydroxylamine derivative or with chloramine.

**10.** Process according to Claim 8, characterised in that it comprises aminating the 1,4-benzodiazepine of formula (III) in two stages, the first stage consisting in reacting it with oximation reagent of formula $R_5$-$(N{=}O)_m$, in which $R_5$ is lower alkoxy or chlorine when m is equal to 1 and is an additional bond between the nitrogen atoms when m is equal to 2, to obtain the oxime of formula (IV):

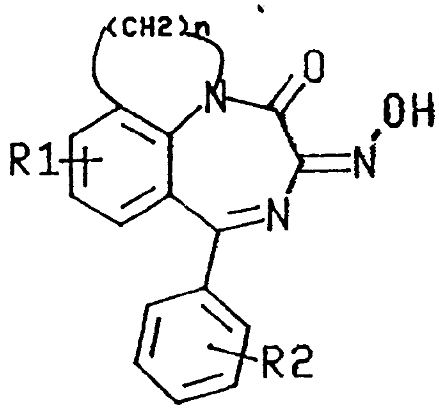

(IV)

which is isolated, and the second stage consisting in reducing the oxime catalytically with hydrogen in the presence of a reduction catalyst or by reaction with zinc in the presence of acetic acid or with stannous chloride in the presence of hydrochloric acid, to obtain the amino derivative.

**11.** Process according to Claim 8, characterised in that it comprises preparing the amine of formula (V) by reacting a 1,4-benzodiazepinone of formula (III) in a basic medium with a reagent capable of introducing an azide group onto a carbanion, to obtain an azide of formula

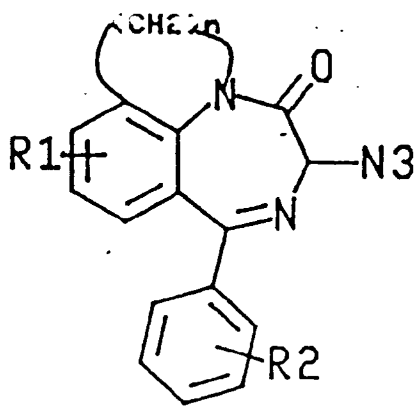

(VI)

**12.** Process for preparing a medicinal product, chiefly for combating gastrointestinal disorders and disorders of the pancreas, the gallbladder and the appetite; pain; and possibly disorders of the central nervous system; characterised in that it comprises mixing a benzodiazepine according to one of claims 1 to 7 with a pharmaceutical carrier.

**13.** The use of a benzodiazepine as defined in claims 1 to 7 for preparing a medicinal product for combating gastrointestinal disorders and disorders of the pancreas, the gallbladder and the appetite, pain; and possibly disorders of the central nervous system.

**14.** Intermediates of formula

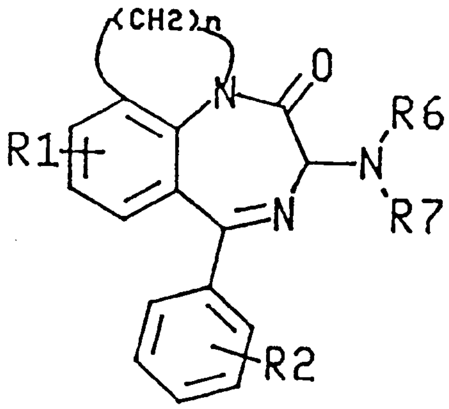

(XI)

n, $R_1$ and $R_2$ having the meanings stated in claim 1, $R_6$ being hydroxy when $R_7$ denotes an additional bond between the nitrogen atom bearing $R_6$ and the diazepine ring, and $R_6$ being hydrogen when $R_7$ is hydrogen.